# EUROPEAN PATENT APPLICATION

(11) **EP 3 819 386 A1**
(43) Date of publication of application: **12.05.2021**
(21) Application number: 19208124.8
(22) Date of filing: 08.11.2019
(51) Int. Cl.: C12Q 1/6883, G16B 30/00

(54) **DIAGNOSING OF MOOD DISORDERS USING BLOOD RNA EDITING BIOMARKERS**

(71) Applicant: ALCEDIAG, 13790 Peynier (FR)
(72) Inventor: WEISSMANN, Dinah, 34270 SAINT MATHIEU DE TREVIERS (FR); SALVETAT, Nicolas, 34070 MONTPELLIER (FR); CHIMIENTI, Fabrice, 34830 JACOU (FR)
(74) Representative: Touroude, Magali Linda

(57) **Abstract**

The present invention is drawn to a method for in vitro diagnosing mood disorders particularly depression disorders, more preferably major depressive disorder (MDD) in a human patient from a biological sample of said patient, using at least one or a combination of particular A to I editing RNA biomarkers associated to a specific depression score and algorithm. The present invention is also directed to a method for monitoring treatment for mood disorders in a human subject, preferably for monitoring depression and MDD, said method implementing the method for diagnosing mood disorders of the present invention. Kit for determining whether a patient presents a mood disorder, preferably depression disorder, more preferably MDD, is also comprised in the present invention.

SEQUENCE LISTING

<110> ALCEDIAG

<120> Diagnosis of mood disorders using blood RNA editing biomarkers

<130> ALC B009 EP1

<160> 36

<170> BiSSAP 1.3.6

<210> 1
<211> 23
<212> RNA
<213> Homo sapiens

<400> 1
atacgggcaa gctcttaact att 23

<210> 2
<211> 20
<212> RNA
<213> Homo sapiens

<400> 2
aacctccacc tcctgttttc 20

<210> 3
<211> 23
<212> RNA
<213> Homo sapiens

<400> 3
aaaatacggg caagctctta act 23

<210> 4
<211> 20
<212> RNA
<213> Homo sapiens

<400> 4
ctgcacttag gctcacagga 20

<210> 5
<211> 21
<212> RNA
<213> Homo sapiens

<400> 5
atgcaagttg tggacatgga g 21

<210> 6

<211> 21
<212> RNA
<213> Homo sapiens

<400> 6
ttctgaaaac aatgggcacc a                                                21

<210> 7
<211> 19
<212> RNA
<213> Homo sapiens

<400> 7
tgtgcaaagg gctccatac                                                   19

<210> 8
<211> 21
<212> RNA
<213> Homo sapiens

<400> 8
ccttgcaagt gtccagtaaa c                                                21

<210> 9
<211> 20
<212> RNA
<213> Homo sapiens

<400> 9
ctagggtgag gtggtgcaat                                                  20

<210> 10
<211> 20
<212> RNA
<213> Homo sapiens

<400> 10
caaggccagg agttcaagac                                                  20

<210> 11
<211> 22
<212> RNA
<213> Homo sapiens

<400> 11
tttaaaagct tctccagtca gc                                               22

<210> 12
<211> 27
<212> RNA
<213> Homo sapiens

<400> 12
caagactgag catacattat attcacc                                          27

<210> 13
<211> 22
<212> RNA
<213> Homo sapiens

<400> 13
tttaaaagct tctccagtca gc                                               22

<210> 14
<211> 20
<212> RNA
<213> Homo sapiens

<400> 14
tcctgagtag ctgggaccat                                                  20

<210> 15
<211> 20
<212> RNA
<213> Homo sapiens

<400> 15
cagcacattc tgttgccttc                                                  20

<210> 16
<211> 20
<212> RNA
<213> Homo sapiens

<400> 16
gtgactttgc ccctcatttg                                                  20

<210> 17
<211> 23
<212> RNA
<213> Homo sapiens

<400> 17
ggccactctg tactatgcaa atc                                              23

<210> 18
<211> 21
<212> RNA
<213> Homo sapiens

<400> 18

tgaatttcag atggacagca a                                                21

<210> 19
<211> 21
<212> RNA
<213> Homo sapiens

<400> 19
aagcccatcc tcgaactaca g                                                21

<210> 20
<211> 20
<212> RNA
<213> Homo sapiens

<400> 20
ttgctctgtc acctggactg                                                  20

<210> 21
<211> 20
<212> RNA
<213> Homo sapiens

<400> 21
gaaccaggct aggtgcaatg                                                  20

<210> 22
<211> 20
<212> RNA
<213> Homo sapiens

<400> 22
ccatgccctg gtaattttg                                                   20

<210> 23
<211> 23
<212> RNA
<213> Homo sapiens

<400> 23
tctgatggaa tcccaaactg aga                                              23

<210> 24
<211> 24
<212> RNA
<213> Homo sapiens

<400> 24
tcagagatat tttgtccgaa gcca                                             24

<210> 25
<211> 20
<212> RNA
<213> Homo sapiens

<400> 25
cccacagttt cagaggtggt                                                    20

<210> 26
<211> 18
<212> RNA
<213> Homo sapiens

<400> 26
ggctcacccc tgtaatcc                                                      18

<210> 27
<211> 20
<212> RNA
<213> Homo sapiens

<400> 27
gggatgtaaa cgctgaatgg                                                    20

<210> 28
<211> 20
<212> RNA
<213> Homo sapiens

<400> 28
atgtgccacc atgactgact                                                    20

<210> 29
<211> 20
<212> RNA
<213> Homo sapiens

<400> 29
cccttccggc aagaatccag                                                    20

<210> 30
<211> 20
<212> RNA
<213> Homo sapiens

<400> 30
acccaagctg cataaaggc                                                     20

<210> 31
<211> 24
<212> RNA

<213> Homo sapiens

<400> 31
gcaaccttag atctcaatgg ctaa                                              24

<210> 32
<211> 21
<212> RNA
<213> Homo sapiens

<400> 32
agcagagtca cagtctacag c                                                 21

<210> 33
<211> 20
<212> RNA
<213> Homo sapiens

<400> 33
ccggggctca tttataggat                                                   20

<210> 34
<211> 20
<212> RNA
<213> Homo sapiens

<400> 34
tgctagatac cgccacattg                                                   20

<210> 35
<211> 20
<212> RNA
<213> Homo sapiens

<400> 35
taaggacgga gttcgagacc                                                   20

<210> 36
<211> 21
<212> RNA
<213> Homo sapiens

<400> 36
tgagagagtt ttgctctgtg g                                                 21

## Description

The present invention is drawn to a method for in vitro diagnosing mood disorders particularly depression disorders, more preferably major depressive disorder (MDD) in a human patient from a biological sample of said patient, using at least one or a combination of particular A to I editing RNA biomarkers associated to a specific depression score and algorithm. The present invention is also directed to a method for monitoring treatment for mood disorders in a human subject, preferably for monitoring depression and MDD, said method implementing the method for diagnosing mood disorders of the present invention. Kit for determining whether a patient presents a mood disorder, preferably depression disorder, more preferably MDD, are also comprised in the present invention.

Major depressive disorder (MDD) is a very common, heterogeneous stress-related mental disorder and the most prevalent, affecting ∼10% of men and ∼20% of women worldwide. MDD is associated with a markedly increased mortality, mostly due to suicidal behavior but also increased risk of developing other medical conditions, e.g. heart disease, diabetes, and stroke. The chronic and recurrent course of MDD, associated with the treatment resistance frequently observed - the remission rate following the first treatment course is 37%- further augment the use of healthcare system. Currently, MDD is diagnosed through a clinical assessment against criteria set out in the 5th edition of Diagnostic and Statistics Manual for Mental Disorders1. MDD is characterized by episodes of at least 2 weeks during which an individual experiences a combination of five or more different symptoms, e.g. depressed mood, loss of interest in daily activities, sleep dysregulation, fatigue or indecisiveness. Hence, the principal problem is that the diagnosis of MDD is based on descriptive criteria and thus subjective by nature. Accurate diagnosis is further complicated by the heterogeneous nature of MDD, for which the most common depression scales might not overlap completely, and by its symptomatic similarity with other psychiatric and somatic disorders.

A wealth of studies have investigated putative biomarkers for MDD², which could help to diagnose disease or provide reliable substitutes for clinical response to treatment. The definition for a biomarker is "a characteristic that is objectively measured and evaluated as an indicator of normal biological processes, pathogenic processes, or pharmacologic responses to a therapeutic intervention". As such, a biomarker can be used as a diagnostic tool, as a tool for staging the disease and/or for prediction and monitoring of clinical response to an intervention. The development of biomarker(s) which could be suitable for a diagnostic test in the field of mood disorders is a complex and difficult challenge. Several biomarkers have been suggested for MDD, including circulating proinflammatory cytokines (e.g. IL6, TNFα, IL-1β) or the inflammation marker CRP, neurotrophic factors (e.g. BDNF), and hormones e.g. ACTH or cortisol (for review see³). However, when used separately none of these biomarkers fulfills the sensitivity and specificity criteria for a clinical application. Over the past decade, numerous studies have focused on brain biomarkers, including functional neuroimaging studies, or explored all the -omics field, including genomics, transcriptomics, proteomics or metabolomics.

Most promising methods seem to reside in a multi-parameter biomarker panel, for which there is a biological explanation. Those biomarkers should be quantified using a routine blood sample and standard laboratory methodology; a specific diagnostic algorithm is often developed in parallel⁴. A major issue in the identification of biomarkers suitable for a diagnostic assay in MDD is that it might require the use of a combination of several biomarkers reflecting changes in different biological mechanisms.

Accumulating evidence have indicated that RNA editing, which is mostly performed by the ADAR (adenosine deaminase acting on RNA) enzymes may be critically involved in neurological or immune diseases, among other pathologies. Inflammation is known as a pathogenic factor, possibly explaining a part of physiopathology of depression. Recent evidence supports the notion that RNA editing plays a crucial role in immune-inflammation processes It has been shown that ADAR1 regulates crucial immune responses through RNA editing. A recent study reported an overall increased RNA editing in systemic lupus erythematosus (SLE), a chronic autoimmune disorder, which could well increase the autoantigen load and promote SLE progression. By editing specific primary miRNAs that target the IL-6 receptor subunit gp130, ADAR2 controls gp130-dependent IL-6 signaling. We previously demonstrated altered RNA editing in psychiatric patients with chronic hepatitis C virus (HCV) undergoing antiviral combination therapy with IFN-α and ribavirin, in whom treatment-emergent depression is a common complication. In the CNS, RNA editing in the coding sequence of proteins key for mood regulation, including AMPA and Kainate (KA) subtypes of glutamate receptor and the serotonin receptor, can modulate the functional properties of the encoded protein products. Decreased editing of the R/G sites of AMPA receptors due to down-regulation of ADAR2 has a possible role in the pathophysiology of mental disorders. RNA editing at the Q/R site of AMPAR, an ionotropic transmembrane receptor for glutamate might be involved in the pathogenesis of mood disorders and in the action of antidepressants. We and others have identified altered serotonin receptor in the brain of patients with major depression or depressed suicide attempters. In a recent work, we have identified in the brain of suicide decedents modifications in PDE8A RNA editing as an immune response-related brain marker for suicide⁵.

In the current study, we focused on transcriptome-wide RNA editing modification by RNA-Seq on MDD patients to identify editing variants that could be used as biomarkers for a clinically useful diagnostic assay of MDD. Using measurement of transcriptome-wide RNA editing modifications we identified significant differential editing rate in a number of genes related to immune system and CNS function. Subsequently, we selected the best 15 best biomarkers for further validation on a prioritization cohort. These RNA editing variants were involved in different pathways relevant for mood disorders and MDD, e.g. immune system or neurotransmitters receptors and postsynaptic transmission. A machine-learning approach was applied to combine RNA editing variants and optimize prediction, before validating the biomarkers panel in a large cohort of 411 depressed patients and age-, race- and sex-matched control subjects. The RNA editing variants panel discriminated MDD patients from healthy controls with high specificity and sensitivity. This study is the first to evaluate multiple RNA editing variants in blood samples of MDD patients. Our findings will contribute to a better understanding of the molecular pathophysiology of MDD, and pave the way for the development of a diagnostic assay for MDD with clinical application.

In a first aspect, the present invention is directed to a method for selected at least one biomarker, preferably a combination of at least two biomarkers, which can be used to diagnose mood disorders, particularly depression disorders, more preferably major depressive disease (MDD), in a human patient from blood sample of said patient, said method comprising the step of:
a) analyzing the RNA-Seq dataset using Editome analysis pipeline and identifying A-to-I differentially edited positions with a minimum coverage of 30x, which ensures a high degree of confidence at particular base positions;
b) performing a differential analysis to identify sites whose editing could be specifically different between mood disorders, particularly depression disorders, more preferably MDD patients and healthy controls;
c) applying the following pre-specified quality criteria of the group consisting of: coverage >30; AUC>0.6; 0.95>FoldChange>1.05, p<0.05 and exclusion of intergenic sites;
d) optionally, checking that no difference in global RNA editing was observed between patients and controls, preferably by calculating the global Alu editing index (AEI), which is a measure of the overall rate of RNA editing in Alu repeats;
e) by GSEA (enrichment analysis on gene sets) on the biomarkers selecting in step c) or d), preferably by using gene ontology tools, identifying and selecting biomarkers reflecting changes in different biological process including immune and CNS (central nervous system) functions; and
f) applying the following specified quality criteria of the group consisting of: coverage >30; AUC>0.8; 0.8>FoldChange>1.20 and p<0.05) to a combination of several biomarkers selected in step e) representing different biological mechanisms, and selected those which clearly discriminated healthy controls and patients having mood disorders, particularly depression disorders, more preferably MDD, in two separate groups.

In a preferred embodiment, in step f), the combinations of at least two biomarkers are selected whether said combinations are statistically significant between control and cases with a p value <10⁻⁴.

In a second aspect, the present invention is directed to an in vitro method for diagnosing mood disorders, particularly depression disorders, more preferably MDD, in a human patient from a biological sample of said patient, said method comprising:
a) determining for one A to I editing RNA biomarker, the relative proportion of RNA editing at a given editing site for at least one or a combination of sites which can be edited on the RNA transcript of said t biomarker, and/or
   - the relative percentage of an isoform (with edited site(s)) or the non-edited isoform) or of a combination of isoforms of the RNA transcript of said biomarker;
   wherein said at least one A to I editing RNA biomarker is selected from the group of consisting of PRKCB, MDM2, PIAS1, IFNAR1, IFNAR2, LYN, AHR, RASSF1, GAB2, CAMK1D, KCNJ15, IL17RA and PTPRC biomarkers
b) determining a value resulting from the percentage(s) obtained in step a);
c) determining whether said result value or depression score obtained in step b) is greater or not greater than a control value obtained for control healthy subject, wherein the control value was determined in a manner comparable to that of the result value, obtained in step b);
wherein:
if said depression score obtained for the patient is greater than a threshold (cut-off), classifying said patient as being positive or having mood disorders, particularly depression disorders, more preferably MDD, or if said depression score is not greater than said threshold, classifying said patient as being negative or not having mood disorders, particularly depression disorders, more preferably MDD.

The criterion symptoms for mood disorders, depression disorders and MDD are well-known from the skilled person and are for example described in the DSM-5™ book from the American Psychiatric Association (2013, pages 155-188).
In the present invention, depression disorders include disruptive mood dysregulation disorder, major depressive disorder (including major depressive episode), persistent depressive disorder (dysthymia), premenstrual dysphoric disorder, substance/medication-induced depressive disorder, depressive disorder due to another medical condition, other specified depressive disorder, and unspecified depressive disorder depress.

In a preferred embodiment, the present invention is related to an in vitro method for diagnosing mood disorders, preferably depression disorders, more preferably MDD in a human patient from a biological sample of said patient, according to present invention wherein said method comprises
a) determining for a combination of at least two A to I editing RNA biomarker:
   - for each selected biomarker, the relative proportion of RNA editing at a given editing site for at least one or a combination of sites which can be edited on the RNA transcript of said at least two biomarkers, and/or
   - the relative percentage of an isoform or of a combination of isoforms of the RNA transcript of each of said two biomarkers;
   wherein said at least two A to I editing RNA biomarkers are selected from the group consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers,
b) determining a result value obtained for each of said at least two biomarkers and a final result value based on an algorithm or equation that includes the result value obtained for each selected biomarker;
c) determining the final result value or depression score obtained for the patient and a control result value obtained for a healthy control subject, wherein the control result value was determined in a manner comparable to that of the final result value; and
d) if said final result or depression score value is greater than a threshold/cut-off, classifying said patient as being positive or having mood disorders, preferably depression disorders, more preferably MDD or, if said final result value is not greater than said threshold, classifying said patient as being negative or not having mood disorders, preferably depression disorders, more preferably MDD.

For example, but non-limited to, in the method of diagnosing according to the present invention, said threshold, cut-off or depression score can be:
- the Z value calculated for healthy control patient when using mROC program, or
- the probability for a patient to be considered as having mood disorders, depression disorders or MDD.

In the present description, the term biomarker or target have the same meaning and can be used interchangeably.

In a preferred embodiment, in the method for diagnosing depression according to present invention, the combinations of at least two biomarkers are selected whether said combinations are statistically significant between control healthy control subject and cases (patients having mood disorders, depression disorders, or MDD) with a p value <10⁻⁴.

In a more preferred embodiment, in step a), said A to I editing RNA biomarker is selected from the group consisting of PRKCB, MDM2, IFNAR1, KCNJ15, LYN, CAMK1D and GAB2.

In a more preferred embodiment, when at least in step a), a combination of at least two A to I editing RNA biomarkers are used,
said at least two A to I editing RNA biomarkers are selected from the group consisting of PRKCB, MDM2, IFNAR1, KCNJ15, LYN, CAMK1D, GAB2 and PDE8A biomarkers.

In an also more preferred embodiment, the present invention is directed to an in vitro method for diagnosing mood disorders, preferably depression disorders, more preferably MDD
according to the invention, wherein in step b), the result value or depression score is calculated by an algorithm implementing a multivariate method including:
- mROC program, particularly to identify the linear combination, which maximizes the AUC (Area Under the Curve) ROC and wherein the equation for the respective combination is provided and can be used as a new virtual marker Z, as follows: Z = a.(Biomarker 1) + b.(Biomarker 2) + ...i.(Biomarker i) +....n .(Biomarker n) where i are calculated coefficients and (Biomarker i) are the level of the considered biomarker (i.e. level of RNA editing site or of isoforms for a given target/biomarker); and/or
- a Random Forest (RF) approach applied to assess the RNA editing site(s) and/or isoforms combinations, particularly to rank the importance of the RNA editing site(s) and/or isoform(s), and to combine the best RNA editing site(s) and/or isoform(s), and/or optionally
- a multivariate analysis applied to assess the RNA editing site(s) and/or isoforms combinations for the diagnostic, said multivariate analysis being selecting for example from the group consisting of:
   - Logistic regression model applied for univariate and multivariate analysis to estimate the relative risk of patient at different level of RNA editing site or isoforms values;
   - CART (Classification And Regression Trees) approach applied to assess RNA editing site(s) and/or isoforms combinations;
   - Support Vector Machine (SVM) approach;
   - Artificial Neural Network (ANN) approach;
   - Bayesian network approach;
   - WKNN (weighted k-nearest neighbours) approach;
   - Partial Least Square - Discriminant Analysis (PLS-DA);
   - Linear and Quadratic Discriminant Analysis (LDA / QDA), and
   - Any other mathematical method that combines biomarkers

In a particular preferred embodiment, said mood disorder or depression disorder which is desired to diagnose is the major depressive disorder (MDD).

In another preferred embodiment, the present invention is directed to a method for diagnosing mood disorders, particularly depression disorders, more preferably MDD according to the present invention, wherein said depression disorders include mild, moderate and severe.

In another preferred embodiment, the present invention is directed to a method for diagnosing mood disorders, particularly depression disorders, more preferably MDD according to the present invention, wherein said depression can be classified as mild, moderate or severe depression depending of the level of the difference between the depression score value obtained in step b) and the control result value obtained for the control subject.

In a preferred embodiment, in the method for diagnosing mood disorders, particularly depression disorders, more preferably MDD according to the present invention, said biological sample is whole blood, serum, plasma, urine, or cerebrospinal fluid, whole blood being the most preferred biological sample

Are preferred selected biomarkers wherein the result value or depression score obtained in step c) is statistically/specifically different from said control result value at p<0.05.

Are also preferred the methods of the present invention wherein the following criteria has to be satisfied for the biomarker or for the combination of biomarkers selected tin step a):
- the coverage >30;
- AUC>0.6;
- 0.95>FoldChange>1.05, and
- p<0.05.

Are also preferred the methods of the present invention wherein said selected A to I editing RNA biomarker(s) comprised in step a) is selected from the group consisting of:
a) a combination comprising at least 3, 4, 5, 6, 7 or 8 of the following biomarkers: PRKCB, MDM2, IFNAR1, KCNJ15, LYN, CAMK1D, GAB2 and PDE8A, preferably the combination of the cited 8 biomarkers.

Are also preferred the methods of the present invention wherein said selected A to I editing RNA biomarker comprised in step a) one or combination of at least 2, 3, 4, 5, 6, 7 or 8 selected biomarkers and wherein the calculation of the relative percentage of at least one of the RNA edition site or isoform is based on the RNA edition site or isoform listed in Table 2A (editing sites), 2B (isoforms) or 3 (editing sites) for each of the selected biomarker.

Are also preferred the methods of the present invention wherein said combination of at least 2, 3, 4, 5, 6, 7 or 8 selected biomarkers is a combination selected from the biomarkers combinations given in Table 4 and in Table 5, preferably the combinations comprising the following 8 biomarkers PRKCB, MDM2, IFNAR1, KCNJ15, LYN, CAMK1D, GAB2 and PDE8A.
Are also preferred the combinations comprising the 8 biomarkers PRKCB, MDM2, IFNAR1, KCNJ15, LYN, CAMK1D, GAB2 and PDE8A and selected from the combinations listed in Table 6.

Are also preferred the methods of the present invention wherein the algorithm or equation allowing the calculation of the result value or depression score Z are selected from the Z equations listed in the examples, preferably the equation implemented a combination of the following 8 biomarkers PRKCB, MDM2, IFNAR1, KCNJ15, LYN, CAMK1D, GAB2 and PDE8A.

In another particular aspect, the present invention concerns an in vitro method for diagnosing mood disorders, particularly depression disorders, more preferably MDD in a human patient according to the invention, wherein if the patient to be tested is a female, in step a) said at least one or combination of at least two A to I editing RNA biomarker(s) is selected from the group of biomarkers consisting of PRKCB, MDM2, PIAS1, IFNAR1, IFNAR2, LYN, AHR, RASSF1, GAB2, CAMK1D, KCNJ15, IL17RA and PTPRC, if one biomarker is selected, or said group further comprising the A to I editing RNA PDE8A if a combination of at least two biomarkers (or targets) are selected.

Are also preferred the methods of the present invention wherein said combination of at least 2, 3, 4, 5, 6, 7 or 8 selected biomarkers is a combination selected from the group consisting of PRKCB, MDM2, IFNAR1, KCNJ15, LYN, CAMK1D, GAB2 and PDE8A biomarkers (or targets), particularly the biomarkers combinations or Z equation given in the Examples or figures with patients of the female population, particularly the combinations comprising the 8 biomarkers PRKCB, MDM2, IFNAR1, KCNJ15, LYN, CAMK1D, GAB2 and PDE8A, more preferably the combinations listed in Table 7.

In another particular aspect, the present invention concerns an in vitro method for diagnosing depression in a human patient according to the invention, wherein if the patient to be tested is a male, in step a) said at least one or combination of at least two A to I editing RNA biomarker is selected from the group of biomarkers consisting of PRKCB, MDM2, PIAS1, IFNAR1, IFNAR2, LYN, AHR, RASSF1, GAB2, CAMK1D, KCNJ15, IL17RA and PTPRC, if one biomarker is selected, or said group further comprising the A to I editing RNA PDE8A if a combination of at least two biomarkers (or targets) are selected.

Are also preferred the methods of the present invention wherein said combination of at least 2, 3, 4, 5, 6, 7 or 8 selected biomarkers is a combination selected from the group consisting of PRKCB, MDM2, IFNAR1, KCNJ15, LYN, CAMK1D, GAB2 and PDE8A biomarkers (or targets), particularly the biomarkers combinations or Z equation given in the Examples or figures with patients of the male population, particularly the combinations comprising the 8 biomarkers PRKCB, MDM2, IFNAR1, KCNJ15, LYN, CAMK1D, GAB2 and PDE8A, more preferably the combinations listed in Table 9.

In a more preferred embodiment, the present invention is directed to a method in vitro for diagnosing depression in a human patient according to the present invention, wherein the combination of biomarkers and/or the Z equation or algorithm associated with said combination used for the mood disorders, particularly depression disorders, more preferably MDD diagnostic is different whether the patient to be tested is a female or a male.

Preferably, the combination of biomarkers and/or the Z equation whether the patient to be tested is a female or a male is selected from the combination and/or the Z equation depicted in the examples or in the figures for this particular case.

Are also preferred the methods of the present invention, wherein in step a) the relative proportion of RNA editing at a given editing and/or the percentage of an isoform are measuring by NGS in said biological sample.

Are also preferred the methods of the present invention, wherein in step a), the amplicon/nucleic sequence used for the detection of the RNA editing sites and/or the isoforms of the RNA transcript of said biomarker is obtained or obtainable with the set of primers listed in Table 1 for each of the selected biomarkers (SEQ ID NO.1 to 36). Are also preferred the methods of the present invention, wherein in step a), the amplicon/nucleic sequence used for the detection of the RNA editing sites and/or the isoforms of the RNA transcript of said biomarker is obtained or obtainable with a set or a combination of sets of primers allowing to obtain amplicon(s)or nucleic acid sequence(s) including, or identical to, the amplicon(s)or nucleic acid sequence(s) obtainable by the set of primers listed in Table 1 (SEQ ID No.1 to SEQ ID No.36). Said amplicon or nucleic acid sequence used for the detection of the RNA editing sites and/or the isoforms of the RNA transcript of said biomarker could be the RNA transcript complete sequence itself.

In another aspect, the present invention is directed to a method for monitoring treatment for mood disorders, preferably depression disorders, more preferably MDD in a human subject from a blood sample of said subject, preferably for monitoring MDD, said method comprising:
A) diagnosing mood disorders, preferably depression disorders, more preferably MDD depression in said human by the method for diagnosing mood disorders, preferably depression disorders, more preferably MDD in a human patient according to the present invention before beginning of the treatment which is desired to be monitored.
B) repeating steps (a) to c) of the method for diagnosing mood disorders, preferably depression disorders, more preferably MDD in a human patient according to the present invention, after a period of time during which said patient receives treatment for said depression, to obtain a post-treatment depression score;
C) comparing the post-treatment depression score from step (c) to:
   - the depression score (i.e. Z value, probability value) obtained before the period of time during which said patient receives treatment for said depression, and
   - to the depression score (control final value) for normal/healthy subjects, and classifying said treatment as being effective if the post-treatment depression score obtained in step B) is closer than the depression score obtained before the period of time during which said patient receives treatment to the depression score obtained for normal/healthy subjects.

In another aspect, the present invention is directed to a kit for determining whether a patient present a depression, preferably a MDD, said kit comprising:
1) instructions to apply the method according to one claims 1 to 20, in order to obtain the result value or depression score the analysis of which determining whether said patient present a mood disorder, preferably depression disorder, more preferably a MDD; and
2)
   - one set of primers from the group of pairs of primers consisting of SEQ ID No.1 to SEQ ID No.4 and SEQ ID No.7 to SEQ ID No.36, the selection of which being dependent of the biomarker used for the depression diagnostic; or
   - a combination of at least two set of primers from the group of pairs of primers consisting of: SEQ ID No.1 to SEQ ID No.36, the selection of which being dependent of the biomarkers combination used for the mood disorder, preferably depression disorder, more preferably MDD diagnostic;
      or
   - a set or a combination of sets of primers allowing to obtain amplicon(s) including, or identical to, the amplicon(s) obtainable by the set of primers listed in Table 1 (SEQ ID No.1 to SEQ ID No.36).

The following examples and the figures and the legends hereinafter have been chosen to provide those skilled in the art with a complete description in order to be able to implement and use the present invention These examples are not intended to limit the scope of what the inventor considers to be its invention, nor are they intended to show that only the experiments hereinafter were carried out.
Other characteristics and advantages of the invention will emerge in the remainder of the description with the Examples, Figures and Tables, for which the legends are given herein below.

### BRIEF DESCRIPTION OF THE DRAWINGS AND THE TABLES

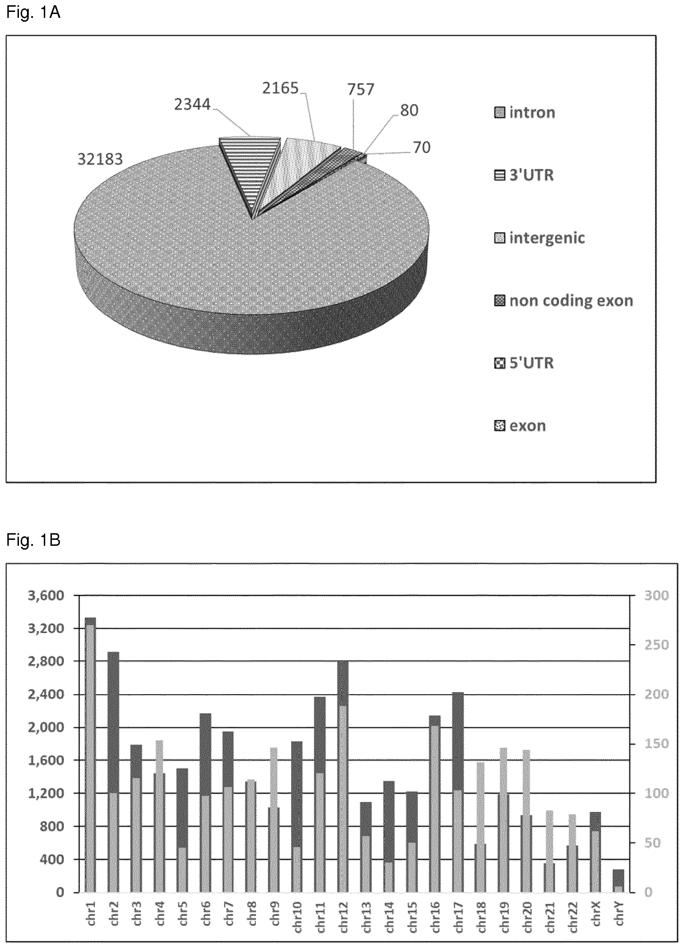

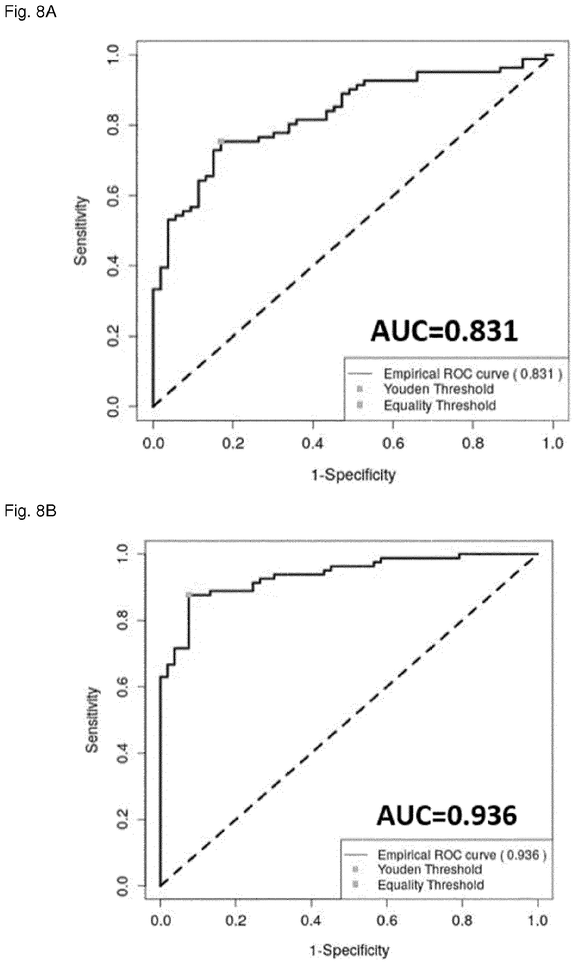

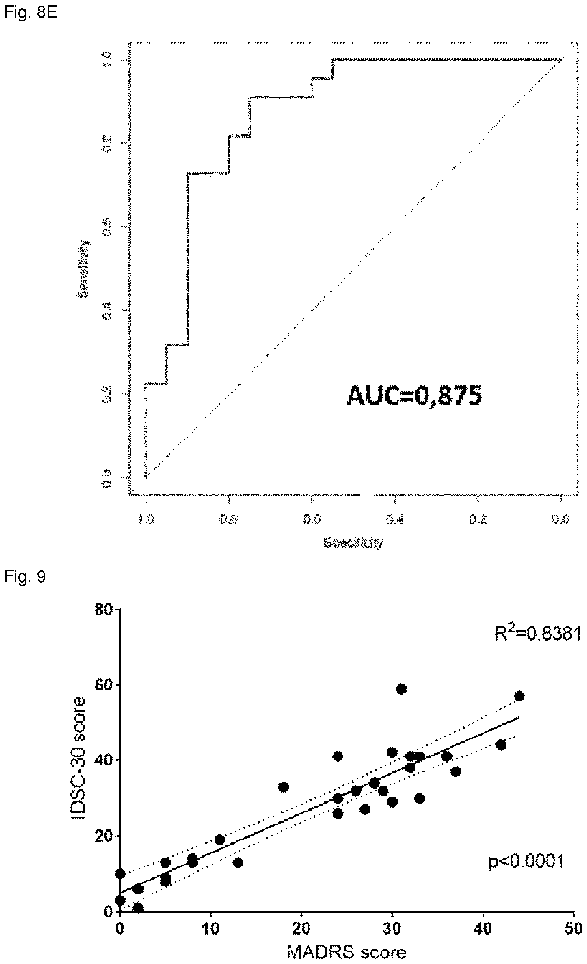

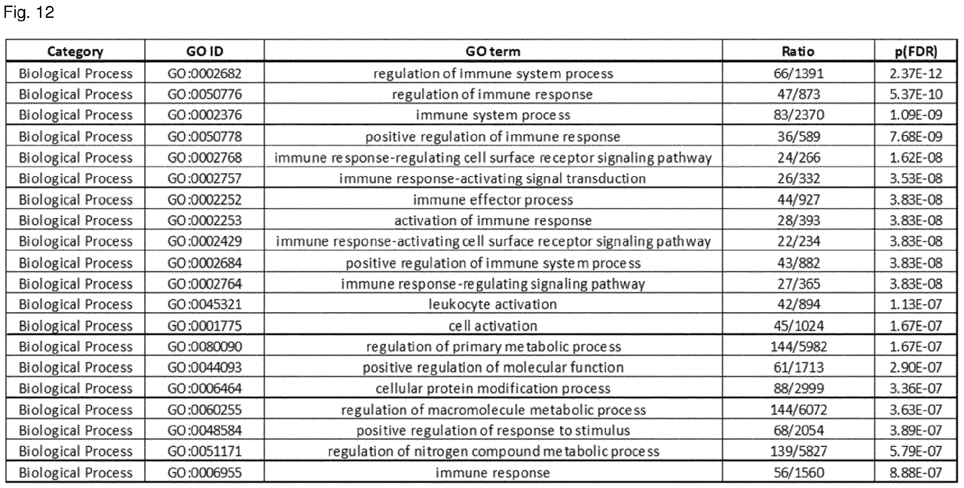

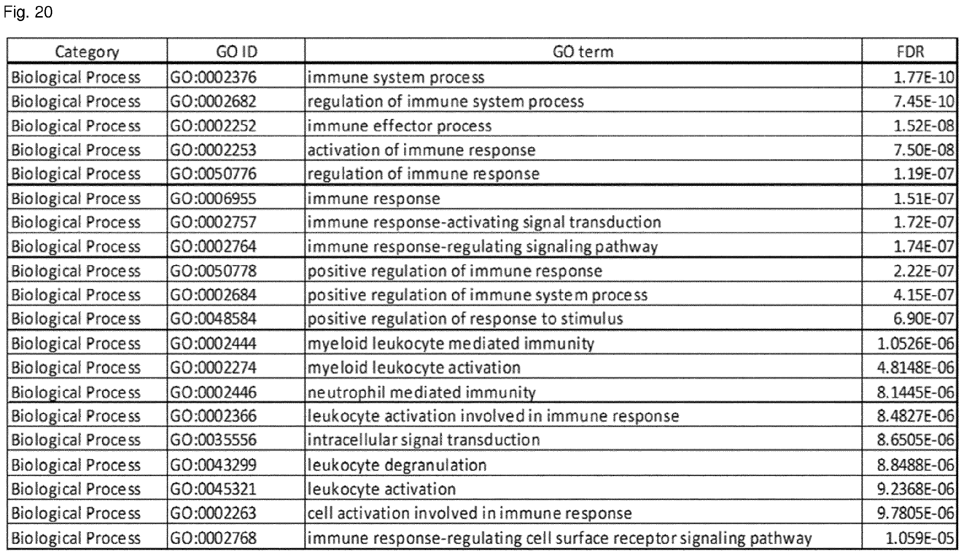

**Figures 1A-1D****:** Identification and validation of differentially A-to-I RNA editing sites between healthy controls and MDD patients using RNA-seq data from human blood.
   A: Genomic localization of detected editing sites
   B: Repartition of A-to-I editing events or edited genes by chromosome.
      Dark grey (left y-axis): Repartition of A-to-I editing events identified with a minimum coverage of 30 in the RNA-Seq study.
      Light grey (right y-axis): Repartition of edited genes identified with a minimum coverage of 30 in the RNA-Seq study
   C: Volcano plot of differentially edited sites between controls and MDD patients. The volcano plot shows the upregulated and downregulated differentially edited genes sites between depressed patients (MDD) group and the control group. For each plot, the x-axis represents the log (2)(fold change) (FC), and the y-axis represents -log 10(p values). Editing sites with an adjusted p value of less than 0.05 AND FC>5% were assigned as differentially edited and are indicated in green.
   D: Alu Editing index between controls and depressed patients. Distribution of Alu editing index (AEI) values in controls and MDD patients. Data are the mean ± SEM. p-value of editing index was calculated using the Wilcoxon rank-sum test.
**Figure 2****:** Principal component analysis of the 646 variants in 366 genes differentially edited between depressed patients and healthy controls identified in the RNA-Seq study.
   The scatter plot visualizes the first, second and third principal components and respective variance percentages on the x-, y- and z-axis of ratio (fold change) of biomarkers. grey circles: MDD patients; black circle: Healthy Controls.
**Figure 3****:** Protein-protein associations in the 14 differentially edited genes used in the prioritization cohorts.
   Diagram shows the protein-protein interactions in the cluster of 14 genes selected in the prioritization cohort. Genes were annotated and colored regarding to their involvement in Biological processes.
   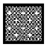 : regulation of immune system process (GO:0002682) ;
   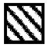 : receptor signaling pathway via JAK-STAT (GO:0007259) ;
   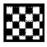 : regulation of immune response (GO:0050776)

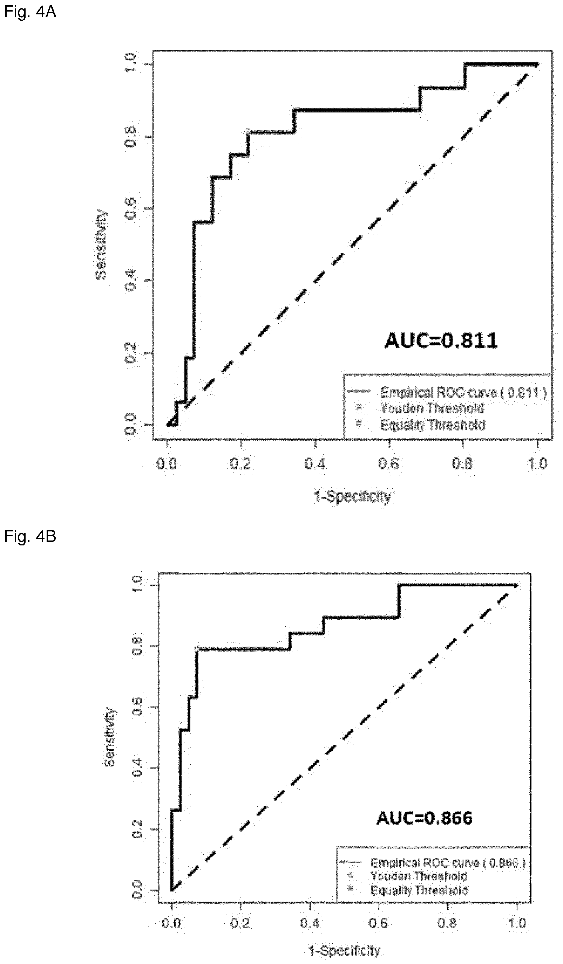

**Figures 4A-4D****:** Examples of diagnostic performance of mRNA editing of the first prioritization cohort (n=76) analyzed by single-plex.
   A: Example of diagnostic performances obtained by using 4 RNA editing sites in 3 different targets.
   B: Example of diagnostic performances obtained by using 4 RNA editing sites in 2 different targets.
   C: Example of diagnostic performances obtained by using 6 RNA editing sites in 4 different targets.
   D: Example of diagnostic performances obtained by using 29 RNA editing sites present in 5 different targets.

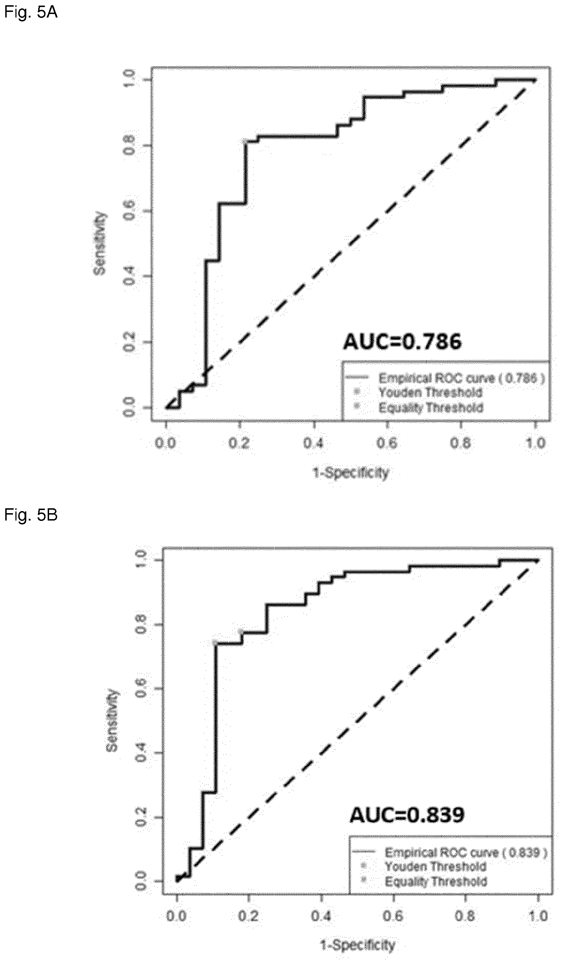

**Figures 5A-4F**: Diagnostic performance of mRNA editing of the second prioritization cohort (n=86) analyzed by multiplex sequencing.
   A: Example of diagnostic performances obtained by using 2 RNA editing sites in 3 different targets.
   B: Example of diagnostic performances obtained by using 3 RNA editing sites in 3 different targets.
   C: Example of diagnostic performances obtained by using 4 RNA editing sites in 4 different targets.
   D: Example of diagnostic performances obtained by using 5 RNA editing sites present in 4 different targets.
   E: Example of diagnostic performances obtained by using 19 RNA editing sites in 8 different targets.
   F: Example of diagnostic performances obtained by using 48 RNA editing sites present in 9 different targets.

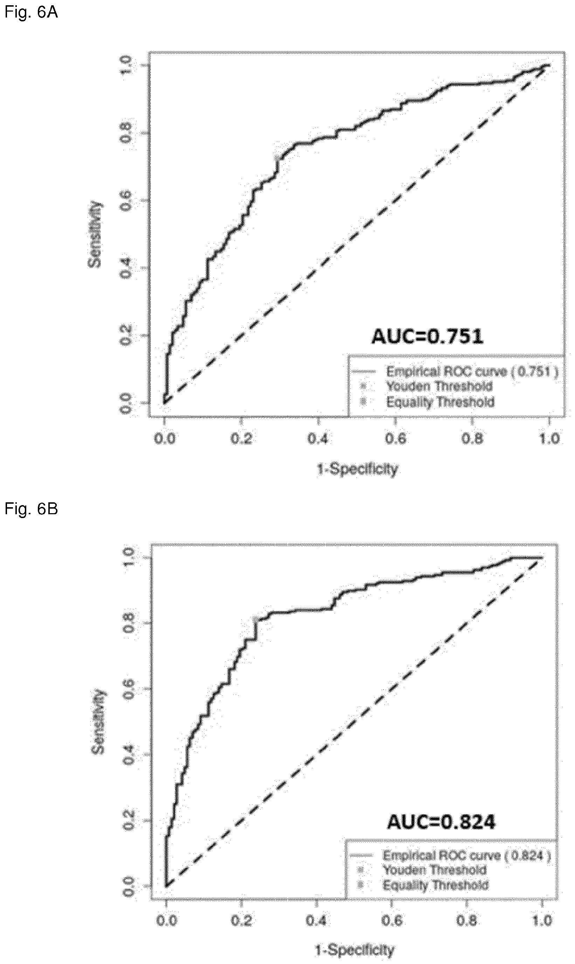

**Figures 6A-6C****:** Diagnostic performance of mRNA editing of the validation cohort (n=411) analyzed by multiplex sequencing of 8 targets.
   A: Example of diagnostic performances obtained by using 17 RNA editing sites in 5 different targets.
   B: Example of diagnostic performances obtained by using 53 RNA editing sites in 8 different targets.
   C: Example of diagnostic performances obtained by using 79 RNA editing sites in 8 different targets.

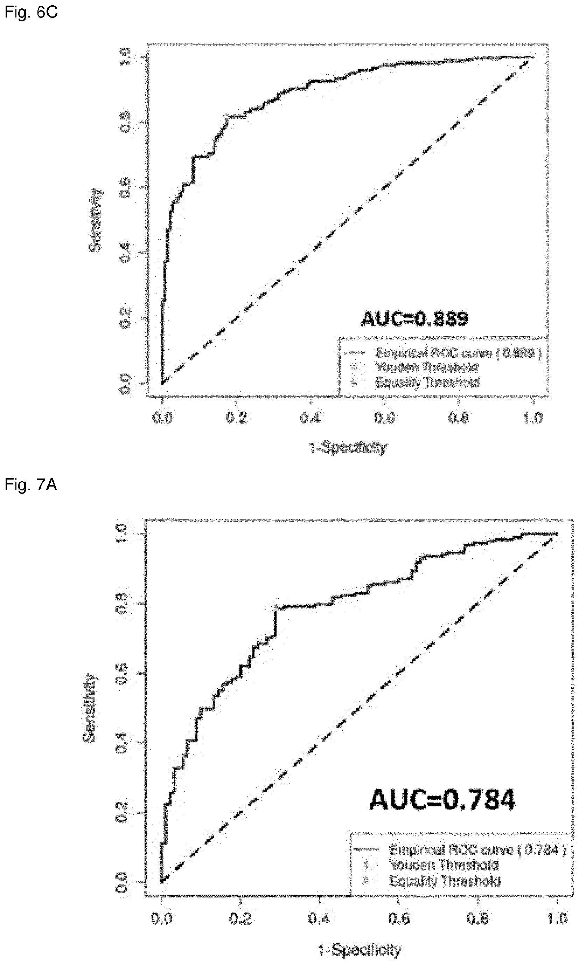

**Figures 7A-7E****:** Diagnostic performance of mRNA editing of the female population of the validation cohort (n=277) analyzed by multiplex sequencing of 8 targets.
   A: Example of diagnostic performances obtained by using 11 RNA editing sites in 4 different targets.
   B: Example of diagnostic performances obtained by using 30 RNA editing sites in 6 different targets.
   C: Example of diagnostic performances obtained by using 49 RNA editing sites in 8 different targets.
   D: ROC curve obtained after machine-learning with the mROC method.
   E: ROC curve obtained after machine-learning with the RandomForest method
**Figures 8A-8E****:** Diagnostic performance of mRNA editing of the male population of the validation cohort (n=134) analyzed by multiplex sequencing of 8 targets.
   A: Example of diagnostic performances obtained by using 8 RNA editing sites in 6 different targets.
   B: Example of diagnostic performances obtained by using 39 RNA editing sites in 7 different targets.
   C: Example of diagnostic performances obtained by using 68 RNA editing sites in 8 different targets.
   D: ROC curve obtained after machine-learning with the mROC method.
   E: ROC curve obtained after machine-learning with the RandomForest method
**Figure 9****:** Correlation between clinical MADRS and IDS-C30 scores for the subjects included in the RNA-Seq study.
   Graph shows the 95% Intervals confidence. The Pearson correlation coefficient and p value are indicated.

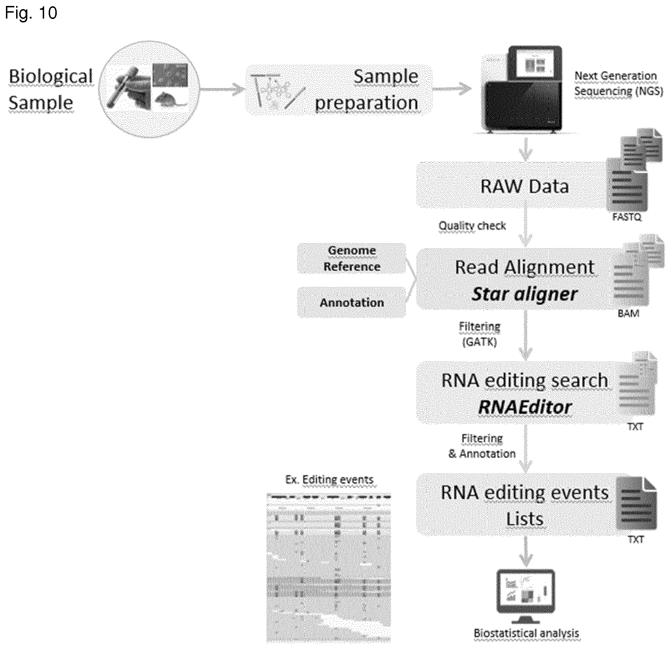

**Figure 10****:** Overview of the Editome analysis pipeline

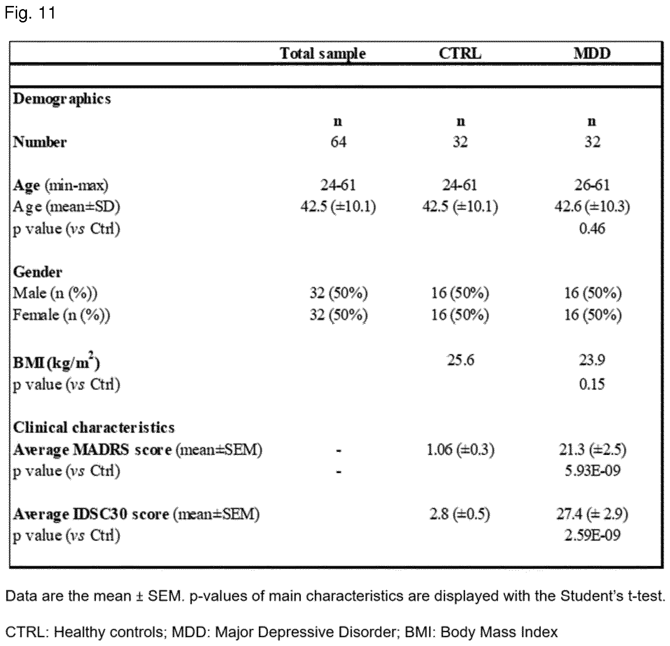

**Figure 11****:** Demographic characteristics and psychiatric diagnosis of the study population included in the RNA-Seq study.
   Data are the mean ± SEM. p-values of main characteristics are displayed with the Student's t-test. CTRL: Healthy controls; MDD: Major Depressive Disorder; BMI: Body Mass Index
**Figure 12****:** Functional categorization of the 366 genes differentially edited between MDD and controls based on gene ontology (GO) annotations.
   Shown are the results of top 20 GO over-representation tests for enrichment of Biological Process GO terms with differentially edited genes. The input into the GO enrichment analysis tools was the list of 366 genes differentially edited between MDD and controls. The ratio of the number of genes/total number of genes included in the GO term and p-values corrected using the Benjamini-Hochberg method for multiple testing are presented. FDR, false discovery rate; GO, gene ontology.

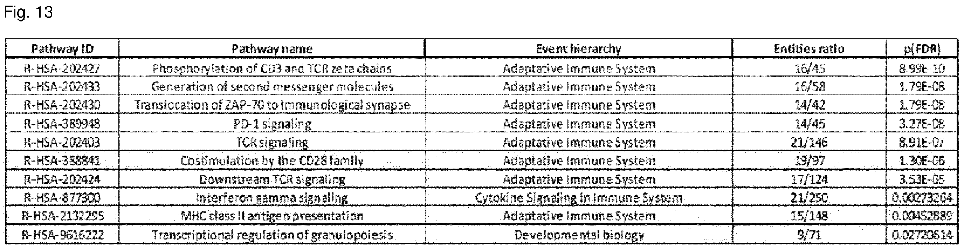

**Figure 13****:** Signaling pathway enrichment analysis of the 366 genes differentially edited between MDD and controls.
   Table 3 shows the significant pathways obtained performing over-representation analysis in Reactome. The ratio of the number of genes/total number of genes included in the pathway and p-value corrected using the Benjamini-Hochberg method for multiple testing are shown.

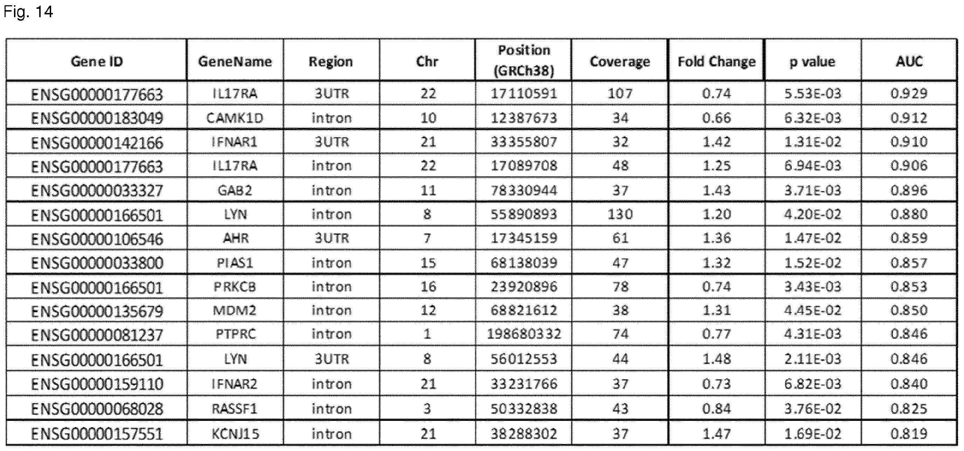

**Figure 14****:** Gene and RNA editing variants analyzed in the prioritization cohort.
   Shown are the gene and position (GRCh38) of the editing variant, coverage following RNA-Seq, Fold Change between controls and MDD patients, p value and Area under the Curve calculated from RNA-Seq data. p-values were calculated using the Wilcoxon rank-sum test.

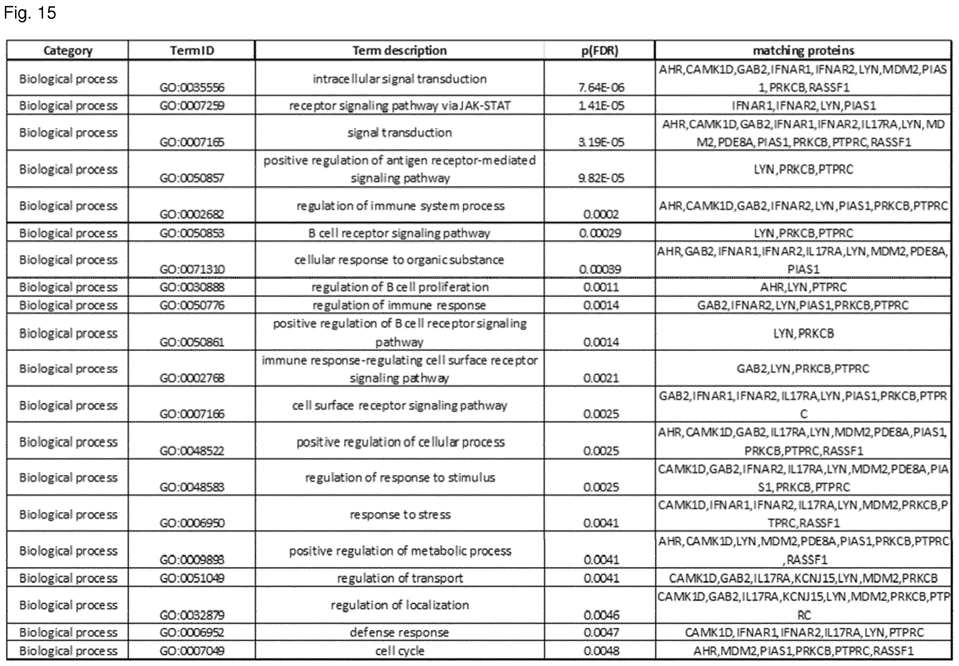

**Figure 15****:** Functional categorization of the 14 differentially edited genes used in the prioritization cohorts. based on gene ontology (GO) annotations.
   Shown are the results of top 20 GO over-representation tests for enrichment of Biological Process GO terms with differentially edited genes. The input into the GO enrichment analysis tools was the list of 14 genes differentially edited between MDD and controls analyzed in the prioritization cohorts. p-values corrected using the Benjamini-Hochberg method for multiple testing are presented. FDR, false discovery rate; GO, gene ontology.

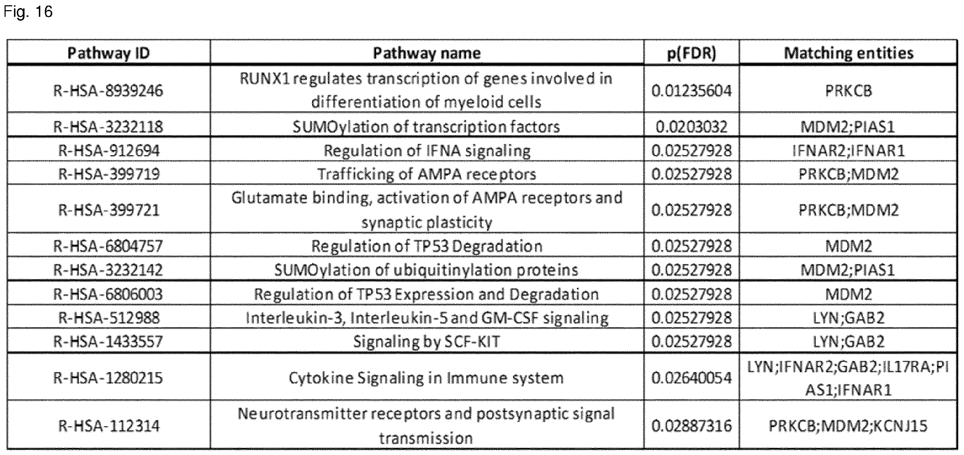

**Figure 16****:** Signaling pathway enrichment analysis of the 14 genes analyzed in the prioritization cohorts.
   Table shows the significant pathways obtained performing over-representation analysis in Reactome. p-value corrected using the Benjamini-Hochberg method for multiple testing are shown.

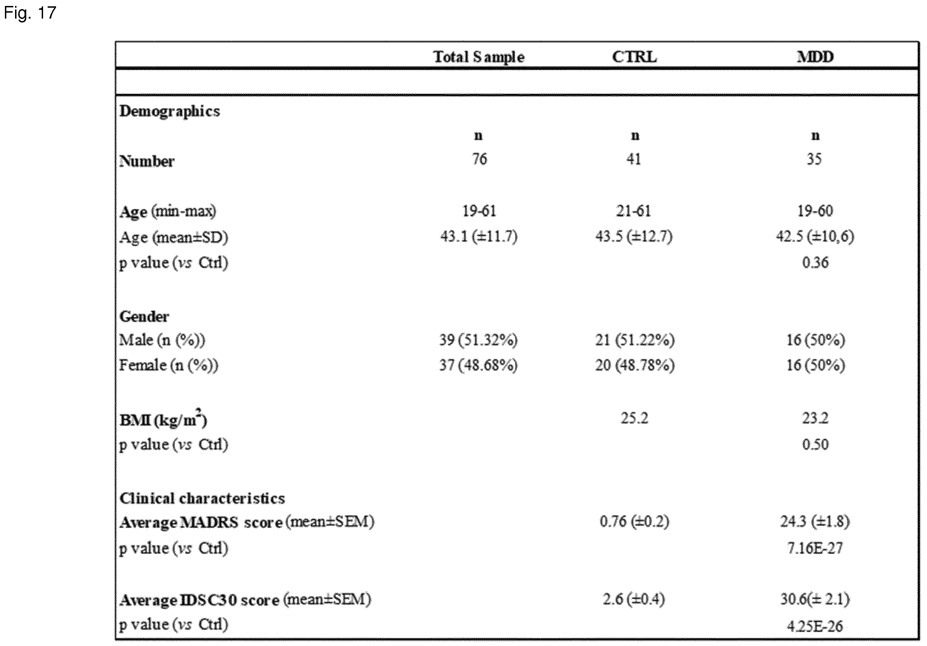

**Figure 17****:** Demographic characteristics and psychiatric diagnosis of the study population included the first prioritization study analyzed in single-plex.
   Data are the mean ± SEM. p-values of main characteristics are displayed with the Student's t-test. CTRL: Healthy controls; MDD: Major Depressive Disorder; BMI: Body Mass Index
   Testing are shown.

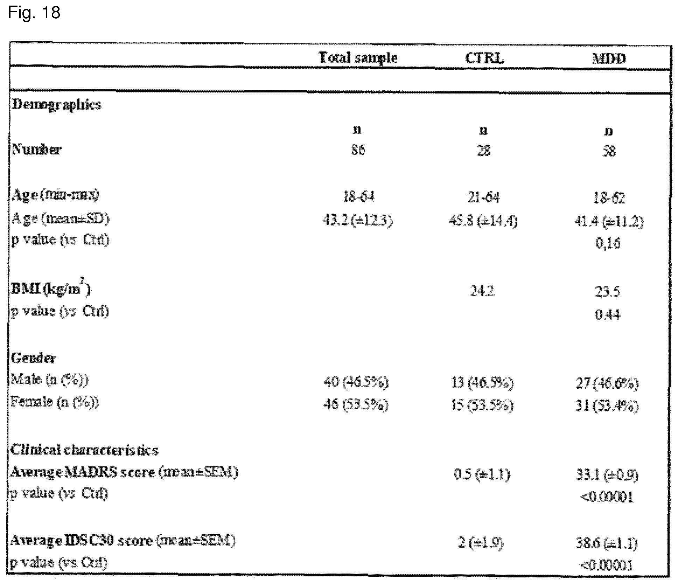

**Figure 18****:** Demographic characteristics and psychiatric diagnosis of the study population included the second prioritization study analyzed by with multiplex sequencing.
   Data are the mean ± SEM. p-values of main characteristics are displayed with the Student's t-test. CTRL: Healthy controls; MDD: Major Depressive Disorder; BMI: Body Mass Index.

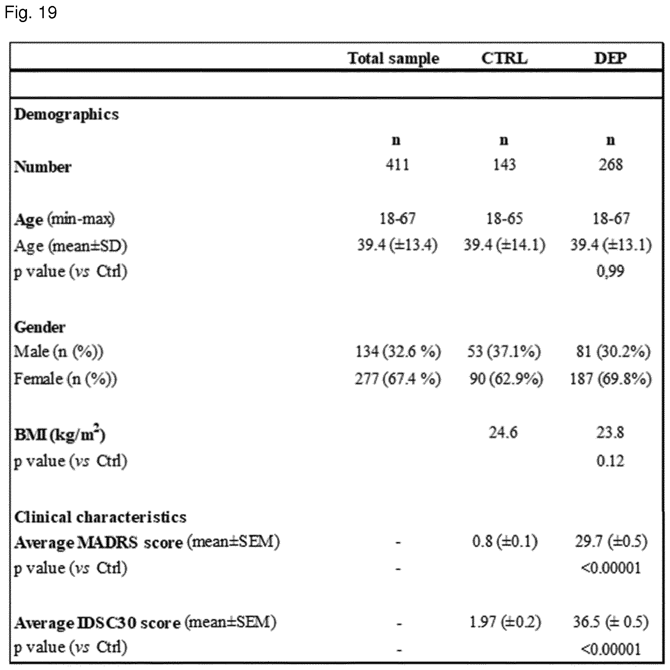

**Figure 19****:** Demographic characteristics and psychiatric diagnosis of the study population included in the validation cohort.
   Data are the mean ± SEM. p-values of main characteristics are displayed with the Student's t-test. CTRL: Healthy controls; MDD: Major Depressive Disorder; BMI: Body Mass Index
**Figure 20****:** Functional categorization of the 366 genes differentially edited between MDD and controls based on g:Profiler annotations.
   Shown are the results of top 20 GO over-representation tests for enrichment of Biological Process GO terms with differentially edited genes. The input into the g:Profiler was the list of 366 genes differentially edited between MDD and controls. The ratio of the number of genes/total number of genes included in the GO term and p-values corrected using the Benjamini-Hochberg method for multiple testing are presented. FDR, false discovery rate; GO, gene ontology.

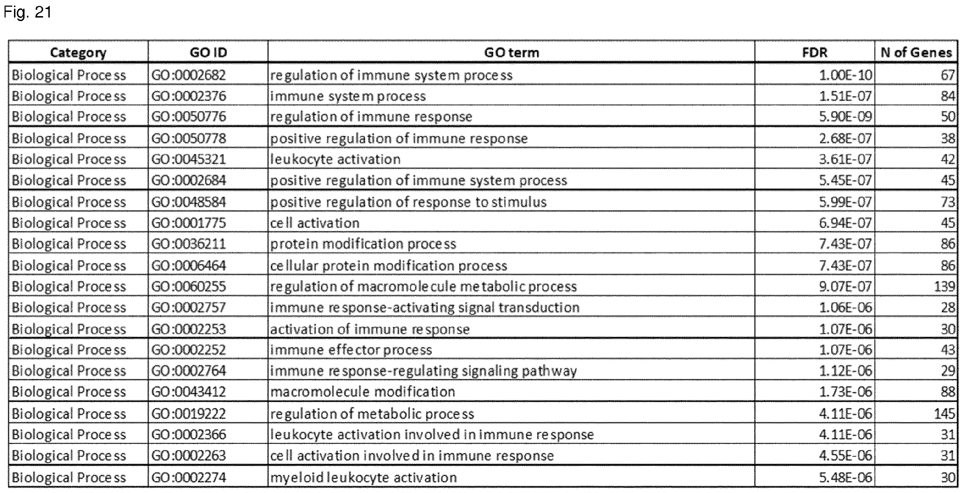

**Figure 21****:** Functional categorization of the 366 genes differentially edited between MDD and controls based on GOnet annotations.

Shown are the results of top 20 GO over-representation tests for enrichment of Biological Process GO terms with differentially edited genes. The input into the GOnet was the list of 366 genes differentially edited between MDD and controls. The ratio of the number of genes/total number of genes included in the GO term and p-values corrected using the Benjamini-Hochberg method for multiple testing are presented. FDR, false discovery rate; GO, gene ontology.

### EXAMPLE 1: Materials and Methods

### Subjects and clinical assessment

Depressed patients (n=268) were recruited from the outpatients of the Department of Emergency Psychiatry and Post-Acute Care (CHRU of Montpellier) according to the principles of the Helsinki Declaration of 1975 and its successive updates. This study was approved by the French local Ethical Committee (CPP Sud-Méditerranée IV in Montpellier, CPP No.A01978-41). All participants, aged between 18 and 65 years, understood and signed a written informed consent before entering the study. The study was approved by the local Institutional Review Board, according to the approval requirements and good clinical practice. All patients met the MDD criteria in Diagnostic and Statistical Manual of Mental disorders IV (DSM-IV) using the Mini-International Neuropsychiatric Interview. The presence or absence of psychiatric diagnoses and mood states at time entering the study were confirmed by trained psychiatrists. During the standardized interview, psychiatrists managed the French version of the Montgomery-Asberg Depression Rating Scale (MADRS) and the 30-item Inventory of Depressive Symptomatology, Clinician Rated (IDS-C30) to score depression. MDD patients, all Caucasian, were recruited among the outpatients of the Department of Emergency Psychiatry Post-Acute Care (CHRU of Montpellier). Age-, race- and sex-matched control subjects (n=143) were recruited from a list of volunteers from the Clinical Investigation Center (CHRU of Montpellier).

### RNA extraction and qualification from whole blood

A volume of 4 ml of whole blood from each patient was retrieved in PAXgene™ blood RNA tubes, which are optimized for stabilization of RNA, and stored at -20°C before being transferred to -80°C. Samples were distributed randomly in the different sets of extractions. Blood samples were thawed at 4°C overnight and centrifuged 10 min at 3000g. The pellet was washed with 4ml of DPBS 1X, resuspended in 400µl of DPBS 1X. Samples total RNAs were isolated using the MagNA Pure 96 Cellular RNA Large Volume Kit (LifeScience), according to the manufacturer's automated protocol. RNAs were eluted in 100µl MagNA Pure 96 Elution Buffer. During sample preparation and RNA extraction, standard precautions were taken to avoid RNA degradation by RNAses. Total RNA concentrations and quality levels were determined with Qubit Fluorometer (Invitrogen) and LabChip (Perkin-Elmer, HT RNA Reagent Kit) instruments, respectively.

### Library preparation for RNA-Sequencing

For RNA library preparation from blood derived RNAs, we chose a specific strategy aiming at depleting in a single step both ribosomal RNA and globin RNAs, which are two forms of abundant RNA, in order to enrich the library with total RNAs (mRNAs). Total RNAs were preferred to messenger RNAs, as it has been shown that RNA editing in blood cells was enriched in intronic region of repetitive elements, which are removed during splicing to produce messenger RNAs. We thus used a TruSeq Stranded Total RNA library kit (Illumina) with Ribo-Zero Globin, which is specifically tailored for blood samples, according to the manufacturer's instructions. Briefly, 300ng total RNAs were depleted in ribosomal RNA and globin RNAs using rRNA and globin depletion probes in combination with magnetic beads. Following purification, the RNAs were fragmented into 250bp fragments in average using divalent cations under elevated temperature. First strand cDNA synthesis was then achieved using Superscript II Reverse Transcriptase (Thermo Fisher Scientific) and random primers. Next, second strand cDNA synthesis is performed using DNA Polymerase I and RNase H. Importantly, during second strand cDNA synthesis dUTP is incorporated in place of dTTP to generate a strand-specific library. Following second strand cDNA synthesis, the 3' ends of the blunt fragments are 3' adenylated before adapter ligation. Importantly, each index has been attributed to all groups to prevent putative bias due to unspecific effect of index on the depth of sequencing. DNA fragments having adapter molecules on both ends were then amplified by PCR to increase the amount of DNA in the library. Quality controls were then performed using both Qubit Fluorometer (Invitrogen) and LabChip (Perkin-Elmer, HT DNA 5K Reagent Kit) instruments for quantification of the DNA library templates and quality control analysis, respectively. Samples were then pooled into a library and purified using Magbio PCR cleanup system. The library was denatured using 0.1 M NaOH and sequenced on an Illumina NextSeq 500/550 High-Output run using paired-end chemistry with 75-bp read length.

### Processing of RNA-Seq data, differential expression analysis and editome analysis

Paired-end reads were generated using an Illumina NextSeq 500 and demultiplexed using bcl2fastq (version 2;17.1.14, Illumina). Sequencing quality was performed using FastQC software (version 0.11.7, https://github.com/s-andrews/FastQC). Read alignments were done using the human genome version hg38 with STAR aligner⁶, following by realignment by Genome Analysis Tool Kit (GATK version 4.1.4.0)⁷ removal of PCR duplicates and final single nucleotides variants (SNV) calling. Identification and quantification of A-to-I editing events was performed using RNAEditor (Version 1.0)⁸. Further filtering and functional annotation of edited events was then performed with ANNOVAR⁹, RepeatMasker¹⁰ and BiomaRt¹¹.

### Targeted Next Generation Sequencing Library preparation and sequencing

For Next Generation Sequencing (NGS) library preparation, we chose a multiplexed targeted approach to selectively sequence the region of interest within each relevant target. Validated PCR primers were used to amplify the region of interest by PCR (Table 20). For PCR amplification, the Q5 Hot Start High Fidelity enzyme (New England Biolabs) was used according to manufacturer guidelines. The PCR reaction was performed on a Peqstar 96x thermocycler using optimized PCR protocol. Both quantity and quality of the PCR product were assessed. Purity of the amplicon was determined with Nucleic Acid Analyzer (LabChipGx, Perkin Elmer) and quantification was performed using the fluorescence-based Qubit method. After quality control, the PCR reactions were purified using magnetic beads (High Prep PCR MAGbio system, Mokascience). DNA was then quantified using Qubit system and purification yield was calculated. Next, samples were individually indexed by PCR amplification using Q5 Hot start High fidelity PCR enzyme and the Illumina 96 Indexes kit (Nextera XT index kit; Illumina). Samples were then pooled into a library and purified using Magbio PCR cleanup system. The library was denatured using 0.1M NaOH and loaded onto a sequencing cartridge (Illumina MiSeq Reagent Kit V3 or Illumina NextSeq 500/550 Mid-Output) according to Illumina's guidelines. A commercial total RNA pool from human blood peripheral leukocytes (Clontech, #636592) was incorporated into the libraries to determine variability between different sequencing flow cells during the course of the experiment. NGS libraries were spiked in to introduce library diversity using PhiX Control V3 (Illumina) and sequenced (single-read sequencing, read length 150bp) at standard concentrations using deep sequencing (>50K sequences per sample).

### Bioinformatics analysis of targeted sequencing data

The sequencing data were downloaded from the NextSeq or MiSeq sequencers (Illumina). Sequencing quality was performed using FastQC software (version 0.11.7 https://github.com/s-andrews/FastQC/). A minimal sequencing depth of 20 000 reads for each sample was considered for further analysis. A pretreatment step was performed consisting of removing adapter sequences and filtering of the sequences according to their size and quality score. Short reads (<100nts) and reads with an average QC<20 were removed. To improve sequence alignment quality of the sequences, flexible read trimming and filtering tools for Illumina NGS data were used (fastx_toolkit v0.0.14 and prinseq version 0.20.4). After performing pre-processing steps, an additional quality control of each cleaned fastq file was carried out prior further analysis.

Alignment of the processed reads was performed using bowtie2¹² (version 2.2.9) with end-to-end sensitive mode. The alignment was done to the latest reference human genome sequence (GRCh38). Non-unique alignments, reads, unaligned or reads containing insertion/deletion (INDEL) were removed from downstream analysis by SAMtools software¹³ (version 1.7). SAMtools mpileup¹⁴ was used for SVN calling. Finding edited position in the alignment was done by using in-house script's in order to count the number of different nucleotides in each genomic location. For each position the percentage of reads supporting nucleotide 'G' instead of nucleotide 'A' is calculated [Number of 'G' reads/ (Number of 'G' reads + Number of 'A' reads)*100]. An editing rate > 0.1 are automatically detected by the script and are considered as 'A-to-I edition site'. The last stage is to compute the percentage of all possible isoforms of each transcript. By definition the relative proportion of RNA editing at a given editing 'site' represents the sum of editing modifications measured at this unique genomic coordinate. Conversely, an mRNA isoform is a unique molecule that may or may not contain multiple editing modifications on the same transcript. For example for a given transcript, the mRNA isoform BC contains an A-to-I modification on both site B and site C within the same transcript. A relative proportion of at least 0.1% was set as the threshold in order to be included in the analysis.

### Statistical analysis of data

All statistics and figures were computed with the "R/Bioconductor" statistical open source software¹⁵ or GraphPad Prism software (version 7.0). Biomarkers (i.e RNA editing sites and isoforms of target genes and mRNA expression of ADARs) values are usually presented as mean ± standard error of the mean (SEM). In order to guarantee normally distributed data, all biomarker could be transformed using bestNormalize R package (version 1.4.2). A differential analysis was carried out using the most appropriate test between the Mann-Whitney rank-sum test, Student's t-test or Welch's t-test according to normality and sample variance distribution. A p-value below 0.05 was considered as statistically significant.

The biomarker diagnostic performance could be characterized by: sensitivity, which represents its ability to detect the 'depressed' group and specificity which represents its ability to detect the 'control' group. The results of the evaluation of a diagnostic test can be summarized in a 2x2 contingency table comparing these two well-defined groups. By fixing a cut-off, the two groups could be classified into categories according to the results of the test, categorized as either positive or negative. Given a particular biomarker, one can identify a number of A patients with a positive test result among the depressed group (the "True Positive": TP) and B patients with a negative test result among the 'control' group (the "True Negative": TN). In the same fashion, C patients with a negative test result among the 'depressed' group (the "False Negative": FN) and D patients with a positive test result among the 'control' group (the "False Positive": FP) are observed. Sensitivity is defined as TP/(TP+FN); which is herein referred to as the "true positive rate". Specificity is defined as TN/(TN+FP); which is herein referred to as the "true negative rate".

The accuracy of each biomarker and its discriminatory power was evaluated using a Receiving Operating Characteristics (ROC) analysis¹⁶. ROC curves are the graphical visualization of the reciprocal relation between the sensitivity (Se) and the specificity (Sp) of a test for various values. In addition, all biomarkers were combined with each other to evaluate the potential increase in sensibility and specificity using multiple approaches as for example mROC program¹⁷ or RandomForest¹⁸.

mROC is a dedicated program to identify the linear combination, which maximizes the AUC (Area Under the Curve) ROC. The equation for the respective combination is provided and can be used as a new virtual marker Z, as follows:
Z =a X biomarker 1 + b X biomarker 2 + c X biomarker 3
where a, b and c are calculated coefficients and biomarkers 1,2 and 3 are the level of the considered biomarker.

Random Forest (RF) was applied as previously to assess the RNA editing isoforms/sites combinations. This method combines Breiman's "bagging" idea and the random selection of features in order to construct a collection of decision trees with controlled variance. Random forests can be used to rank the importance of editing isoforms/sites and to combine the best isoforms. Multiple down-sizing (MultDS) asymmetric bagging with embedded variable classifier was implemented. This method tries to take advantage of the whole information of majority calls by multiple down sampling but keeping the minority class fix. Our algorithm generated 100 random forest from the training set, each containing the same fix number of the minority class and equal or adjusted number of the randomly sampled, generating nearly balanced trees. For classification of new data, the results afterwards were combined by majority voting of the trained 100 random forest. To estimate RF parameters for each trained 100 randomForest, a 10-fold cross-validation were applied. The implementation was done using the R randomForest package (version 4.6-14) and R caret package (version 6.0-84).

### EXAMPLE 2: Results

### Characteristics of the Discovery Study Samples

Patients and control subjects included in the RNA-Seq samples (n=32 for each condition) were matched with respect to all variables, e.g. sex, age, or BMI, which were not statistically different between groups (Figure 11). We chose two different clinical evaluations to confirm the presence or absence of psychiatric diagnoses, namely MADRS and IDSC-30, both designed by the American Psychiatry Association Diagnostic and Statistical Manual of Mental Disorders. Healthy controls did not show any sign of depressed mood at the time on sample collection (MADRS<7 and IDSC-30<10). Depressed patients (MDD) had significantly higher scores in both MADRS (p-value 5.93E-09) and IDSC-30 (p-value 2.59E-09) (Table 1). Moreover, we observed a significant association (r2=0.84, p<0.0001) between the two clinical evaluations (Figure 9), suggesting a correct assignment of patients and a relative homogeneity in the scoring of depression.

### Editome analysis

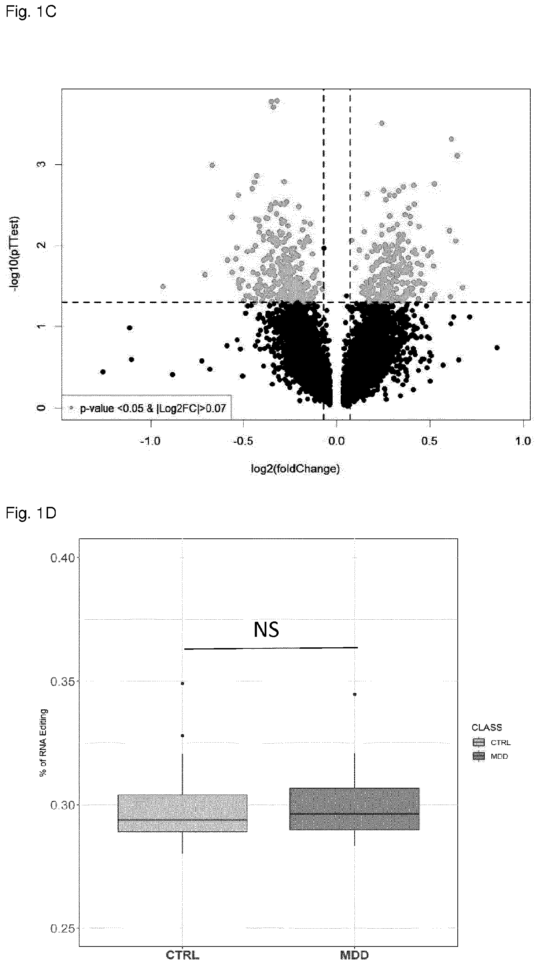

To obtain a global landscape of the modification of RNA editing events in depressed patients, we analyzed the RNA-Seq dataset using an in-house editome analysis pipeline (see methods and figure 10). We identified 37, 599 A-to-I differentially edited positions with a minimum coverage of 30x, which ensures a high degree of confidence at particular base positions. The coverage describes the average number of reads that align to, or "cover," known reference bases. Most of the edited position (32, 183 sites, 85.6% of total) were present in introns (Figure 1A), consistent with other studies in human tissues. The percentage of edited position in 3' untranslated regions (3'UTR) of mRNAs was 6.2% (2344 sites), in line with previous reports using RNA-Seq. The number of editing sites had a homogeneous repartition all over the genome, though edited genes on chromosomes 4, 9, 18, 19, 20, 21 and 22 were slightly overrepresented (Figure 1B). We then performed a differential analysis to identify sites whose editing could be specifically different between MDD and healthy controls. After applying pre-specified quality criteria (exclusion of intergenic sites, coverage >30; AUC>0.6; 0.95>FoldChange>1.05 and p<.05,), we identified 646 variants differentially edited between depressed patients and healthy controls (Figure 1C), representing 366 genes. The quality criteria were set in order to provide potential diagnostic biomarkers (BMKs) for depressive disorders with clinical usefulness. Importantly, no difference in global RNA editing was observed between patients and controls. Indeed, we calculated the global Alu editing index (AEI), which is a measure of the overall rate of RNA editing in Alu repeats. Alu repeats are by far the most common edited region in the human genome, and thus are indicative of the global editing rate. We found no significant differences in AEI between controls and depressed patients (AEI control mean=0.300±0.002, AEI MDD mean=0.299±0.003, p value=0.57, figure 1D). As the Alu Editing index characterizes the weighted average editing level across all expressed Alu sequences, these data suggest that differential RNA editing was target-specific rather than dependent on the disease status.

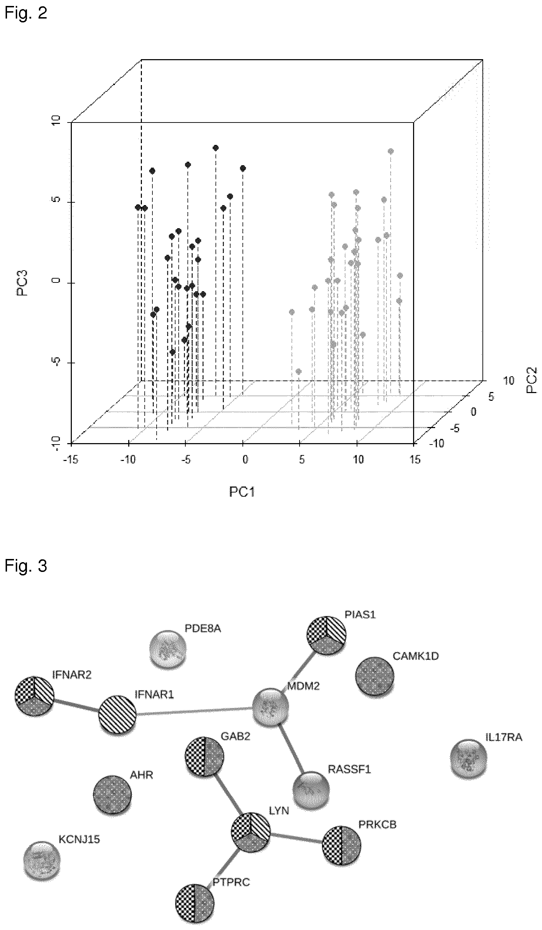

GSEA (enrichment analysis on gene sets) on these 366 genes using gene ontology tools¹⁹ displayed a strong term enrichment (false discovery rate <0.05) for biological processes including multiple immune categories (response, activation and regulation), cell activation and metabolic processes (Figure 12). This analysis was replicated with other tools, such as G:profiler²⁰ (Figure 9) or GOnet²¹ (Figure 10), and consistently indicated a strong enrichment in biological processes related to immune system. Moreover the reactome pathways analysis of those 366 genes revealed a strong enrichment in pathways related to the immune system, i.e. TCR signaling, Translocation of ZAP-70 to Immunological synapse or Interferon gamma signaling (Figure 13). Noteworthy, Principal Component Analysis (PCA) showed that this subset of the 646 editing variants representing 366 genes clearly discriminated controls and depressed patients in 2 separate groups (Figure 2), suggesting that editing variants in those genes could be used in combination to selectively discriminate between depressed patients and healthy controls.

To further narrow down the list of genes, much more stringent quality criteria were used (coverage >30; AUC>0.8; 0.8>FoldChange>1.20 and p<0.05). The database of these markers was then manually curated to reach a combination of several biomarkers representing different biological mechanisms. Using these secondary criteria, a list of 15 variants, representing 13 genes (Figure 14), was selected for both diagnostic performance and for their potential role in the immune system or psychiatric disease biology and to be studied in a subsequent prioritization cohort. Additionally, we nominated one other target, PDE8A, for which previous data indicated altered editing in suicide brain and in blood of drug-induced depression in HCV patients^{5,22}. Protein-protein association networks analysis with STRING database²³ revealed a network with strong enrichment in protein-protein interaction (PPI pvalue <4.5.10-6). The interactions inside STRING were based on evidence and consist of direct (physical) and indirect (functional) interactions; we focused here on a minimum required interaction score of 0.7, indicative of high confidence in actual protein-protein interactions. Functional protein association network analysis of the 14 selected genes using gene ontology tools¹⁹ also showed a strong enrichment in genes related to: regulation of immune system process (8 genes, GO:0002682; p(FDR=4.10-4), regulation of immune response, (6 genes, (GO:0050776, p(FDR=2.2.10-3)) or receptor signaling pathway via JAK-STAT (4 genes, GO:0007259), which is typically triggered following interferon-alpha stimulation (Figure 3 and Figure 15). Furthermore, Reactome pathways analysis indicated that those 14 genes were enriched in pathways related to immune system, (e.g. Regulation of IFNA signaling (HSA-912694, p(FDR)<0.025) or Cytokine signaling (HSA-1280215, p(FDR)<0.026)) and neurotransmitter regulation (e.g. Trafficking of AMPA receptors (HSA-399719, p(FDR)<0.025), glutamate binding and synaptic plasticity (HAS-399721, p(FDR)<0.025) or Neurotransmitter receptors and postsynaptic signal transmission (HSA-112314, p(FDR)<0.029)) (Figure 16). Interestingly, the Top pathway identified in the Reactome analysis (p(FDR)<0.012) is linked to the transcription factor Runx1, which has recently been shown to mediate the effect of chronic social defeat stress in a mouse depression model through regulation of the miR-30 Family of miRNAs.

### Target description

**PRKCB:** Protein Kinase C Beta (PRKCB), which belongs to the family of serine- and threonine-specific protein kinases, is well known to play a crucial role in the immune system, as Mice knockout for PKCB develop immunodeficiency characterized by impaired B-cell activation, B cell Receptor response, and defects in T-cell-independent immune responses. PRKCB also act as a regulator of the HPA axis response to stress²⁴. An association study of 44 candidate genes with depressive and anxiety symptoms in post-partum women revealed an association between PRKCB and major depression. Indeed, PRKCB interacts with and phosphorylates CREB1, which directly regulates the expression of several genes involved in stress response in the brain, such as BDNF, the BDNF receptor Trk-b, and the glucocorticoid receptor²⁵.

**MDM2:** MDM2 is a primary cellular inhibitor of p53, and might be a target for anticancer treatment. However, MDM2 also catalyzes ubiquitination of β-arrestin, which is a target for antidepressants. In the CNS, MDM2 is involved in AMPAR (glutamate receptor) surface expression during synaptic plasticity²⁶. Interestingly, RNA editing in MDM2 has been shown to regulate protein levels. Indeed, increased RNA editing at the 3' UTR of MDM2 abolishes microRNA-mediated repression, which may increase its mRNA levels.

**PIAS1:** PIAS1 (Protein Inhibitor of Activated STAT 1) function as a SUMO ligase which blocks the DNA binding activity of Stat1 and inhibited Stat1-mediated gene activation in response to interferon. Sumoylation of transcription factors (HSA-3232118) is one of the major Reactome pathways in the 14 genes selected for analysis in the prioritization cohort (Figure 16). PIAS1 selectively inhibits interferon-inducible genes and is an important player in innate immunity²⁷. In addition, Pias1 interacts with the presynaptic Metabotropic Glutamate Receptor 8 (mGluR8) and add a post transcriptional modification by sumoylation, suggesting that PIAS1 might have regulatory functions in targeting and modulating mGluR8 receptor signaling.

**IFNAR1:** A wealth of studies have linked interferons to inflammation-induced changes in brain function and depression, at least in part though the effect of IL-6, CXCL10 or by induction of indoleamine 2,3-dioxygenase1 (IDO1)²⁸. Moreover, it has been shown that polymorphisms in the promoter region of the IFN-alpha/beta receptor 1 (IFNAR1) can influence the risk of developing depression. Interferons are used for the treatment of viral hepatitis, hemato-proliferative disorders, autoimmune disorders and malignancies. However, irrespective of the indication for IFN therapy, IFNs are associated with a 30-70% risk of treatment-emergent depression. In a previous work we have shown that RNA editing biomarkers allowed discrimination between patients who developed a treatment-emergent depression and those who did not.

**IFNAR2:** Interferons act through their receptors; the interferon type I receptor (IFNaR) is composed of two subunits, IFNAR1 and IFNAR2. A polymorphism in the IFNAR2 gene (SNP 11876) showed an association with multiple sclerosis susceptibility (p = 0.035). Interestingly, the same study also identified a polymorphism in the IFNAR1 gene (SNP 18417), which is a member of the same receptor.

**LYN:** LYN encodes a non-receptor tyrosine-protein kinase that transmits signals from cell surface receptors. Lyn plays an important role in the regulation of innate and adaptive immune responses²⁹. In the mammalian central nervous system, Lyn is highly expressed in the nucleus accumbens, cerebral cortex and thalamus. Moreover, Lyn participates in the control of N-methyl-D-aspartate receptor (NMDAR, an ionotropic glutamate receptor) receptor pathway. Indeed, behavioral tests showed that spontaneous motor activity is suppressed in Lyn-/- mice, due to enhancement of NMDA receptor signaling. Furthermore, Lyn physically interact with AMPA receptor, a member of the glutamate receptor family³⁰. Following stimulation of the receptor, Lyn activates the mitogen-activated protein kinase (MAPK) signalling pathway, which in turn increases expression of brain-derived neurotrophic factor (BDNF). Importantly, activation of AMPA receptors along with release of BDNF and activation of downstream synaptogenic signaling pathways mediate the anti-depressant effects of ketamine79. Lyn kinase-glutamate receptor interactions undergo drastic adaptations in mood disorders such as major depressive disorder.

**AHR:** The aryl hydrocarbon receptor (AHR) is a highly conserved ligand-dependent transcription factor that senses environmental toxins and endogenous ligands. AHR also modulates immune cell differentiation and response³¹. AHR also controls both T(reg) and Th17 cell differentiation in a ligand-specific fashion. Activation AHR induces IDO and thus increases kynurenine levels. The role of Kynureine is well documented in the CNS, where it plays important roles in the pathophysiology of inflammation-induced depression. Pharmacological blockade of AHR rescued systemic inflammation-induced monocyte trafficking, neuroimmune disturbance, and depressive-like behavior in mice. RNA Editing affects the 3'UTR of the human AHR mRNA, thereby creating a microRNA recognition sequence which modulates AHR expression.

**RASSF1:** Ras association domain family member 1 (RASSF1) RASSF1A is a tumor suppressor gene whose inactivation has been implicated in a wide variety of sporadic human cancers and is epigenetically inactivated at high frequency in a variety of solid tumors. RASSF1A associates with MDM2 and enhances the self-ubiquitin ligase activity of MDM2³². Interestingly, a microarray analysis recently identified RASSF1A as a significantly downregulated gene in women with history of postpartum depression.

**GAB2:** Gab proteins function downstream of multiple receptors, such as the receptors for EGF, hepatocyte growth factor, and FGFs. GAB2 is an important component of neuronal differentiation induced by retinoic acid, partly by acting downstream of bFGF to mediate survival through PI3 kinase-AKT. GAB2 positively upregulated IL-4 and IL-13 expression in activated T cells, suggesting that GAB2 might be an important regulator of the human Th2 immune response³³.

**CAMK1D:** CAMK1 D is a member of the calcium/calmodulin-dependent protein kinase 1 family and is involved in the chemokine signal transduction pathway that regulates granulocyte function³⁴. In the CNS, CAMK1 D is mostly expressed in the CA1 region of the hippocampus, where it is induced following Long-term potentiation (LTP), a long lasting enhancement of synaptic efficacy in the central nervous system. It could translocate to the nucleus in response to stimuli that trigger intracellular Ca2+ influx, e.g. glutamate, and activate CREB-dependent gene transcription. Noteworthy, SNP rs2724812 in CAMK1D has been associated to remission after selective serotonin reuptake inhibitors treatment by a deep learning approach³⁵.

**KCNJ15:** Potassium voltage-gated channel subfamily J member 15 (KCNJ15) encodes an inward-rectifier type potassium channel, Kir4.2. In the CNS, the KIR4.2 channel is expressed late in development in both mice and human brain. Inwardly rectifying potassium channels (Kir channels) are important regulators of neuronal signaling and membrane excitability. Gating of Kir channel subunits plays crucial role in polygenic CNS diseases. Moreover, rs928771 in KCNJ15 was found to be associated with alterations in KCNJ15 transcript levels in whole blood; network analysis of hippocampus and blood transcriptome datasets suggested that suggests that the risk variants in the KCNJ15 locus might act through their regulatory effects on immune-related pathways³⁶.

**IL17RA:** The Interleukin 17 Receptor A (IL17RA) is a type I membrane glycoprotein, which is a similar structure to IFNAR1, that binds with low affinity to interleukin 17A. Interleukin 17A (IL17A) is a proinflammatory cytokine primarily secreted by activated Th17 lymphocytes. Upon biding to its cognate receptor, IL17 signals through ERK, MAPK and NFKB pathways, all of which have been implicated in depressive disorder. Th17 cells are potent regulators of brain function as they target various brain resident cells including neurons, astrocytes, and microglia and participate in neuroinflammation³⁷. IL17RA is highly expressed in blood-brain barrier (BBB) endothelial cells in multiple sclerosis lesions, where IL17 increases BBB permeability by disrupting tight junctions, thereby promoting CNS inflammation. Pro-inflammatory IL17 is also secreted by activated astrocytes and microglia and has detrimental effect on neurons. Flow cytometry analysis of PBMCs in unmedicated depressed subjects revealed a significant increase in peripheral Th17 cell number, and elevated serum concentration of IL-17, suggesting a potential role of Th17 cells in MDD patients. Similarly, Th17 cells are increased in the brain of mice after learned helplessness or chronic restraint stress, which are two common depression-like models. Inhibiting the production or function of Th17 cells reduces vulnerability to depression-like behavior in the same mouse models

**PTPRC:** Protein Tyrosine Phosphatase, Receptor Type C (PTPRC), also known as leukocyte-common antigen, or CD45, is an integral membrane protein tyrosine phosphatase, which contains an external segment and is capable of initiating transmembrane signaling in response to external ligands³⁸. In the immune system, PTPRC is essential for activation of T and B cells by mediating cell-to-cell contacts and regulating protein-tyrosine kinases involved in signal transduction. PTPRC suppresses negatively regulates cytokine receptor signaling; selective disruption of PTPRC expression leads to enhanced interferon-receptor-mediated activation of JAK and STAT kinases proteins³⁹. Remarkably, a point mutation in PTPRC (C-to-G nucleotide transition in position 77 of exon 4) has been associated with the development of multiple sclerosis, the most common demyelinating disease of the CNS. In the brain, anxiety-like behavior induced by repeated social defeat, a mouse model of depression, corresponded with an increase in brain macrophages overexpressing PTPRC. In the same mouse model of depression, classical benzodiazepines were shown to reverse symptoms and to block stress-induced accumulation of macrophages expressing high levels of PTPRC in the CNS. Furthermore, in patients diagnosed with major depressive disorder, PTPRC was higher in patients than in healthy subjects. PTPRC ratio decreased with antidepressant treatment such as venlafaxine or fluoxetine and was positively correlated with the severity of depression⁴⁰, suggesting that antidepressant treatment contributes to immune regulation in patients with major depressive disorder.

**PDE8A:** Glutamate or serotonin receptors are G-protein-coupled receptors (GPCRs) involved in a variety of psychiatric disorders, for which the spatial and temporal regulation of second messenger concentration is crucial for subsequent signaling. Phosphodiesterases (PDE) are key modulators of signal transduction and are involved in inflammatory cell activation, behavior, memory and cognition. PDE8A is a cAMP-specific phosphodiesterase expressed in the brain and blood. Altered RNA editing in PDE8A transcripts has been identified in in T cells of patients suffering from systemic lupus erythematosus, a chronic autoimmune disorder, as in normal T cells with IFN-α. There is a two-fold decrease in the expression of phosphodiesterase 8A (PDE8A) in the temporal cortex of major depressive disorder (MDD) patients⁴¹. We have recently shown brain region-specific alterations of RNA editing in PDE8A mRNA in suicide decedents, which provided the first immune response-related brain marker for suicide⁵. PDE8A RNA editing is also increased in SHSY-5Y neuroblastoma cells treated with interferon alpha, as in HCV patients undergoing antiviral combination therapy with IFN-α and ribavirin. Importantly, measurement of RNA editing in PDE8A allowed discrimination between the group of patients who developed a treatment-emergent depression and those who did not, suggesting that A-I RNA editing biomarkers could be useful to monitor treatment-emergent depression²².

In order to prioritize a subset of variants clinically useful for a diagnostic assay, each of the target genes described above was tested individually in two separate 'prioritization' cohorts (n=76, Figure 17 and n=86, Figure 18) by measuring editing rate in controls and depressed patients by NGS. In the first cohort each target was sequenced individually, while in the second cohort editing rates of biomarkers were measured by multiplexing 4 BMKs. A multivariate analysis statistical method, evaluating for each combination of RNA edited sites and/or isoforms, was performed to find the best combination of biomarkers. To potentiate diagnostic performance, while minimizing biomarker number, the best combination was calculated with different numbers of biomarkers.
The list of primers used for the detection of RNA editing sites is indicated in Table 1

**Table 1: Primers used for the detection of RNA editing sites**

| **PRIMER NAME** | **Gene Name** | **PCR product Size (bp)** | **Primer Sequence (5'→3')** | **Primer length (bp)** | **SEQ ID NO.** |
|---|---|---|---|---|---|
| IFNAR1 FORWARD_1 | IFNAR1 | 259 | ATACGGGCAAGCTCTTAACTATT | 23 | 1 |
| IFNAR1 REVERSE_1 | | | AACCTCCACCTCCTGTTTTC | 20 | 2 |
| IFNAR1_ MiSeqF | IFNAR1 | 472 | AAAATACGGGCAAGCTCTTAACT | 23 | 3 |
| IFNAR1_ MiSeqR | | | CTGCACTTAGGCTCACAGGA | 20 | 4 |
| | | | | | |
| PDE8A Forward | PDE8A | 225 | ATGCAAGTTGTGGACATGGAG | 21 | 5 |
| PDE8A Reverse | | | TTCTGAAAACAATGGGCACCA | 21 | 6 |
| | | | | | |
| CAMK1 DPCR0_F | CAMK1D | 577 | TGTGCAAAGGGCTCCATAC | 19 | 7 |
| CAMK1 DPCR0_R | | | CCTTGCAAGTGTCCAGTAAAC | 21 | 8 |
| | | | | | |
| CAMK1D MPx_F1 | | 195 | CTAGGGTGAGGTGGTGCAAT | 20 | 9 |
| CAMK1D MPx_R1 | | | CAAGGCCAGGAGTTCAAGAC | 20 | 10 |
| | | | | | |
| LYN_F_PCR0 | LYN | 358 | TTTAAAAGCTTCTCCAGTCAGC | 22 | 11 |
| LYN_R_PCR0 | | | CAAGACTGAGCATACATTATATTCACC | 27 | 12 |
| | | | | | |
| LYN MPx_F1 | | 193 | TTTAAAAGCTTCTCCAGTCAGC | 22 | 13 |
| LYN MPx_R1 | | | TCCTGAGTAGCTGGGACCAT | 20 | 14 |
| | | | | | |
| PRKCB_MPx_F1 | PRKCB | 176 | CAGCACATTCTGTTGCCTTC | 20 | 15 |
| PRKCB_MPx_R2 | | | GTGACTTTGCCCCTCATTTG | 20 | 16 |
| | | | | | |
| KCNJ15_MPx_F1 | KCNJ15 | 160 | GGCCACTCTGTACTATGCAAATC | 23 | 17 |
| KCNJ15_MPx_R2 | | | TGAATTTCAGATGGACAGCAA | 21 | 18 |
| | | | | | |
| MDM2_F5 MPx-Rev | MDM2 | 302 | AAGCCCATCCTCGAACTACAG | 21 | 19 |
| MDM2_R5 MPx-Rev | | | TTGCTCTGTCACCTGGACTG | 20 | 20 |
| | | | | | |
| GAB2 MPx_F1 | GAB2 | 154 | GAACCAGGCTAGGTGCAATG | 20 | 21 |
| GAB2 MPx_R2 | | | CCATGCCCTGGTAATTTTTG | 20 | 22 |
| | | | | | |
| AHR-F1 | AHR | 409 | TCTGATGGAATCCCAAACTGAGA | 23 | 23 |
| AHR_F2 | | | TCAGAGATATTTTGTCCGAAGCCA | 24 | 24 |
| | | | | | |
| IFNAR2 MPx_F1_rev | IFNAR2 | 344 | CCCACAGTTTCAGAGGTGGT | 18 | 25 |
| IFNAR2 MPx_R1_rev | | | GGCTCACCCCTGTAATCC | 20 | 26 |
| | | | | | |
| IL17RA_Forward1 | IL17RA | 172 | GGGATGTAAACGCTGAATGG | 20 | 27 |
| IL17RA_ Reverse1 | | | ATGTGCCACCATGACTGACT | 20 | 28 |
| | | | | | |
| RASSF1_F1 | | 480 | CCCTTCCGGCAAGAATCCAG | 20 | 29 |
| RASSF1_R1 | | | ACCCAAGCTGCATAAAAGGC | 20 | 30 |
| | | | | | |
| PIAS1_F1 | PIAS1 | 407 | GCAACCTTAGATCTCAATGGCTAA | 24 | 31 |
| PIAS1_R2 | | | AGCAGAGTCACAGTCTACAGC | 21 | 32 |
| | | | | | |
| PTPRC-MiSeqF | PTPTC | 505 | CCGGGGCTCATTTATAGGAT | 20 | 33 |
| PTPRC-MiSeqR | | | TGCTAGATACCGCCACATTG | 20 | 34 |
| PTPRC F rd2-1 | PTPRC | 215 | TAAGGACGGAGTTCGAGACC | 20 | 35 |
| PTPRC R rd2-1 | | | TGAGAGAGTTTTGCTCTGTGG | 21 | 36 |

As it was important to validate the results obtained in the RNA-Seq study, we first ran a differential analysis of each target on the prioritization cohorts. As shown in the tables below (Tables 2A-2B and3), all the sites and isoforms identified in the RNA-Seq study were found significant in a subsequent analysis on a larger population.

**Tables 2A and 2B:** Differential analysis of editing sites (2A) and isoforms (2B) identified in the RNA-Seq study in the patients of the first prioritization cohort analyzed in single-plex.
Shown are examples of RNA-Seq data of different targets with target name, differentially edited site or isoform, p-value and fold change of the edited site or isoform, AUC ROC, the mean value of editing at the considered site and population number.

**Table 2A**

| **ID** | **Target** | **Chr:Pos (hg38)** | **Sites** | **p-Value** | **foldChange** | **AUC ROC** | **%Editing (Mean)** | **Population** |
|---|---|---|---|---|---|---|---|---|
| 1 | AHR | 7:17345070 | X118 | 0.0318 | 1.80 | 0.635 | 0.52 | N= 76 [41 (0) ; 35 (1)1 |
| 3 | IFNAR1 | 21:33355789 | X111 | 0.0239 | 1.07 | 0.651 | 19.16 | N= 76 [41 (0) ; 35 (1)] |
| 4 | IFNAR1 | 21:33355793 | X115 | 0.0098 | 1.11 | 0.678 | 10.89 | N= 76 [41 (0) ; 35 (1)] |
| 5 | IFNAR1 | 21:33355807 | X129 | 0.0337 | 1.06 | 0.647 | 20.53 | N= 76 [41 (0) ; 35 (1)] |
| 6 | IFNAR1 | 21:33355838 | X160 | 0.0030 | 1.13 | 0.682 | 7.60 | N= 76 [41 (0) ; 35 (1)] |
| 7 | IFNAR1 | 21:33355840 | X162 | 0.0069 | 1.10 | 0.681 | 10.05 | N= 76 [41 (0) ; 35 (1)] |
| 8 | IFNAR1 | 21:33355854 | X176 | 0.0004 | 1.18 | 0.745 | 0.54 | N= 76 [41 (0) ; 35 (1)] |
| 9 | IFNAR1 | 21:33355860 | X182 | 0.0142 | 1.13 | 0.652 | 4.30 | N= 76 [41 (0) ; 35 (1)] |
| 10 | IFNAR1 | 21:33355865 | X187 | 0.0019 | 1.15 | 0.698 | 1.96 | N= 76 [41 (0) ; 35 (1)] |
| 11 | IFNAR1 | 21:33355869 | X191 | 0.0059 | 1.14 | 0.688 | 7.47 | N= 76 [41 (0) ; 35 (1)] |
| 12 | IFNAR1 | 21:33355905 | X227 | 0.0066 | 1.10 | 0.681 | 5.45 | N= 76 [41 (0) ; 35 (1)] |
| 13 | IFNAR1 | 21:33355909 | X231 | 0.0121 | 1.10 | 0.661 | 10.39 | N= 76 [41 (0) ; 35 (1)] |
| 14 | IFNAR1 | 21:33355762 | X84 | 0.0056 | 1.12 | 0.678 | 10.56 | N= 76 [41 (0) ; 35 (1)] |
| 15 | IFNAR1 | 21:33355763 | X85 | 0.0067 | 1.12 | 0.681 | 6.25 | N= 76 [41 (0) ; 35 (1)] |
| 16 | IL17RA | 22:17110647 | X151 | 0.0278 | 1.05 | 0.637 | 8.40 | N= 76 [41 (0) ; 35 (1)] |
| 17 | IL17RA | 22:17110680 | X184 | 0.0049 | 1.13 | 0.674 | 0.83 | N= 76 [41 (0) ; 35 (1)] |
| 18 | IL17RA | 22:17110551 | X55 | 0.0164 | 1.14 | 0.655 | 1.41 | N= 76 [41 (0) ; 35 (1)] |
| 20 | IL17RA | 22:17110574 | X78 | 0.0049 | 1.09 | 0.675 | 1.95 | N= 76 [41 (0) ; 35 (1)] |
| 21 | IL17RA | 22:17110591 | X95 | 0.0411 | 1.05 | 0.629 | 15.97 | N= 60 [41 (0) ; 19 (1)] |
| 22 | LYN | 8:56012521 | X116 | 0.0266 | 1.12 | 0.652 | 0.97 | N= 76 [41 (0) ; 35 (1)] |
| 23 | LYN | 8:56012553 | X148 | 0.0238 | 1.06 | 0.676 | 18.79 | N= 60 [41 (0) ; 19 (1)] |
| 24 | LYN | 8:56012558 | X153 | 0.0074 | 1.10 | 0.661 | 3.81 | N= 76 [41 (0) ; 35 (1)] |
| 25 | LYN | 8:56012689 | X284 | 0.0140 | 1.10 | 0.633 | 4.34 | N= 76 [ 41 (0) ; 35 (1)] |
| 26 | LYN | 8:56012705 | X300 | 0.0161 | 1.10 | 0.652 | 3.03 | N= 76 [41 (0) ; 35 (1)] |
| 27 | LYN | 8:56012490 | X85 | 0.0049 | 1.13 | 0.679 | 0.75 | N= 76 [41 (0) ; 35 (1)] |
| 28 | MDM2 | 12:68821545 | X166 | 0.0142 | 0.91 | 0.661 | 2.91 | N= 76 [41 (0) ; 35 (1)] |
| 29 | MDM2 | 12:68821629 | X250 | 0.0215 | 0.89 | 0.649 | 4.42 | N= 76 [41 (0) ; 35 (1)] |
| 30 | PIAS1 | 15:68138001 | X201 | 0.0446 | 1.09 | 0.664 | 0.64 | N= 60 [41 (0) ; 19(1)] |
| 31 | PTPRC | 1:198680346 | X240 | 0.0216 | 1.06 | 0.689 | 25.83 | N= 60 [41 (0) ; 19 (1)] |
| 32 | PTPRC | 1:198680405 | X299 | 0.0263 | 1.08 | 0.675 | 4.30 | N= 60 [41 (0) ; 19 (1)] |
| 33 | RASSF1 | 3:50333653 | X192 | 0.0204 | 1.09 | 0.654 | 1.32 | N= 60 [41 (0) ; 19 (1)] |
| 35 | PDE8A | 15:85212876 | X116246 | 0.0055 | 0.92 | 0.668 | 19.81 | N= 76 [41 (0) ; 35 (1)] |

**Table 2B**

| **Target** | **Isoform** | **p-Value** | **Fold-change** | **AUC ROC** | **%Editing (Mean)** | **Population** |
|---|---|---|---|---|---|---|
| IFNAR1 | A | 0.0002 | 1.13 | 0.735 | 0.53 | N= 76 [41 (0) ; 35 (1)] |
| IFNAR1 | ABCDEFGH | 0.0042 | 1.19 | 0.686 | 0.99 | N= 76 [41 (0) ; 35 (1)] |
| IFNAR1 | GH | 0.0002 | 1.16 | 0.742 | 0.73 | N= 76 [41 (0) ; 35 (1)] |
| LYN | DO | 0.0275 | 0.94 | 0.660 | 1.45 | N= 76 [41 (0) ; 35 (1)] |
| MDM2 | GVA | 0.0023 | 1.09 | 0.710 | 0.94 | N= 76 [41 (0) ; 35 (1)] |
| MDM2 | W | 0.0154 | 0.93 | 0.669 | 0.45 | N= 76 [41 (0) ; 35 (1)] |
| PDE8A | B | 0.0059 | 0.92 | 0.660 | 19.00 | N= 76 [41 (0) ; 35 (1)] |
| PIAS1 | F | 0.0359 | 1.04 | 0.641 | 1.10 | N= 76 [41 (0) ; 35 (1)] |
| PTPRC | HI | 0.0331 | 1.07 | 0.623 | 1.61 | N= 76 [41 (0) ; 35 (1)] |
| RASSF1 | ABE | 0.0224 | 1.04 | 0.637 | 1.87 | N= 76 [41 (0) ; 35 (1)] |
| RASSF1 | ABEH | 0.0290 | 1.07 | 0.695 | 0.69 | N= 76 [41 (0) ; 35 (1)] |
| RASSF1 | HJ | 0.0260 | 0.93 | 0.624 | 0.83 | N= 76 [41 (0) ; 35 (1)] |

**Table 3: Differential analysis of editing sites and isoforms identified in the RNA-Seq study in the patients of the second prioritization cohort analyzed with multiplex sequencing.**

| | | | | | | | |
|---|---|---|---|---|---|---|---|
| Shown are examples of RNA-Seq data of different targets with target name, differentially edited site, p-value and fold change of the edited site, AUC ROC, the mean value of editing at the considered site and population number. | | | | | | | |

| **Target** | **Chr:Pos (hg38)** | **Sites** | **p-Value** | **Fold-change** | **AUC ROC** | **% Editing (Mean)** | **Population** |
|---|---|---|---|---|---|---|---|
| CAMK1D | 10:12378423 | X131 | 0.0136 | 0.90 | 0.659 | 1.33 | N= 86 [28 (0) ;58 (1)] |
| CAMK1D | 10:12378341 | X49 | 0.0303 | 0.92 | 0.639 | 5.59 | N= 86 [28 (0) ;58 (1)] |
| CAMK1D | 10:12378415 | X123 | 0.0387 | 0.93 | 0.698 | 25.23 | N= 30 [15 (0) ;15 (1)] |
| GAB2 | 11:78330952 | X88 | 0.0004 | 0.88 | 0.705 | 1.5 | N= 86[28 (0) ;58 (1)] |
| GAB2 | 11:78330914 | X126 | 0.0260 | 0.96 | 0.610 | 17.18 | N= 86 [28 (0) ;58 (1)] |
| IFNAR1 | 21:33355779 | X101 | 0.0033 | 0.87 | 0.717 | 0.87 | N= 86 [28 (0) ;58 (1)] |
| IFNAR1 | 21:33355820 | X142 | 0.0159 | 0.89 | 0.663 | 1.3 | N= 86 [28 (0) ;58 (1)] |
| IFNAR1 | 21:33355754 | X76 | 0.0330 | 0.91 | 0.643 | 4.4 | N= 86 [28 (0) ;58 (1)] |
| KCNJ15 | 21:38287590 | X134 | 0.0099 | 0.89 | 0.673 | 3.6 | N= 86 [28 (0) ;58 (1)] |
| KCNJ15 | 21:38287586 | X130 | 0.0308 | 0.93 | 0.650 | 25.2 | N= 86 [28 (0) ;58 (1)] |
| KCNJ15 | 21:38287585 | X129 | 0.0237 | 0.91 | 0.647 | 3.2 | N= 86 [28 (0) ;58 (1)] |
| KCNJ15 | 21:38287589 | X133 | 0.0197 | 0.90 | 0.644 | 14.53 | N= 86 [28 (0) ;58 (1)] |
| KCNJ15 | 21:38287587 | X131 | 0.0488 | 0.91 | 0.634 | 0.9 | N= 86 [28 (0) ;58 (1)] |
| LYN | 8:56012495 | X90 | 0.0040 | 0.89 | 0.696 | 1.2 | N= 86 [28 (0) ;58 (1)] |
| LYN | 8:56012560 | X155 | 0.0443 | 1.09 | 0.635 | 0.6 | N= 86 [28 (0) ;58 (1)] |
| LYN | 8:56012553 | X148 | 0.0435 | 0.99 | 0.532 | 15.7 | N= 57 [28 (0) ;29 (1)] |
| MDM2 | 12:68821586 | X207 | 0.0004 | 0.89 | 0.746 | 12.58 | N= 86 [28 (0) ;58 (1)] |
| MDM2 | 12:68821522 | X143 | 0.0018 | 0.79 | 0.699 | 0.76 | N= 86 [28 (0) ;58 (1)] |
| MDM2 | 12:68821570 | X191 | 0.0296 | 0.92 | 0.652 | 1.09 | N= 86 [28 (0) ;58 (1)] |
| PDE8A | 15:85212876 | X116248 | 0.0032 | 0.90 | 0.680 | 20.3 | N= 86 [28 (0) ;58 (1)] |
| PRKCB | 16:23920909 | X103 | 0.0236 | 0.94 | 0.649 | 5.7 | N= 86 [28 (0) ;58 (1)] |
| PRKCB | 16:23920932 | X126 | 0.0235 | 0.94 | 0.649 | 1.5 | N= 86 [28 (0) ;58 (1)] |
| PRKCB | 16:23920938 | X132 | 0.0360 | 0.96 | 0.637 | 17.3 | N= 86 [28 (0) ;58 (1)] |
| PTPRC | 1:198680365 | X259 | 0.0466 | 0.94 | 0.625 | 0.8 | N= 86 [28 (0) ;58 (1)] |
| PTPRC | 1:198680332 | X226 | 0.0295 | 0.95 | 0.664 | 13.0 | N= 57 [28 (0) ;29 (1)] |
| MDM2 | 12:68848833 | X76 | 0.0035 | 0.83 | 0.704 | 0.5 | N= 86 [28 (0) ;58 (1)] |
| MDM2 | 12:68848843 | X86 | 0.0410 | 0.89 | 0.664 | 1.0 | N= 86 [28 (0) ;58 (1)] |
| MDM2 | 12:68848844 | X87 | 0.0388 | 0.95 | 0.639 | 4.8 | N= 86 [28 (0) ;58 (1)] |

Combinations of biomarkers were then analyzed to identify the best diagnostic performance with different numbers of biomarkers. In the first cohort, when only 4 sites from 3 different targets were used, we obtained an AUC of 0.811 (Sp 78.1%; Se 81.3%, Figure 4A). Another combination of 4 sites, using 2 BMKs, gave even better performances, with an AUC of 0.866 (Sp 92.7%; Se 79.0%, Figure 4B). The equation used for this combination is the following:
Z= + (0,361161202728045)* IFNAR1_X227_H + (-0,0763586204473731)* IFNAR1_X162_E + (1,35409123139041)* LYN_X284_P + (-2,2905526156995)* LYN_X207_M.

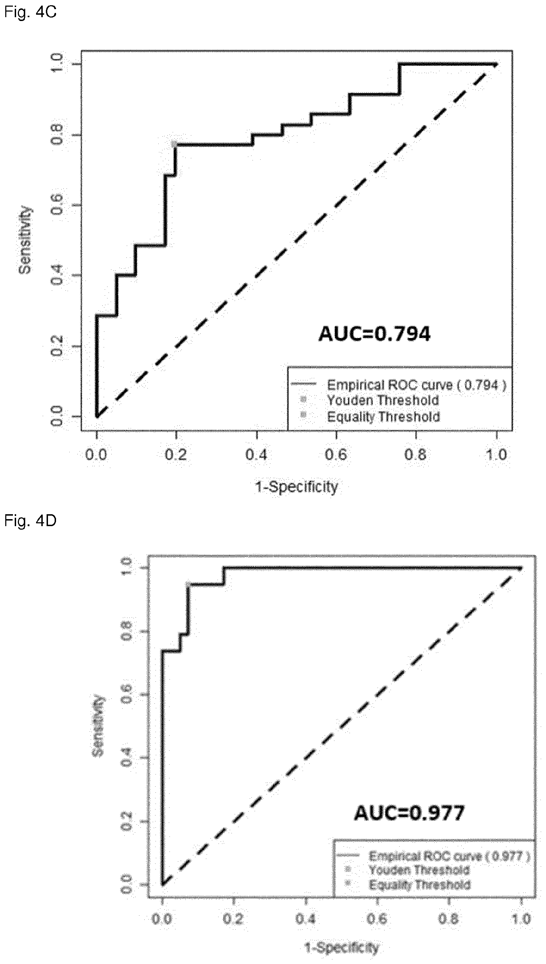

Though measurement of RNA editing variants gave good performances with all the combinations tested, the performance was not necessarily reflected by the number of biomarkers: when the number of biomarkers was increased to 6, with 4 different targets, the AUC was 0.794 (Sp 80.5%; Se 77.1%, Figure 4C). However, when 5 BMKs were combined and analyzed all the differentially edited sites in those targets, we obtained an outstanding performance, with an AUC of 0.977, with excellent specificity and sensitivity (92.7% and 94.7%, respectively, Figure 4D). The equation used for this combination is the following: Z= + (1,242350672584)* IFNAR1_X227_H + (1,36013909308819)* IFNAR1_X115_G + (-0,739944712498771)* IFNAR1_X84_F + (1,42700898962888)* IFNAR1_X162_E + (-1,66838875199491)* IFNAR1_X231_D + (-0,887010245248778)* IFNAR1_X129_B + (0,0876014897108424)* IFNAR1_X111_A + (0,985129912485808)* LYN_X153_G + (4,33197064304577)* LYN_X284_P + (-0,200203331256123)* IFNAR1_X152_C + (-15,708397165937)* IL17RA_X78_D + (2,50160465829557)* LYN_X116_T + (5,53594778946524)* IL17RA_X77_C + (-3,04072407838489)* LYN_X47_Q + (1,82611854035731)* IL17RA_X184_H + (1,14130391422593)* LYN_X85_R + (-1,36538923404951)* LYN_X63_C + (-1,52552008654041)* LYN_X172_J + (3,47820883385469)* LYN_X203_L + (-1,80085106045942)* IL17RA_X55_B + (-1,25213023530841)* LYN_X163_Z + (-1,01733883175651)* PTPRC_X240_I + (122,471673425459)* IL17RA_X151_G + (1,09079061375429)* LYN_X64_D + (2,32804478266105)* LYN_X255_N + (-3,86751458009041)* LYN_X167_I + (-12,6254508356666)* LYN_X148_A + (3,00274639389995)* PTPRC_X299_O - (1,08899988893022)* PDE8A_SiteB.
The list of all combination tested in this cohort is presented in Table 4. Noteworthy, in the first cohort some samples were not analyzed and the total population in the combination may be lower than the total population in the cohort. All combinations were statistically significant between control and cases with a p value <10⁻⁴.

**Table 4: Diagnostic performances obtained with patients of the first prioritization cohort analyzed in single-plex.**

| **Num** | **Combinaison mROC** | **Combi** | **ID** | **AUC ROC** | **Cl 95%** | **Threshold** | **Acc (%)** | **Sp (%)** | **Se (%)** | **VPP (%)** | **VPN (%)** | **Population** |
|---|---|---|---|---|---|---|---|---|---|---|---|---|
| | | | | | | | | | | | | |
| **1** | RASSF1_X263 + AHR_X156 | C2 | 2 | 0.782 | [0,628 ; 0,936] | -1.04 | 80.7 | 82.9 | 75.0 | 63.2 | 89.5 | N= 57 [41 (1); 16 (2)] |
| **2** | AHR_X118 + AHR_X156 | C2 | 4 | 0.745 | [0,599 ; 0,892] | 0.12 | 80.7 | 92.7 | 50.0 | 72.7 | 82.6 | N= 57 [41 (1); 16 (2)] |
| **3** | AHR_X156 + PDE8A_X116246 | C2 | 6 | 0.773 | [0,633 ; 0,913] | -6.50 | 77.2 | 78.1 | 75.0 | 57.1 | 88.9 | N= 57 [41 (1); 16 (2) |
| **4** | IFNAR1_X227 + PTPRC_X299 | C2 | 28 | 0.815 | [0,698 ; 0,932] | 3.53 | 76.7 | 68.3 | 94.7 | 58.1 | 96.6 | N= 60 [41 (1); 19 (2)] |
| **5** | IFNAR1_X84 + PTPRC_X299 | C2 | 83 | 0.810 | [0,694 ; 0,926] | 3.95 | 76.7 | 68.3 | 94.7 | 58.1 | 96.6 | N= 60 [41 (1); 19 (2)] |
| **6** | RASSF1_X263 + AHR_X118 | C2 | 1 | 0.767 | [0,626 ; 0,907] | -1.42 | 75.4 | 73.2 | 81.3 | 54.2 | 90.9 | N= 57 [41 (1); 16 (2)] |
| **7** | IFNAR1_X227 + PDE8A_X116246 | C2 | 36 | 0.764 | [0,655 ; 0,873] | -3.28 | 75.0 | 73.2 | 77.1 | 71.1 | 79.0 | N= 76 [41 (1); 35 (2)] |
| **8** | IFNAR1_X84 + PDE8A_X116246 | C2 | 55 | 0.748 | [0,638 ; 0,859] | -2.70 | 73.7 | 73.2 | 74.3 | 70.3 | 76.9 | N= 76 [41 (1); 35 (2)] |
| **9** | IFNAR1_X227 + PTPRC_X240 | C2 | 22 | 0.813 | [0,692 ; 0,933] | 2.05 | 73.3 | 65.9 | 89.5 | 54.8 | 93.1 | N= 60 [41 (1); 19 (2)] |
| **10** | IL17RA_X77 + IFNAR1_X162 | C2 | 1 | 0.703 | [0,584 ; 0,823] | -0.36 | 72.4 | 73.2 | 71.4 | 69.4 | 75.0 | N= 76 [41 (1); 35 (2)] |
| **11** | LYN_X153 + PTPRC_X299 | C2 | 203 | 0.793 | [0,679 ; 0,908] | 4.08 | 71.7 | 61.0 | 94.7 | 52.9 | 96.2 | N= 60 [41 (1); 19 (2)] |
| **12** | PTPRC_X229 + PDE8A_X116246 | C2 | 435 | 0.791 | [0,679 ; 0,903] | -1.73 | 71.7 | 61.0 | 94.7 | 52.9 | 96.2 | N= 60 [41 (1); 19 (2)] |
| **13** | LYN_85 + PDE8A_X116246 | C2 | 46 | 0.749 | [0,639 ; 0,859] | -5.33 | 71.1 | 58.5 | 85.7 | 63.8 | 82.8 | N= 76 [41 (1); 35 (2)] |
| **14** | IFNAR1_X162 + PDEBA_X116246 | C2 | 25 | 0.743 | [0,632 ; 0,854] | -2.21 | 71.1 | 73.2 | 68.6 | 68.6 | 73.2 | N= 76 [41 (1); 35 (2)] |
| **15** | IFNAR1_X231 + PDE8A_X116246 | C2 | 85 | 0.734 | [0,622 ; 0,847] | -2.80 | 71.1 | 61.0 | 82.9 | 64.4 | 80.7 | N= 76 [41 (1); 35 (2)] |
| **16** | IL17RA_X77 + IFNAR1_X227 | C2 | 2 | 0.713 | [0,595 ; 0,831] | -0.74 | 71.1 | 70.7 | 71.4 | 67.6 | 74.4 | N= 76 [41 (1); 35 (2)] |
| **17** | IFNAR1_X84 + IFNAR1_X231 | C2 | 51 | 0.702 | [0,582 ; 0,821] | 1.68 | 71.1 | 75.6 | 65.7 | 69.7 | 72.1 | N= 76 [41 (1); 35 (2)] |
| **18** | AHR_X118 + PDE8A_X116246 | C2 | 5 | 0.755 | [0,626 ; 0,883] | -7.44 | 68.4 | 61.0 | 87.5 | 46.7 | 92.6 | N= 57 [ 41 (1); 16 (2)] |
| **19** | RASSF1_X263 + PDE8A_X116246 | C2 | 3 | 0.753 | [0,621 ; 0,885] | -6.76 | 64.9 | 53.7 | 93.8 | 44.1 | 95.7 | N= 57 [41 (1); 16 (2)] |
| **20** | RASSF1_X263 + AHR_X156 + PDE8A_X116246 | C3 | 3 | 0.793 | [0,65 ; 0,936] | -6.42 | 84.2 | 87.8 | 75.0 | 70.6 | 90.0 | N= 57 [41 (1); 16 (2)] |
| **21** | RASSF1_X263 + AHR_X118 + AHR_X156 | C3 | 1 | 0.788 | [0,638 ; 0,938] | -1.10 | 84.2 | 90.2 | 68.8 | 73.3 | 88.1 | N= 57 [41 (1); 16 (2)] |
| **22** | IFNAR1_X227 + LYN_X284 + LYN_X63 | C3 | 184 | 0.861 | [0,754 ; 0,969] | 2.16 | 83.3 | 87.8 | 73.7 | 73.7 | 87.8 | N= 60 [41 (1); 19 (2)] |
| **23** | IFNAR1_X227 + LYN_X284 + LYN_X172 | C3 | 185 | 0.874 | [0,787 ; 0,962] | 3.81 | 80.0 | 80.5 | 79.0 | 65.2 | 89.2 | N= 60 [41 (1); 19 (2)] |
| **24** | IFNAR1_X227 + PTPRC_X299 + PDE8A_X116246 | C3 | 406 | 0.859 | [0,763 ; 0,955] | -0.10 | 80.0 | 75.6 | 89.5 | 63.0 | 93.9 | N= 60[41 (1); 19 (2)] |
| **25** | IFNAR1_X227 + LYN_X284 + LYN_X64 | C3 | 191 | 0.852 | [0,748 ; 0,957] | 2.13 | 80.0 | 80.5 | 79.0 | 65.2 | 89.2 | N= 60 [41 (1); 19 (2)] |
| **26** | IFNAR1_X84 + LYN_X284 + LYN_X172 | C3 | 914 | 0.850 | [0,754 ; 0,945] | 4.30 | 78.3 | 78.1 | 79.0 | 62.5 | 88.9 | N= 60 [41 (1); 19 (2)] |
| **27** | IFNAR1_X227 + IFNAR1_X111 + PDE8A_X116246 | C3 | 199 | 0.769 | [0,66 ; 0,878] | -3.55 | 77.6 | 73.2 | 82.9 | 72.5 | 83.3 | N= 76 [41 (1); 35 (2)] |
| **28** | AHR_X118 + AHR_X156 + PDE8A _X116246 | C3 | 4 | 0.777 | [0,649 ; 0,905] | -6.99 | 77.2 | 82.9 | 62.5 | 58.8 | 85.0 | N= 57 [41 (1); 16 (2)] |
| **29** | IFNAR1_X84 + IFNAR1_X231 + PDE8A_X116246 | C3 | 274 | 0.771 | [0,665 ; 0,878] | -3.58 | 76.3 | 78.1 | 74.3 | 74.3 | 78.1 | N= 76 [41 (1); 35 (2)] |
| **30** | RASSF1_X263 + AHR_X118 + PDE8A_X116246 | C3 | 2 | 0.793 | [0,664 ; 0,921] | -7.18 | 75.4 | 73.2 | 81.3 | 54.2 | 90.9 | N= 57 [41 (1); 16 (2)] |
| **31** | IFNAR1_X227 + IL_X55 + PDE8A_X116246 | C3 | 196 | 0.771 | [0,663 ; 0,88] | -3.21 | 75.0 | 75.6 | 74.3 | 72.2 | 77.5 | N= 76 [41 (1); 35 (2)] |
| **32** | IFNAR1_X162 + IFNAR1_X227 + PDE8A_X116246 | C3 | 89 | 0.766 | [0,657 ; 0,875] | -3.40 | 75.0 | 73.2 | 77.1 | 71.1 | 79.0 | N= 76 [41 (1); 35 (2)] |
| **33** | IFNAR1_X84 + PTPRC_X299 + PDE8A_X116246 | C3 | 1135 | 0.851 | [0,757 ; 0,945] | -0.03 | 73.3 | 63.4 | 94.7 | 54.6 | 96.3 | N= 60 [41 (1); 19 (2)] |
| **34** | IFNAR1_X227 + IFNAR1_X162 + LYN_X284 + LYN_X207 | C4 | 831 | 0.866 | [0,761 ; 0,972] | -0.44 | 88.3 | 92.7 | 79.0 | 83.3 | 90.5 | N= 60 [41 (1); 19 (2)] |
| **35** | IFNAR1_X227 + IFNAR1_X129 + LYN_X284 + LYN_X207 | C4 | 1407 | 0.864 | [0,752 ; 0,976] | -3.03 | 88.3 | 92.7 | 79.0 | 83.3 | 90.5 | N= 60 [41 (1); 19 (2)] |
| **36** | IFNAR1_X227 + IFNAR1_X115 + LYN_X284 + LYN_X207 | C4 | 155 | 0.863 | [0,756 ; 0,97] | -0.20 | 88.3 | 92.7 | 79.0 | 83.3 | 90.5 | N= 60 [41 (1); 19 (2)] |
| **37** | IFNAR1_X227 + LYN_X284 + LYN_X207 + PTPRC_X240 | C4 | 2239 | 0.858 | [0,748 ; 0,967] | -1.45 | 88.3 | 92.7 | 79.0 | 83.3 | 90.5 | N= 60 [41 (1); 19 (2)] |
| **38** | IFNAR1_X227 + LYN_X255 + PTPRC_X299 + PDE8A_X116246 | C4 | 3650 | 0.860 | [0,763 ; 0,957] | -0.25 | 83.3 | 80.5 | 89.5 | 68.0 | 94.3 | N= 60 [41 (1); 19 (2)] |
| **39** | IFNAR1_X227 + IFNAR1_X231 + PTPRC_X299 + PDE8A_X116246 | C4 | 1354 | 0.861 | [0,762 ; 0,961] | -1.14 | 81.7 | 75.6 | 94.7 | 64.3 | 96.9 | N= 60 [41 (1); 19 (2)] |
| **40** | IFNAR1_X227 + LYN_X255 + LYN_X148 + PTPRC_X299 | C4 | 3648 | 0.845 | [0,731 ; 0,958] | 40.28 | 81.7 | 75.6 | 94.7 | 64.3 | 96.9 | N= 60 [41 (1); 19(2)] |
| **41** | RASSF1_X263 + AHR_X118 + AHR_X156 + PDE8A_X116246 | C4 | 1 | 0.811 | [0,677 ; 0,945] | -7.18 | 79.0 | 78.1 | 81.3 | 59.1 | 91.4 | N= 57 [41 (1); 16(2)] |
| **42** | IFNAR1_X84 + IFNAR1_X231 + IFNAR1_X111 + PDE8A_X116246 | C4 | 871 | 0.775 | [0,669 ; 0,881] | -3.43 | 77.6 | 80.5 | 74.3 | 76.5 | 78.6 | N= 76 [41 (1); 35 (2)] |
| **43** | IFNAR1_X227 + L17RA_X55 + IFNAR1_X111 + PDE8A_X116246 | C4 | 670 | 0.774 | [0,666 ; 0,882] | -3.60 | 76.3 | 68.3 | 85.7 | 69.8 | 84.9 | N= 76 [41 (1); 35 (2)] |
| **44** | IFNAR1_X227 + IFNAR1_X84 + IFNAR1_X111 + PDE8A_X116246 | C4 | 587 | 0.772 | [0,666 ; 0,878] | -3.00 | 76.3 | 73.2 | 80.0 | 71.8 | 81.1 | N= 76 [41 (1); 35 (2)] |
| **45** | IFNAR1_X84 + IL17RA_X184 + IFNAR1_X231 + PDE8A_X116246_PDE8A_X116246 | C4 | 849 | 0.772 | [0,665 ; 0,879] | -4.12 | 76.3 | 80.5 | 71.4 | 75.8 | 76.7 | N= 76 [41 (1); 35 (2)] |
| **46** | IFNAR1_X227 + LYN_X85 + IFNAR1_X231 + PDE8A_X116246 | C4 | 545 | 0.782 | [0,678 ; 0,886] | -5.41 | 75.0 | 73.2 | 77.1 | 71.1 | 79.0 | N= 76 [41 (1); 35 (2)] |
| **47** | IFNAR1_X84 + LYN_X153 + IFNAR1_X231 + PDE8A_X116246 | C4 | 859 | 0.776 | [0,671 ; 0,881] | -2.93 | 75.0 | 78.1 | 71.4 | 73.5 | 76.2 | N= 76 [41 (1); 35 (2)] |
| **48** | IL17RA_X77 + IFNAR1_X227 + IFNAR1_X84 + IFNAR1 X231 + PDE8A_ X116246 | C5 | 295 | 0.803 | [0,704 ; 0,903] | -6.86 | 77.6 | 75.6 | 80.0 | 73.7 | 81.6 | N= 76 [41 (1); 35 (2)] |
| **49** | IL17RA_X77 + IFNAR1_X227 + IFNAR1_X115 + IFNAR1_X231 + PDE8A_ X116246 | C5 | 323 | 0.792 | [0,689 ; 0,896] | -6.82 | 77.6 | 78.1 | 77.1 | 75.0 | 80.0 | N= 76 [41 (1); 35 (2)] |
| **50** | IFNAR1_X227 + IFNAR1_X115 + IFNAR1_X231 + IL17RA_X55 + PDE8A_X116246 | C5 | 1463 | 0.794 | [0,691 ; 0,898] | -4.96 | 76.3 | 73.2 | 80.0 | 71.8 | 81.1 | N= 76 [41 (1); 35 (2)] |
| **51** | IL17RA_X77 + IFNAR1_X227 + LYN_X85 + IFNAR1_X231 + PDE8A_X116246 | C5 | 259 | 0.790 | [0,687 ; 0,893] | -6.62 | 76.3 | 75.6 | 77.1 | 73.0 | 79.5 | N= 76 [ 41 (1); 35 (2)] |
| **52** | IFNAR1_X227 + LYN_X85 + IL17RA_X184 + IFNAR1_X231 + PDE8A_X116246 | C5 | 1304 | 0.788 | [0,685 ; 0,891] | -5.73 | 76.3 | 75.6 | 77.1 | 73.0 | 79.5 | N= 76 [ 41 (1); 35 (2)] |
| **53** | IFNAR1_X227 + IFNAR1_X84 + IL17RA_X184 + IFNAR1_X231 + PDE8A_X116246 | C5 | 1388 | 0.801 | [0,702 ; 0,899] | -6.10 | 75.0 | 70.7 | 80.0 | 70.0 | 80.6 | N= 76 [ 41 (1); 35 (2)] |
| **54** | IFNAR1_X227 + LYN_X85 + IFNAR1_X115 + IFNAR1_X231 + PDE8A_X116246 | C5 | 1268 | 0.796 | [0,696 ; 0,896] | -6.07 | 75.0 | 73.2 | 77.1 | 71.1 | 79.0 | N= 76 [41 (1); 35 (2)] |
| **55** | IFNAR1_X227 + IFNAR1_X84 + LYN_X153 + IFNAR1_X231 + PDE8A_X116246 | C5 | 1398 | 0.798 | [0,699 ; 0,897] | -4.70 | 73.7 | 68.3 | 80.0 | 68.3 | 80.0 | N= 76 [41 (1); 35 (2)] |
| **56** | IFNAR1_X227 + LYN_X85 + IFNAR1_X84 + IFNAR1_X231 + PDE8A_X116246 | C5 | 1240 | 0.794 | [0,694 ; 0,894] | -6.17 | 73.7 | 75.6 | 71.4 | 71.4 | 75.6 | N= 76 [41 (1); 35 (2)] |
| **57** | IFNAR1_X227 + IFNAR1_X84 + IL17RA_X78 + IFNAR1_X231 + PDE8A_X116246 | C5 | 1373 | 0.792 | [0,691 ; 0,893] | -5.16 | 73.7 | 70.7 | 77.1 | 69.2 | 78.4 | N= 76 [41 (1); 35 (2)] |
| **58** | IFNAR1_X227 + IFNAR1_X84 + IFNAR1_X231 + IL17RA_X55 + PDE8A_X116246 | C5 | 1407 | 0.790 | [0,688 ; 0,892] | -5.34 | 73.7 | 68.3 | 80.0 | 68.3 | 80.0 | N= 76 [41 (1); 35 (2)] |
| **59** | IL17RA_X77 + LYN_X85 + IFNAR1_X84 + IFNAR1_X231 + PDE8A_X116246 | C5 | 415 | 0.790 | [0,69 ; 0,89] | -6.12 | 73.7 | 73.2 | 74.3 | 70.3 | 76.9 | N= 76 [41 (1); 35 (2)] |
| **60** | IFNAR1_X227 + IFNAR1_X84 + IFNAR1_X231 + IFNAR1_X111 + PDE8A_X116246 | C5 | 1410 | 0.790 | [0,688 ; 0,891] | -5.30 | 72.4 | 61.0 | 85.7 | 65.2 | 83.3 | N= 76 [41 (1); 35 (2)] |
| **61** | IFNAR1_X227 + IFNAR1_X84 + IFNAR1_X115 + IFNAR1_X231 + PDE8A_X116246 | C5 | 1352 | 0.789 | [0,688 ; 0,89] | -5.43 | 72.4 | 61.0 | 85.7 | 65.2 | 83.3 | N= 76 [41 (1); 35 (2)] |
| **62** | IL17RA_X77 + IFNAR1_X227 + LYN_X85 + IFNAR1_X115 + IFNAR1_X231 + PDE8A_X116246 | C6 | 553 | 0.803 | [0,702 ; 0,903] | -7.26 | 80.3 | 92.7 | 65.7 | 88.5 | 76.0 | N= 76 [41 (1); 35 (2)] |
| **63** | IL17RA_X77 + IFNAR1_X227 + LYN_X85 + IL17RA_X78 + IFNAR1_X231 + PDE8A_X116246 | C6 | 574 | 0.794 | [0,692 ; 0,897] | -7.59 | 79.0 | 80.5 | 77.1 | 77.1 | 80.5 | N= 76 [41 (1); 35 (2)] |
| **64** | IL17RA_X77 + IFNAR1_X227 + IFNAR1_X115 + IL17RA_X78 + IFNAR1_X231 + PDE8A_X116246 | C6 | 714 | 0.797 | [0,694 ; 0,899] | -7.06 | 77.6 | 78.1 | 77.1 | 75.0 | 80.0 | N= 76 [41 (1); 35 (2)] |
| **65** | IL17RA_X77 + IFNAR1_X162 + IFNAR1_X227 + IFNAR1_X84 + IFNAR1_X231 + PDE8A_X116246 | C6 | 75 | 0.805 | [0,707 ; 0,903] | -6.63 | 76.3 | 73.2 | 80.0 | 71.8 | 81.1 | N= 76 [41 (1); 35 (2)] |
| **66** | IL17RA_X77 + IFNAR1_X227 + IFNAR1_X115 + IFNAR1_X231 + IFNAR1_X111 + PDE8A_X116246 | C6 | 751 | 0.803 | [0,702 ; 0,904] | -6.68 | 76.3 | 75.6 | 77.1 | 73.0 | 79.5 | N= 76 [41 (1); 35 (2)] |
| **67** | IL17RA_X77 + IFNAR1_X227 + IFNAR1_X84 + IFNAR1_X231 + IFNAR1_X111 + PDE8A_X116246 | C6 | 695 | 0.802 | [0,703 ; 0,902] | -6.61 | 76.3 | 78.1 | 74.3 | 74.3 | 78.1 | N= 76 [41 (1); 35 (2)] |
| **68** | IL17RA_X77 + IFNAR1_X227 + IFNAR1_X84 + IL17RA_X78 + IFNAR1_X231 + PDE8A_X116246 | C6 | 658 | 0.801 | [0,702 ; 0,901] | -7.18 | 76.3 | 75.6 | 77.1 | 73.0 | 79.5 | N= 76 [41 (1); 35 (2)] |
| **69** | IFNAR1_X227 + IFNAR1_X84 + IL17RA_X78 + IL17RA_X184 + IFNAR1_X231 + PDE8A_ X116246 | C6 | 2354 | 0.798 | [0,698 ; 0,897] | -6.44 | 76.3 | 70.7 | 82.9 | 70.7 | 82.9 | N= 76 [41 (1); 35 (2)] |
| **70** | IFNAR1_X227 + LYN_X85 + IFNAR1_X84 + IL17RA_X184 + IFNAR1_X231 + PDE8A_X116246 | C6 | 2137 | 0.808 | [0,711 ; 0,904] | -6.43 | 75.0 | 82.9 | 65.7 | 76.7 | 73.9 | N= 76 [41 (1); 35 (2)] |
| **71** | IFNAR1_X227 + LYN_X85 + IFNAR1_X84 + IFNAR1_X231 + IFNAR1_X111 + PDE8A_X116246 | C6 | 2159 | 0.803 | [0,706 ; 0,901] | -5.94 | 75.0 | 82.9 | 65.7 | 76.7 | 73.9 | N= 76 [41 (1); 35 (2)] |
| **72** | IL17RA_X77 + IFNAR1_X227 + IFNAR1_X84 + IFNAR1_X231 + LYN_X116_T + PDE8A_X116246 | C6 | 694 | 0.803 | [0,705 ; 0,901] | -6.96 | 75.0 | 80.5 | 68.6 | 75.0 | 75.0 | N= 76 [41 (1); 35 (2)] |
| **73** | IFNAR1_X227 + LYN_X85 + IFNAR1_X115 + IFNAR1_X231 + IL17RA_X55 + PDE8AX116246 | C6 | 2212 | 0.802 | [0,702 ; 0,902] | -5.94 | 75.0 | 78.1 | 71.4 | 73.5 | 76.2 | N= 76 [41 (1); 35 (2)] |
| **74** | IFNAR1_X227 + IFNAR1_X84 + IFNAR1_X115 + IL17RA_X184 + IFNAR1_X231 + PDE8A_X116246 | C6 | 2319 | 0.802 | [0,704 ; 0,901] | -6.11 | 75.0 | 70.7 | 80.0 | 70.0 | 80.6 | N= 76 [41 (1); 35 (2)] |
| **75** | IFNAR1_X227 + LYN_X85 + IFNAR1_X84 + LYN_X153 + IFNAR1_X231 + PDE8A_X116246 | C6 | 2147 | 0.801 | [0,704 ; 0,899] | -5.36 | 75.0 | 87.8 | 60.0 | 80.8 | 72.0 | N= 76 [41 (1); 35 (2)] |
| **76** | IL17RA_X77 + IFNAR1_X227 + IFNAR1_X115 + IFNAR1_X231 + IL17RA_X55 + PDE8A_X116246 | C6 | 748 | 0.801 | [0,701 ; 0,9] | -8.23 | 75.0 | 70.7 | 80.0 | 70.0 | 80.6 | N= 76 [41 (1); 35 (2)] |
| **77** | IFNAR1_X227 + IFNAR1_X84 + IL17RA_X184 + IFNAR1_X231 + IL17RA_X55 + PDE8A_X116246 | C6 | 2393 | 0.801 | [0,702 ; 0,899] | -6.14 | 75.0 | 70.7 | 80.0 | 70.0 | 80.6 | N= 76 [41 (1); 35 (2)] |
| **78** | IFNAR1_X227 + IFNAR1_X84 + LYN_X153 + IFNAR1_X231 + IL17RA_X55 + PDE8A_X116246 | C6 | 2403 | 0.799 | [0,7 ; 0,897] | -4.86 | 73.7 | 58.5 | 91.4 | 65.3 | 88.9 | N= 76 [41 (1); 35 (2)] |
| **79** | IFNAR1_X227 + IFNAR1_X84 + IL17RA_X78 + LYN_X153 + IFNAR1_X231 + PDE8A _X116246 | C6 | 2364 | 0.802 | [0,704 ; 0,9] | -4.86 | 72.4 | 56.1 | 91.4 | 64.0 | 88.5 | N= 76 [41 (1); 35 (2)] |
| **80** | IFNAR1_X227 + IFNAR1_X115 + IFNAR1_X84 + IFNAR1_X162 + IFNAR1_X231 + IFNAR1_X129 + IFNAR1_X111 + LYN_X153 + LYN_X284 + IFNAR1_X152 + IL17RA_X78 + LYN_X116_T + IL17RA_X77 + LYN_X47 + IL17RA_X184 + LYN_X85 + LYN_X63 + LYN_X172 + LYN_X203 + IL17RA_X55 + LYN_X163 + PTPRC_X240 + IL17RA_X151 + LYN_X64 + LYN_X255 + LYN_X167 + LYN_X148 + PTPRC_X299 + PDE8A_X116246 | C29 | 14 | 0.977 | [0,948 ; 1] | 68.06 | 93.3 | 92.7 | 94.7 | 85.7 | 97.4 | N= 60 [41 (1); 19 (2)] |

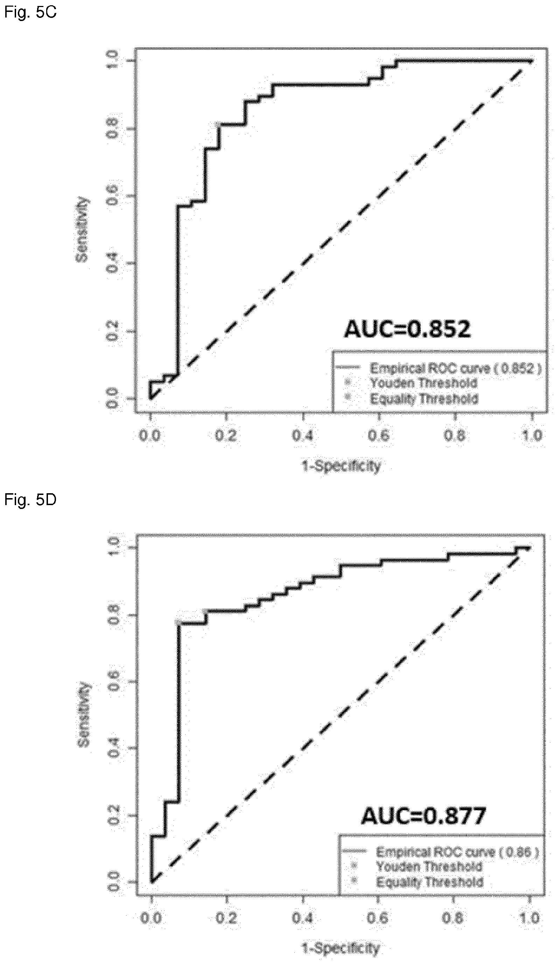

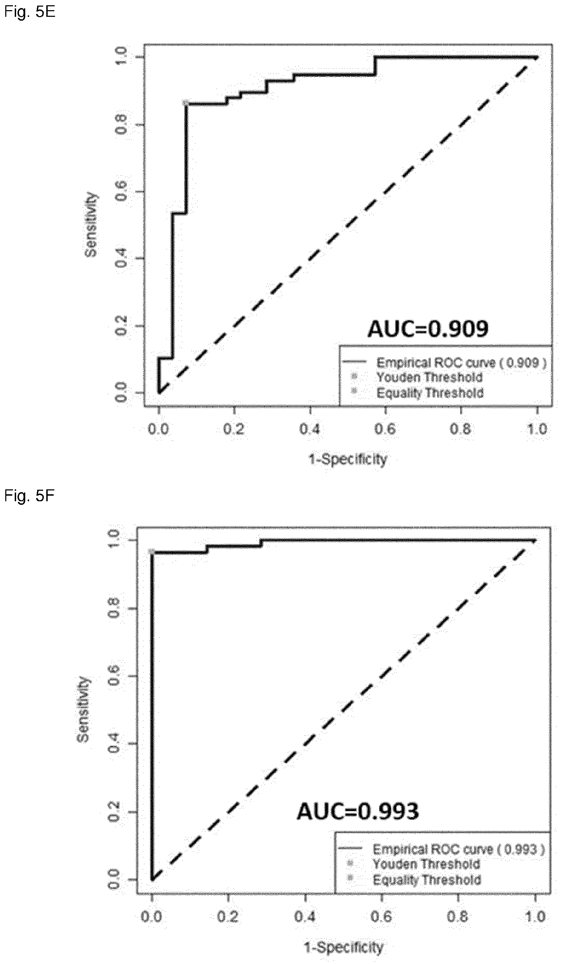

In the second cohort, using multiplexed sequencing, when only 2 sites, each for one BMK, were chosen, the AUC was 0.786 (Sp 78.6%; Se 81.0%, Figure 5A). In this cohort, increasing the number of biomarkers systematically increased diagnostic overall performance of the assay. When 3 sites in 3 BMKs were combined, the AUC increased to 0.839 (Sp 89.3%; Se 74.1%, Figure 5B). With 4 and 5 sites, the AUC rose to 0.852 and 0.860, with specificities of 82.1% and 92.9% and sensitivities of 81% and 77.6%, respectively (Figure 5C and D). When the combination was increased to include 19 sites, the AUC was above 0.9 (AUC 0.909, Sp 92.9%, Se 86.2%, figure 5E). Furthermore, we when we combined 48 differentially edited sites present in 9 different BMKs, we obtained an exceptional AUC of 0.993, with 100% specificity and 96.6% sensitivity (Figure 5F). for this combination the equation used is the following: Z= - (0.179462180023875)* MDM2_Site207_W - (-1.04404354492012)* IFNAR1_Site101_nan - (2.64479093229565)* GAB2_Site88 - (4.11125599566128 )* v1 MDM2_Site76 - (1.61229526347695)* MDM2_Site143 - (5.57184346092994)* LYN_Site90_S - (5.25866173918605)* PDE8A_Site116248_B - (18.995426153835 )* KCNJ15_Site134_D - (-1.04699821617189)* v1MDM2_Site86 - ( 0.480007114248953)* CAMK1D_Site131 - (3.56662634744781)* KCNJ15_Site130_A - (2.4602823172239)* KCNJ15_Site129_B - (-9.43134276848121)* KCNJ15_Site133_C - (1.42715048538683)* IFNAR1_Site76_nan - (1.00606326866477)* CAMK1D_Site49 - ( 0.0542132983688757)* v1MDM2_Site87_E + (5.91151340839856)* LYN_Site155_X - (-2.20437543623734)* KCNJ15_Site131 - (1.30800292033623)* PTPRC_Site169_E - (4.82052635778379)* MDM2_Site277 - (-10.1173014238565 )* KCNJ15_Site190 - (-3.7454636873821)* PTPRC_Site170_F - ( 1.03362539706716)* PTPRC_Site259 - (-4.87935759821363)* PTPRC_Site163_D - (0.628308423459796)* PRKCB_Site119_E - (-0.901688238257015)* KCNJ15_Site193_E - (-0.425246156880022)* MDM2_Site264 - (-0.533167398964742)* IFNAR1_Site123_nan - (-5.64307592868436)* IFNAR1_Site131_nan - (47.981969789844)* GAB2_Site131_H - ( 0.0402950393688472)* MDM2_Site237_X - (-3.33278344933062)* MDM2_Site192 - (0.928449146700883)* CAMK1D_Site64_D - (-1.08069775385396)* GAB2_Site126_A - (-4.23184777312141)* CAMK1D_Site113_G - ( 1.99562009099246)* GAB2_Site52_B - (2.3505776086063)* IFNAR1_Site142_nan - (3.97251790792544)* PRKCB_Site103_C - (0.570977372558063)* PRKCB_Site126 - (-0.410508492870566)* PRKCB_Site132_H - ( 0.975605316457069)* IFNAR1_Site128_nan - (-1.09288061572838)* PDE8A_Site116278_C + (12.6078350770629)* CAMK1D_Site125 - ( 1.48817555399057)* PTPRC_Site253 - (-0.716028094006562)* PRKCB_Site99 - ( -0.722585574440859)* IFNAR1_Site151_nan - (0.00161227142613181)* IFNAR1_Site152_C - (-7.23169188321118)* PRKCB_Site183.
The list of all combination tested in the second prioritization cohort, all of which included the 86 patients, is presented in Table 5. All combinations were statistically significant between control and cases with a p value <10⁻⁴.

**Table 5: Diagnostic performances obtained with patients of the second prioritization cohort analyzed with multiplex sequencing**

| **Num** | **Combinaison mROC** | **Combi** | **ID** | **AUC ROC** | **CI 95%** | **Threshold** | **Acc (%)** | **Sp (%)** | **Se (%)** | **VPP (%)** | **VPN (%)** | **Population** |
|---|---|---|---|---|---|---|---|---|---|---|---|---|
| 1 | GAB2_X88 + KCNJ15_X133 | C2 | 44 | 0.786 | [0.667 ; 0.905] | -3.1179 | 80.2 | 78.6 | 81.0 | 88.7 | 66.7 | N= 86 [28 (1); 58 (2)] |
| 2 | GAB2_X88 + KCNJ15_X134 | C2 | 39 | 0.800 | [0.685 ; 0.915] | -2.4900 | 79.1 | 78.6 | 79.3 | 88.5 | 64.7 | N= 86 [28 (1); 58 (2)] |
| 3 | LYN_X90 + KCNJ15_X134 | C2 | 73 | 0.786 | [0.676 ; 0.896] | -1.9946 | 79.1 | 60.7 | 87.9 | 82.3 | 70.8 | N= 86 [28 (1); 58 (2)] |
| 4 | GAB2_X88 + KCNJ15_X130 | C2 | 42 | 0.772 | [0.651 ; 0.892] | -3.7777 | 79.1 | 71.4 | 82.8 | 85.7 | 66.7 | N= 86 [28 (1); 58 (2)] |
| 5 | GAB2_X88 + KCNJ15_X193 | C2 | 51 | 0.778 | [0.662 ; 0.895] | -2.5714 | 77.9 | 78.6 | 77.6 | 88.2 | 62.9 | N= 86 [28 (1); 58 (2)] |
| 6 | GAB2_X88 + KCNJ15_X129 | C2 | 43 | 0.782 | [0.663 ; 0.901] | -2.0607 | 76.7 | 75.0 | 77.6 | 86.5 | 61.8 | N= 86 [28 (1); 58 (2)] |
| 7 | GAB2_X88 + KCNJ15_X190 | C2 | 48 | 0.774 | [0.656 ; 0.892] | -1.3815 | 75.6 | 75.0 | 75.9 | 86.3 | 60.0 | N= 86 [28 (1); 58 (2)] |
| 8 | MDM2_X207 + CAMK1 D_X125 | C2 | 31 | 0.779 | [0.676 ; 0.881] | -1.2096 | 74.4 | 78.6 | 72.4 | 87.5 | 57.9 | N= 86 [28 (1); 58 (2)] |
| 9 | LYN_X90 + KCNJ15_X129 | C2 | 77 | 0.770 | [0.655 ; 0.884] | -1.3684 | 74.4 | 71.4 | 75.9 | 84.6 | 58.8 | N= 86 [28 (1); 58 (2)] |
| 10 | MDM2_X207 + LYN_X90 | C2 | 2 | 0.812 | [0.712 ; 0.911] | -2.5068 | 73.3 | 85.7 | 67.2 | 90.7 | 55.8 | N= 86 [28 (1); 58 (2)] |
| 11 | LYN_X90 + CAMK1 D_X131 | C2 | 74 | 0.772 | [0.664 ; 0.879] | -0.8314 | 73.3 | 78.6 | 70.7 | 87.2 | 56.4 | N= 86 [28 (1); 58 (2)] |
| 12 | MDM2_X207 + GAB2_X88 | C2 | 1 | 0.785 | [0.685 ; 0.885] | -2.7339 | 72.1 | 82.1 | 67.2 | 88.6 | 54.8 | N= 86 [28 (1); 58 (2)] |
| 13 | MDM2_X207 + IFNAR1_X142 | C2 | 26 | 0.782 | [0.683 ; 0.881] | -2.2274 | 72.1 | 89.3 | 63.8 | 92.5 | 54.4 | N= 86 [28 (1); 58 (2)] |
| 14 | LYN_X90 + PDE8A_X116248 | C2 | 72 | 0.772 | [0.665 ; 0.878] | -7.3860 | 70.9 | 82.1 | 65.5 | 88.4 | 53.5 | N= 86 [28 (1); 58 (2)] |
| 15 | GAB2_X88 + LYN_X90 + KCNJ15_X130 | C3 | 635 | 0.823 | [0.714; 0.933] | -3.9804 | 83.7 | 67.9 | 91.4 | 85.5 | 79.2 | N= 86 [28 (1); 58 (2)] |
| 16 | GAB2_X88 + LYN_X90 + KCNJ15_X190 | C3 | 641 | 0.816 | [0.708 ; 0.924] | -1.7582 | 82.6 | 71.4 | 87.9 | 86.4 | 74.1 | N= 86 [28 (1); 58 (2)] |
| 17 | GAB2_X88 + KCNJ15_X134 + CAMK1D_X125 | C3 | 724 | 0.817 | [0.709 ; 0.924] | -1.7720 | 82.6 | 71.4 | 87.9 | 86.4 | 74.1 | N= 86 [28 (1); 58 (2)] |
| 18 | GAB2_X88 + KCNJ15_X134 + MDM2_X191 | C3 | 699 | 0.804 | [0.69 ; 0.919] | -2.4499 | 82.6 | 75.0 | 86.2 | 87.7 | 72.4 | N= 86 [28 (1); 58 (2)] |
| 19 | GAB2_X88 + KCNJ15_X134 + KCNJ15_X193 | C3 | 709 | 0.803 | [0.688 ; 0.918] | -1.5841 | 81.4 | 85.7 | 79.3 | 92.0 | 66.7 | N= 86 [28 (1); 58 (2)] |
| 20 | GAB2_X88 + KCNJ15_X134 + KCNJ15_X190 | C3 | 706 | 0.808 | [0.692 ; 0.924] | -3.0436 | 81.4 | 82.1 | 81.0 | 90.4 | 67.7 | N= 86 [28 (1); 58 (2)] |
| 21 | GAB2_X88 + KCNJ15_X133 + IFNAR1_X131 | C3 | 856 | 0.789 | [0.672 ; 0.906] | -3.0585 | 81.4 | 78.6 | 82.8 | 88.9 | 68.8 | N= 86 [28 (1); 58 (2)] |
| 22 | GAB2_X88 + KCNJ15_X133 + GAB2_X52 | C3 | 863 | 0.789 | [0.67 ; 0.909 ] | -2.3545 | 81.4 | 78.6 | 82.8 | 88.9 | 68.8 | N= 86 [28 (1); 58 (2)] |
| 23 | GAB2_X88 + KCNJ15_X133 + GAB2_X126 | C3 | 861 | 0.787 | [0.668 ; 0.906] | -3.0734 | 81.4 | 78.6 | 82.8 | 88.9 | 68.8 | N= 86 [28 (1); 58 (2)] |
| 24 | GAB2_X88 + KCNJ15_X134 + MDM2_X237 | C3 | 713 | 0.808 | [0.696 ; 0.921] | -3.3208 | 80.2 | 82.1 | 79.3 | 90.2 | 65.7 | N= 86 [28 (1); 58 (2)] |
| 25 | GAB2_X88 + KCNJ15_X134 + CAMK1D_X64 | C3 | 715 | GAB2_X88 0.797 | [0.681 ; 0.912] | -2.8067 | 80.2 | 82.1 | 79.3 | 90.2 | 65.7 | N= 86 [28 (1); 58 (2)] |
| 26 | GAB2_X88 + LYN_X90 + KCNJ15_X133 | C3 | 637 | 0.829 | [0.72 ; 0.938 ] | -3.0116 | 79.1 | 89.3 | 74.1 | 93.5 | 62.5 | N= 86 [28 (1); 58 (2)] |
| 27 | GAB2_X88 + LYN_X90 + KCNJ15_X134 | C3 | 632 | GAB2_X88 0.839 | [0.733 ; 0.945] | -2.5595 | 79.1 | 89.3 | 74.1 | 93.5 | 62.5 | N= 86 [28 (1); 58 (2)] |
| 28 | GAB2_X88 + LYN_X90 + KCNJ15_X134 + MDM2_X191 | C4 | 7175 | 0.840 | [0.734 ; 0.945] | -2.6812 | 86.1 | 78.6 | 89.7 | 89.7 | 78.6 | N= 86 [28 (1); 58 (2)] |
| 29 | MDM2_X207 + GAB2_X88 + L YN_X90 + MDM2_X192 | C4 | 19 | 0.841 | [0.742 ; 0.939] | -2.9468 | 84.9 | 78.6 | 87.9 | 89.5 | 75.9 | N= 86 [28 (1); 58 (2)] |
| 30 | MDM2_X207 + GAB2_X88 + L YN_X90 + CAMK1D_X131 | C4 | 3 | 0.854 | [0.76 ; 0.948 ] | -3.3309 | 83.7 | 78.6 | 86.2 | 89.3 | 73.3 | N= 86 [28 (1); 58 (2)] |
| 31 | GAB2_X88 + KCNJ15_X134 + MDM2_X237 + GAB2 X126 | C4 | 8592 | 0.820 | [0.709 ; 0.932] | -2.6335 | 82.6 | 82.1 | 82.8 | 90.6 | 69.7 | N= 86 [28 (1); 58 (2)] |
| 32 | GAB2_X88 + KCNJ15_X134 + KCNJ15_X129 + KCNJ15 _X190 | C4 | 8324 | 0.813 | [0.696 ; 0.93] | -3.7791 | 82.6 | 85.7 | 81.0 | 92.2 | 68.6 | N= 86 [28 (1); 58 (2)] |
| 33 | MDM2_X207 + KCNJ15_X134 + IFNAR1_X142 + IFNAR1 X128 | C4 | 2129 | 0.816 | [0.718; 0.914] | -3.0729 | 81.4 | 89.3 | 77.6 | 93.8 | 65.8 | N= 86 [28 (1); 58 (2)] |
| 34 | GAB2_X88 + LYN_X90 + KCNJ15_X134 + CAMK1D_X125 | C4 | 7200 | 0.852 | [0.752 ; 0.953] | -1.6869 | 81.4 | 82.1 | 81.0 | 90.4 | 67.7 | N= 86 [28 (1); 58 (2)] |
| 35 | MDM2_X207 + GAB2_X88 + LYN_X90 + KCNJ15_X134 + IFNAR1_X142 | C5 | 27 | 0.850 | [0.751 ; 0.949] | -3.8492 | 88.4 | 82.1 | 91.4 | 91.4 | 82.1 | N= 86 [ 28 (1); 58 (2)] |
| 36 | GAB2_X88 + LYN_X90 + KCNJ15_X134 + CAMK1D_X131 + PTPRC_X170 | C5 | 4005 | 0.854 | [0.753 ; 0.955] | -3.2924 | 87.2 | 71.4 | 94.8 | 87.3 | 87.0 | N= 86 [ 28 (1); 58 (2)] |
| 37 | GAB2 X88 + LYN X90 + KCNJ15_X134 + PRKCB_X103 + PRKCB_X132 | C5 | 4097 | 0.853 | [0.751 ; 0.955] | -2.7727 | 86.1 | 78.6 | 89.7 | 89.7 | 78.6 | N= 86 [ 28 (1); 58 (2)] |
| 38 | GAB2 X88 + LYN_X90 + MDM2_X191 + KCNJ15_X129 + PRKCB_X132 | C5 | 4214 | 0.828 | [0.722 ; 0.935] | -2.1014 | 86.1 | 75.0 | 91.4 | 88.3 | 80.8 | N= 86 [ 28 (1); 58 (2)] |
| 39 | GAB2_X88 + LYN_X90 + CAMK1D_X131 + KCNJ15_X130 + CAMK1D_X49 | C5 | 4118 | 0.845 | [0.744 ; 0.945] | -3.6631 | 84.9 | 82.1 | 86.2 | 90.9 | 74.2 | N= 86 [ 28 (1); 58 (2)] |
| 40 | GAB2_X88 + LYN_X90 + KCNJ15 _X134 + MDM2_X191 + KCNJ15_X190 | C5 | 4017 | 0.841 | [0.736 ; 0.946] | -3.3395 | 84.9 | 82.1 | 86.2 | 90.9 | 74.2 | N= 86 [ 28 (1); 58 (2)] |
| 41 | GAB2_X88 + LYN_X90 + PDE8A_X116248 + CAMK1D_X131 + KCNJ15_X129 | C5 | 3894 | 0.840 | [0.74 ; 0.94] | -5.7536 | 84.9 | 82.1 | 86.2 | 90.9 | 74.2 | N= 86 [ 28 (1); 58 (2)] |
| 42 | MDM2 X207 + LYN_X90 + KCNJ15_X134 + CAMK1D_X131 + KCNJ15_X133 | C5 | 940 | 0.860 | [0.77 ; 0.951] | -0.7551 | 82.6 | 92.9 | 77.6 | 95.7 | 66.7 | N= 86 [ 28 (1); 58 (2)] |
| 43 | MDM2_X207 + GAB2_X88 + LYN_X90 + KCNJ15_X134 + IFNAR1_X76 + IFNAR1_X142 | C6 | 185 | 0.849 | [0.75 ; 0.947] | -3.6239 | 88.4 | 85.7 | 89.7 | 92.9 | 80.0 | N= 86 [28 (1); 58 (2)] |
| 44 | GAB2_X88 + LYN_X90 + KCNJ15_X134 + MDM2_X191 + PRKCB_X103 + PRKCBX_X132 | C6 | 12353 | 0.858 | [0.756 ; 0.959] | -2.4262 | 88.4 | 78.6 | 93.1 | 90.0 | 84.6 | N= 86 [28 (1); 58 (2)] |
| 45 | MDM2_X207 + GAB2_X88 + LYN_X90 + KCNJ15_X134 + IFNAR1_X142 + IFNAR1_X128 | C6 | 219 | 0.853 | [0.756 ; 0.95] | -3.9043 | 87.2 | 85.7 | 87.9 | 92.7 | 77.4 | N= 86 [28 (1); 58 (2)] |
| 46 | GAB2_X88 + LYN_X90 + CAMK1D_X131 + KCNJ15_X129 + CAMK1D_X49 + PTPRC_X170 | C6 | 12809 | 0.855 | [0.755 ; 0.954] | -3.3713 | 87.2 | 82.1 | 89.7 | 91.2 | 79.3 | N= 86 [28 (1); 58 (2)] |
| 47 | GAB2_X88 + LYN_X90 + KCNJ15_X134 + IFNAR1_X142 + PRKCB_X103 + PRKCB_X132 | C6 | 12635 | 0.854 | [0.754 ; 0.954] | -2.5817 | 87.2 | 82.1 | 89.7 | 91.2 | 79.3 | N= 86 [28 (1); 58 (2)] |
| 48 | GAB2_X88 + LYN_X90 + KCNJ15_X133 + PRKCB_X103 +PRKCB_X126 + PRKCB_X132 | C6 | 13795 | 0.840 | [0.734 ; 0.946] | -2.9446 | 87.2 | 82.1 | 89.7 | 91.2 | 79.3 | N= 86 [28 (1); 58 (2)] |
| 49 | MDM2_X207 + GAB2_X88 + LYN_X90 + KCNJ15_X134 + CAMK1D_X131 + KCNJ15_X190 | C6 | 128 | 0.877 | [0.786 ; 0.968] | -5.1449 | 86.1 | 85.7 | 86.2 | 92.6 | 75.0 | N= 86 [28 (1); 58 (2)] |
| 50 | MDM2_X207 + GAB2_X88 + LYN_X90 + PDE8A_X116248 + CAMK1D_X49 + IFNAR1_X142 | C6 | 88 | 0.861 | [0.773 ; 0.949] | -7.4108 | 86.1 | 85.7 | 86.2 | 92.6 | 75.0 | N= 86 [28 (1); 58 (2)] |
| 51 | MDM2_X207 + GAB2_X88 + LYN_X90 + PDE8A_X116248 + KCNJ15_X134 + IFNAR1_X142 | C6 | 11 | 0.858 | [0.766 ; 0.95] | -7.0457 | 86.1 | 85.7 | 86.2 | 92.6 | 75.0 | N= 86 [28 (1); 58 (2)] |
| 52 | MDM2_X207 + GAB2_X88 + LYN_X90 + KCNJ15_X134 + KCNJ15 X190 + IFNAR1_X142 | C6 | 206 | 0.852 | [0.755 ; 0.948] | -5.4371 | 86.1 | 85.7 | 86.2 | 92.6 | 75.0 | N= 86 [28 (1); 58 (2)] |
| 53 | MDM2_X207 + KCNJ15_X134 + CAMK1D_X131 + MDM2_ X191 + IFNAR1_X142 + IFNAR1_X128 | C6 | 7332 | 0.819 | [0.724; 0.914] | -3.3357 | 83.7 | 89.3 | 81.0 | 94.0 | 69.4 | N= 86 [28 (1); 58 (2)] |
| 54 | MDM2_X207 + LYN_X90 + KCNJ15_X134 + CAMK1D_X131 + KCNJ15_X133 + CAMK1D_X49 | C6 | 3658 | 0.861 | [0.77 ; 0.951] | -0.8548 | 82.6 | 92.9 | 77.6 | 95.7 | 66.7 | N= 86 [28 (1); 58 (2)] |
| 55 | MDM2_X207 + GAB2_X88 + LYN_X90 + PDE8A_X116248 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + IFNAR1_X142 + PRKCB_X103 + PRKCB_X126 + PRKCB_X132 + IFNAR1_X128 | C19 | 6 | 0.909 | [0.833 ; 0.985] | -6.0428 | 88.4 | 92.9 | 86.2 | 96.2 | 76.5 | N= 86 [28 (1); 58 (2)] |
| 56 | MDM2_X207 + GAB2_X88 + LYN_X90 + PDE8A_X116248 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + PTPRC_X170 + PRKCB_X103 + PRKCB_X126 + PRKCB_X132 + IFNAR1_X128 | C19 | 5 | 0.908 | [0.829 ; 0.986] | -5.2338 | 88.4 | 89.3 | 87.9 | 94.4 | 78.1 | N= 86 [28 (1); 58 (2)] |
| 57 | MDM2_X207 + GAB2_X88 + LYN_X90 + PDE8A_X116248 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + PTPRC_X170 + PRKCB_X119 + KCNJ15_X193 + IFNAR1_X123 + IFNAR1_X131 + GAB2_X131 + MDM2_X237 + MDM2_X192 + CAMK1 D_X64 + GAB2_X126 + CAMK1D_X113 + GAB2_X52 + PRKCB_X103 + PRKCB_X126 + PRKCB_X132 + IFNAR1_X128 + CAMK1D_X125 + MDM2_X82 + PTPRC_X253 + PRKCB_X99 + IFNAR1_X151 + IFNAR1_X152 | C36 | 11 | 0.969 | [0.938 ; 0.999] | 36.3081 | 94.2 | 92.9 | 94.8 | 96.5 | 89.7 | N= 86 [28 (1); 58 (2)] |
| 58 | MDM2_X207 + GAB2_X88 + LYN_X90 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + PTPRC_X170 + PRKCB_X119 + KCNJ15_X193 + IFNAR1_X123 + IFNAR1_X131 + GAB2_X131 + MDM2_X237 + MDM2_X192 + CAMK1D_X64 + GAB2_X126 + CAMK1D_X113 + GAB2_X52 + IFNAR1_X142 + PRKCB_X103 + PRKCB_X126 + PRKCB_X132 + IFNAR1_X128 + CAMK1D_X125 + MDM2_X82 + PTPRC_X253 + PRKCB_X99 + IFNAR1_X151 + IFNAR1_X152 | C36 | 34 | 0.963 | [0.925 ; 1] | 46.2509 | 94.2 | 96.4 | 93.1 | 98.2 | 87.1 | N= 86 [28 (1); 58 (2)] |
| 59 | MDM2_X207 + GAB2_X88 + LYN_X90 + PDE8A_X116248 + KCNJ15_X134 + CAMK1D_X131 + MDM2_X191 + KCNJ15_X130 + KCNJ15 _X129 + KCNJ15_X133 + IFNAR1_X76 + CAMK1 D_X49 + MDM2_X87 + KCNJ15_X190 + PTPRC_X170 + PRKCB_X119 + KCNJ15_X193 + IFNAR1_X123 + IFNAR1_X131 + GAB2_X131 + MDM2 _X237 + MDM2_X192 + CAMK1D_X64 + GAB2_X126 + CAMK1D_X113 + GAB2_X52 + IFNAR1_X142 + PRKCB_X103 + PRKCB_X126 + PRKCB_X132 + CAMK1D_X125 + MDM2_X82 + PTPRC_X253 + PRKCB_X99 + IFNAR1_X151 + IFNAR1_X152 | C36 | 7 | 0.967 | [0.935 ; 0.999] | 15.6122 | 89.5 | 100.0 | 84.5 | 100.0 | 75.7 | N= 86 [28 (1); 58 (2)] |
| 60 | MDM2_X207 + IFNAR1_X101 + GAB2_X88 + MDM2_X76 + MDM2_X143 + LYN_X90 + PDE8A_X116248 + KCNJ15_X134 + MDM2_X86 + CAMK1D_X131 + KCNJ15_X130 + KCNJ15_X129 + KCNJ15 _X133 + IFNAR1_X76 + CAMK1D_X49 + MDM2_X87 + LYN_X155_X + KCNJ15_X131 + PTPRC_X169 + MDM2_X277 + KCNJ15_X190 + PTPRC_X170 + PTPRC_X259 + PTPRC_X163 + PRKCB_X119 + KCNJ15_X193 + MDM2_X264 + IFNAR1_X123 + IFNAR1_X131 + GAB2_X131 + MDM2_X237 + MDM2_X192 + CAMK1D_X64 + GAB2_X126 + CAMK1D_X113 + GAB2_X52 + IFNAR1_X142 + PRKCB_X103 + PRKCB_X126 + PRKCB_X132 + IFNAR1_X128 + PDE8A_X116278 + CAMK1D_X125 + PTPRC_X253 + PRKCB_X99 + IFNAR1_X151 + IFNAR1_X152 + PRKCB_X183 | C48 | 747 | 0.993 | [0.981 ; 1] | -84.5770 | 97.7 | 100.0 | 96.6 | 100.0 | 93.3 | N= 86 [ 28 (1); 58 (2)] |

Altogether, these data suggest that the different biomarkers in the panel of 14 genes selected in the prioritization cohorts could be used to sequentially diagnose MDD. Importantly, all the identified biomarkers included in this panel of 14 genes were included in at least one combination, suggesting that all those BMKs could be used to diagnose MDD with suitable diagnostic performances.
A panel of the 8 most interesting biomarkers was selected and sequenced in a multiplex format to further validate our results in a large cohort (validation cohort) of 411 subjects, of which 143 were healthy controls and 268 were MDD patients (Figure 19). When we analyzed the editing sites of the whole population and combined 17 editing variants in 5 BMKs, we obtained an AUC of 0.751 (Sp 70.6%; Se 72.4%, Figure 6A). When the 8 biomarkers included in the multiplex were used and 53 differentially edited sites were combined, the AUC rose to 0.824, with a Specificity of 76.2% and Sensitivity of 81% (Figure 6B). When the number of editing sites in those 8 BMKs was further increased to reach 79 sites, all of which were sequenced in multiplex in a single experiment, the AUC further increased to 0.899 (Sp 82.5%; Se 81.7%, Figure 6C). In the latter case, the equation used is the following: Z= - (-0.181885362537332)* MDM2_Site68821586_W - (0.45875321689627)* MDM2_SWX + (0.932293378426399)* PRKCB_Site23920910_J - ( 0.235744342326864)* MDM2_W - (0.188045930057369)* MDM2_Site68821643 + ( 0.129971821791191)* PRKCB_DIJK - (0.112543222902224)* MDM2_SVW + ( 0.0714766020787205)* CAMK1D_E - (-0.0566149561843161)* MDM2_SW + ( 0.343458398647021)* PRKCB_B - (0.0511754835571903)* GAB2_Site78331054 + (0.0777964634309061)* PRKCB_Site23920911 + (-0.309760257245042)* PRKCB_Site23920919_K + (0.0892196021964463)* MDM2_T + ( 0.0591702551145443)* PRKCB_J - (0.163034043783367)* KCNJ15_CE - (-0.3554615992579)* MDM2_Site68821616_X + (0.194383441652219)* CAMK1D_AE - (-0.0357242710579534)* KCNJ15_C + (-0.137804039545265)* PRKCB_JK - (0.073268779527063)* GAB2_CE - (0.583431075372027)* PDE8A_Site85096687_B - (0.083656284732413)* KCNJ15_Site38287529_C + ( 0.241597825714507)* PRKCB_Site23920902_I + (0.0897642626007787)* PRKCB_DIJ + (0.311212124874424)* PRKCB_Site23920893_G - (-0.0694365074403336)* KCNJ15_Site38287586 - (0.356961148091174)* KCNJ15_Site38287525_B - (0.0284155839590126)* MDM2_Site68821573 + ( 0.161274457136425)* IFNAR1.1_Site33355809 - (-0.578010271214577)* PDE8A_B + (0.00936832259402957)* PRKCB_Site23920921_L - (-0.0220552696975651)* MDM2_WX + (0.0416981587266934)* IFNAR1.1_Site33355817 - (0.056383762969179)* KCNJ15_Site38287589_E + ( 0.034447202518999)* IFNAR1.1_Site33355838_P - (0.181855602464856)* KCNJ15_Site38287590 + (-0.232428154178395)* PRKCB_Site23920884_D - ( 0.0566507858081719)* KCNJ15_Site38287530_D + (0.00350968357773277)* PRKCB_IJK - (0.0986696717892891)* KCNJ15_Site38287591 + ( 0.0416357072635886)* PRKCB_Site23920889_F + (-0.510062501581064)* PRKCB_Site23920844_B + (-0.201713338082801)* PRKCB_DJ - ( 0.219330533848547)* LYN_AC + (-0.0902753379195889)* IFNAR1.1_Site33355798 + (0.0364611306918377)* PRKCB_DHIJK + (-0.0154244319603596)* GAB2_BCDE + (-0.0721590982562632)* IFNAR1.1_Site33355801_N + (0.0351216920222297)* CAMK1D_A + ( 0.130383083484889)* LYN_AG + (0.136853965680293)* GAB2_Site78331030 - ( 0.201599671305856)* CAMK1D_Site12378440 + (-0.0494625908197476)* IFNAR1.1_Site33355793_G - (0.112438468962481)* MDM2_KSX + ( 0.129569099073175)* IFNAR1.1_Site33355839_L - (0.12561875043809)* GAB2_ABDE - (0.127875837752046)* PRKCB_DJM - (-0.421356734328941)* KCNJ15_ACE - (0.125572361520418)* MDM2_ASV - (-0.0421188089161209)* MDM2_Site68821576_V + (0.333082982874752)* LYN_A + (0.205974310972437)* IFNAR1.1_Site33355762_F - (0.26063881758559)* MDM2_Site68821602 - ( 0.16091158013623)* LYN_Site56012491 - (0.585856554487505)* PRKCB_Site23920927_M - (-0.328951375049536)* KCNJ15_Site38287526_A + ( 0.0681422708577013)* GAB2_EJ + (0.0265646151191535)* PRKCB_I + (-0.114728592530574)* LYN_Site56012490 + (0.0423564487829415)* CAMK1D_Site12378387 + (-0.270495232628549)* IFNAR1.1_Site33355807_B - ( 0.202529212838335)* PRKCB_Site23920952 - (-0.0881700257476326)* KCNJ15_ACD + (0.0213312643325165)* GAB2_DE + (-0.00540846808767115)* MDM2_STX + (0.18510874500411)* GAB2_Site78331008_D - ( 0.060213548631588)* MDM2_Site68821567 - (-0.123438884158084)* MDM2_Site68821612_A
The list of combinations tested on the whole population in the validation cohort (n=411), is presented in Table 6. All combinations were statistically significant between control and cases with a p value <10⁻⁴.

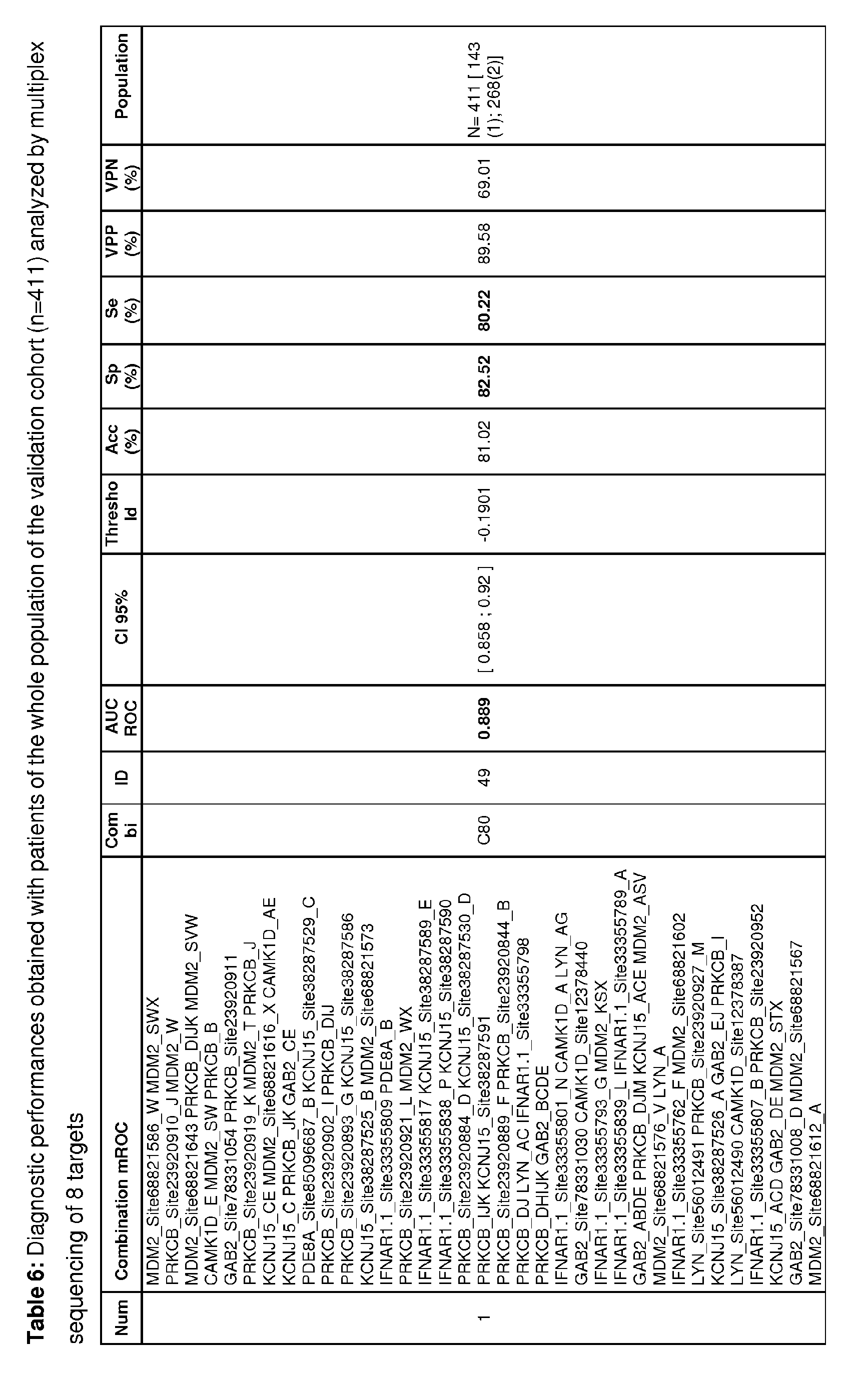

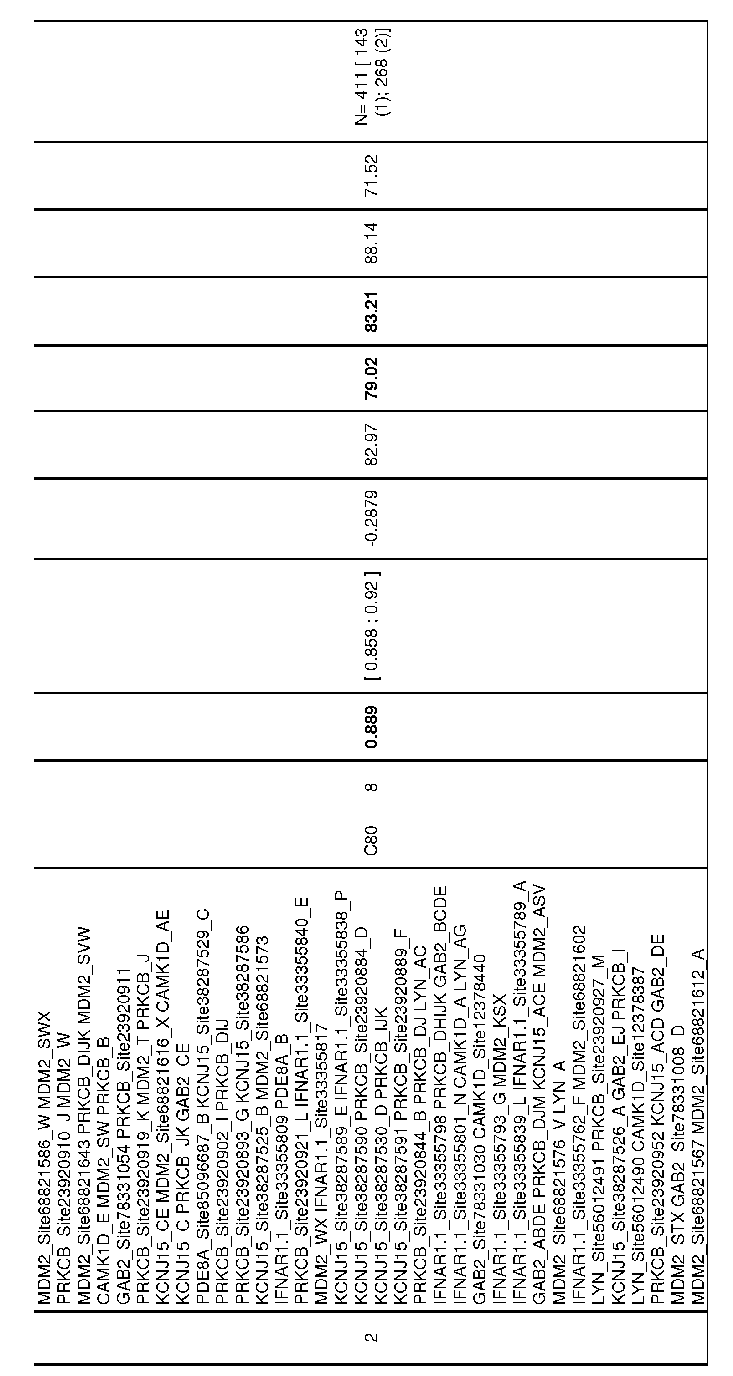

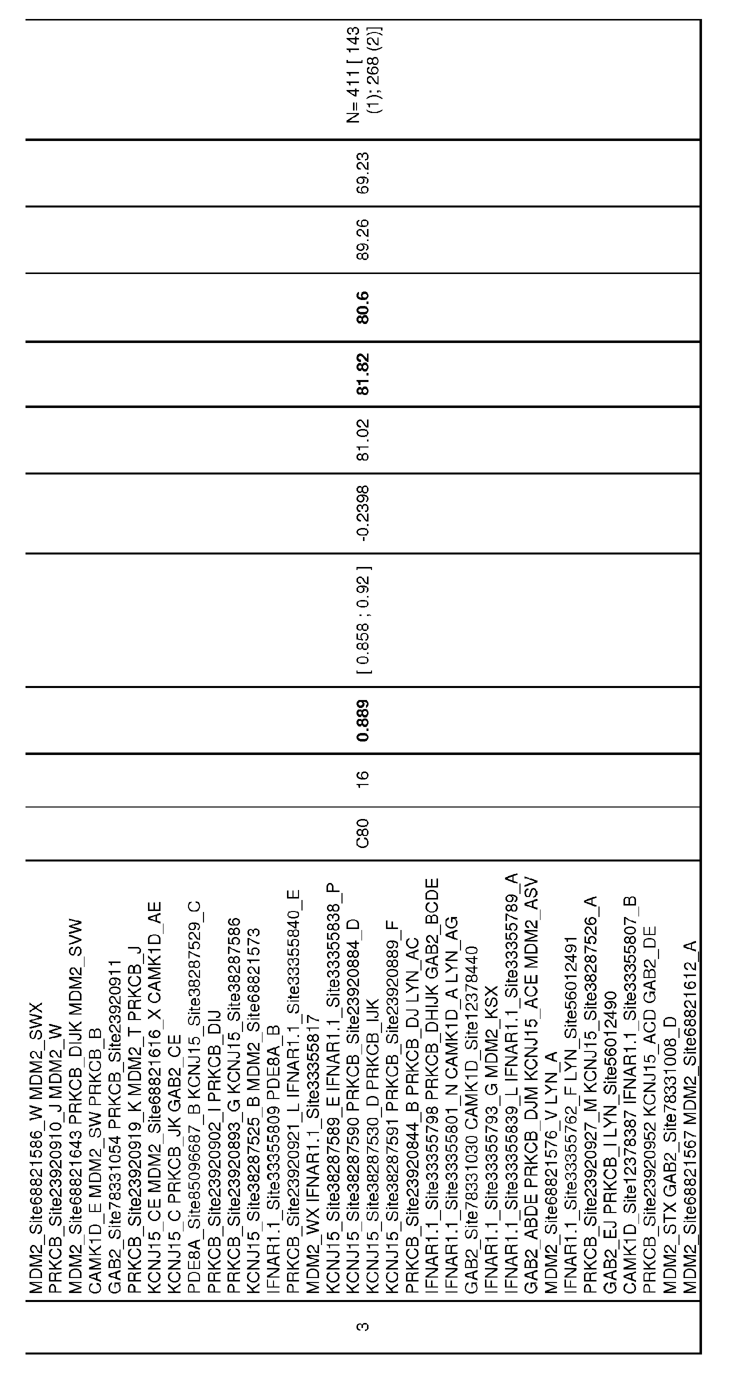

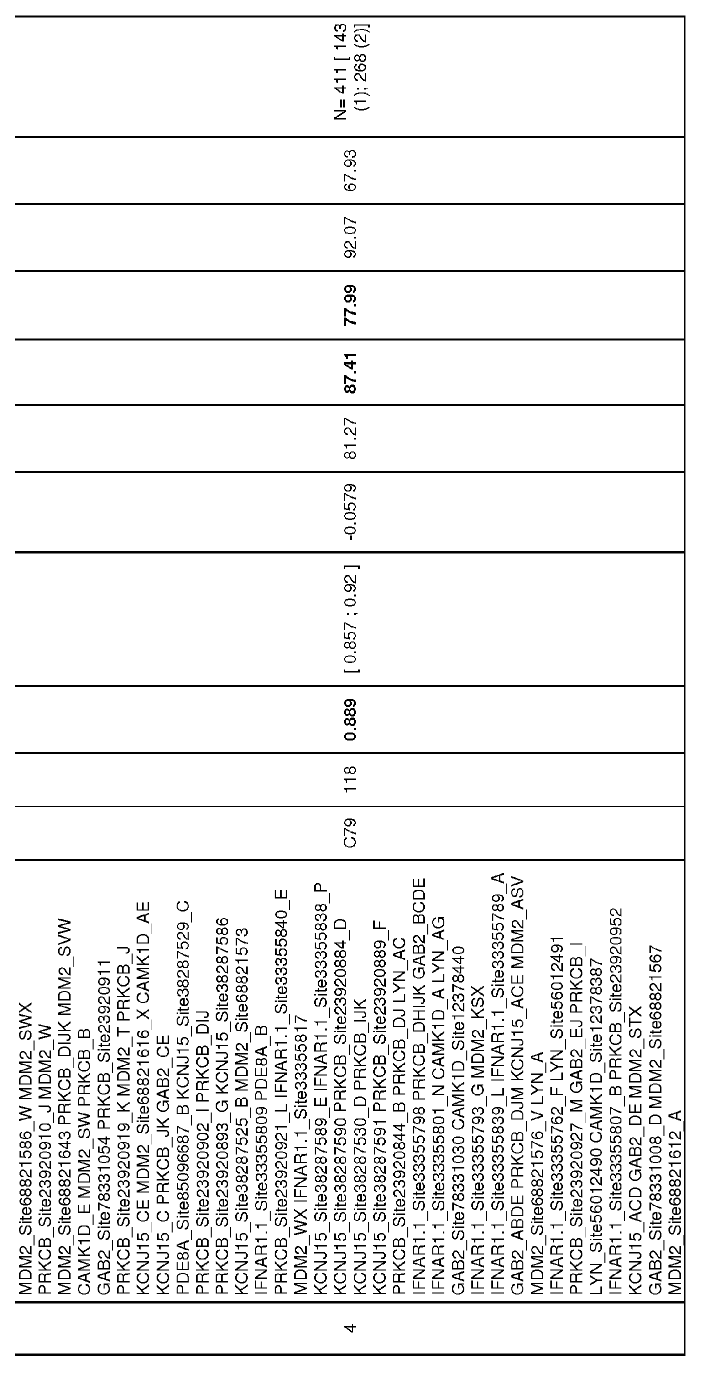

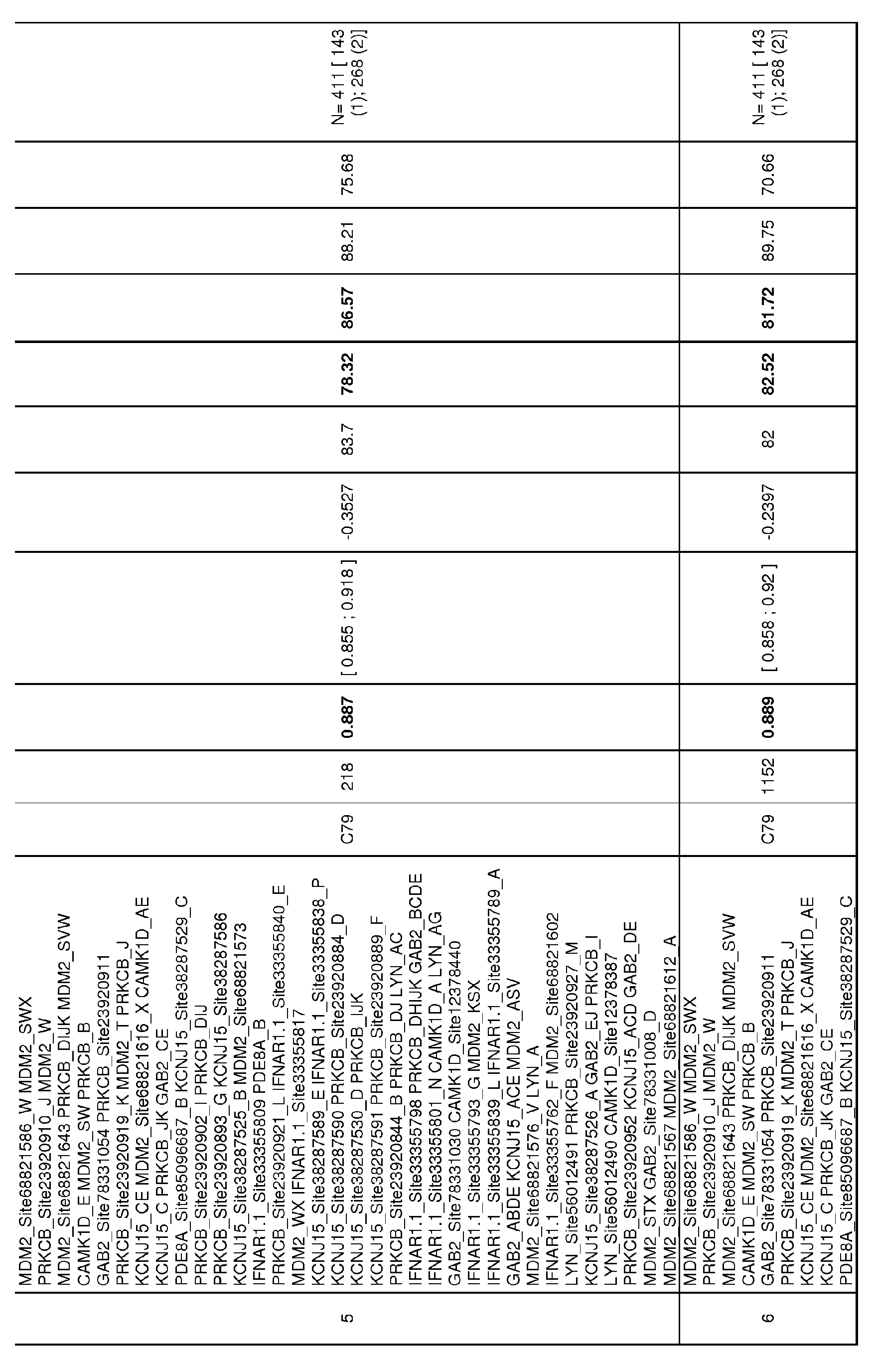

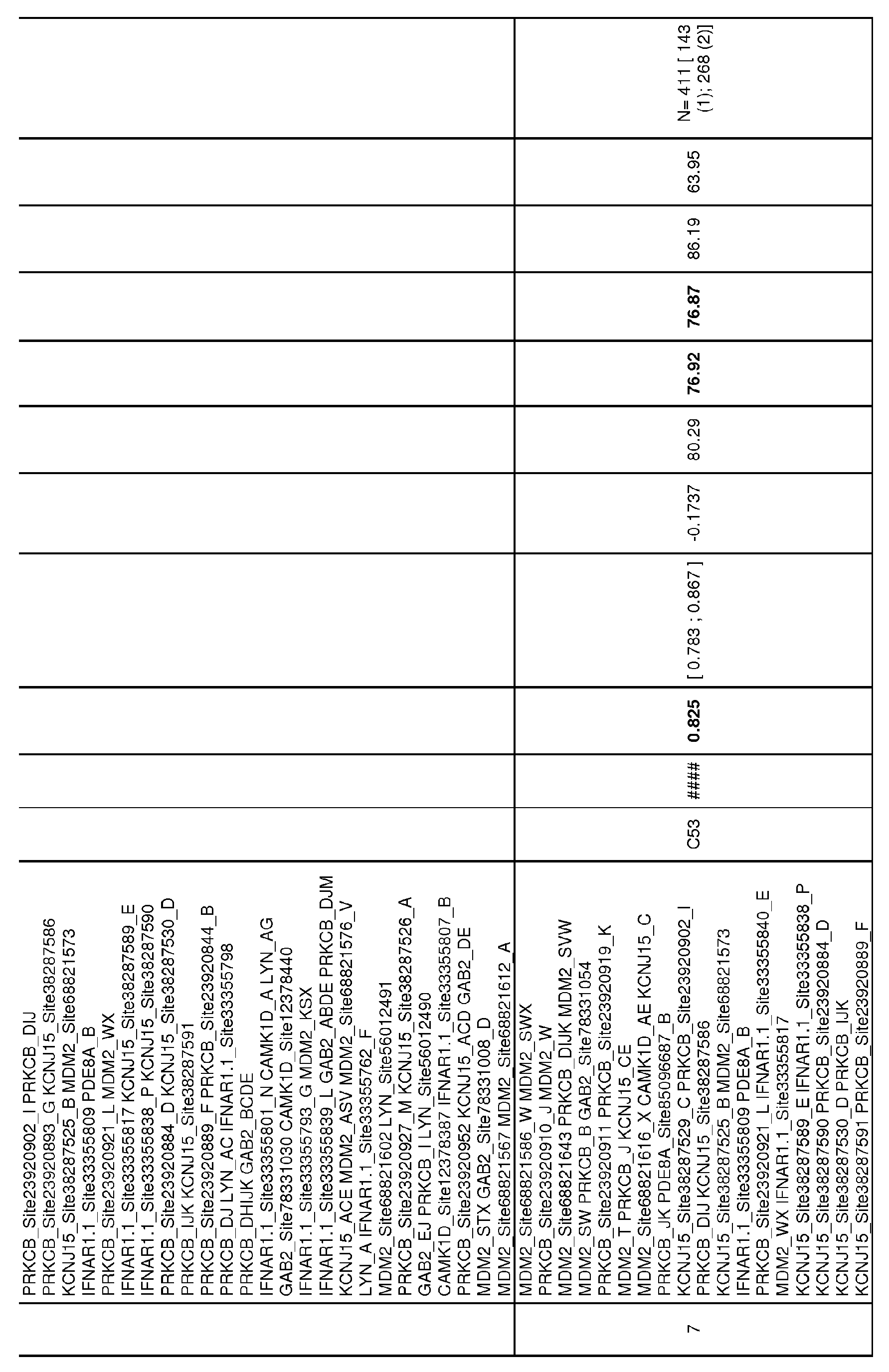

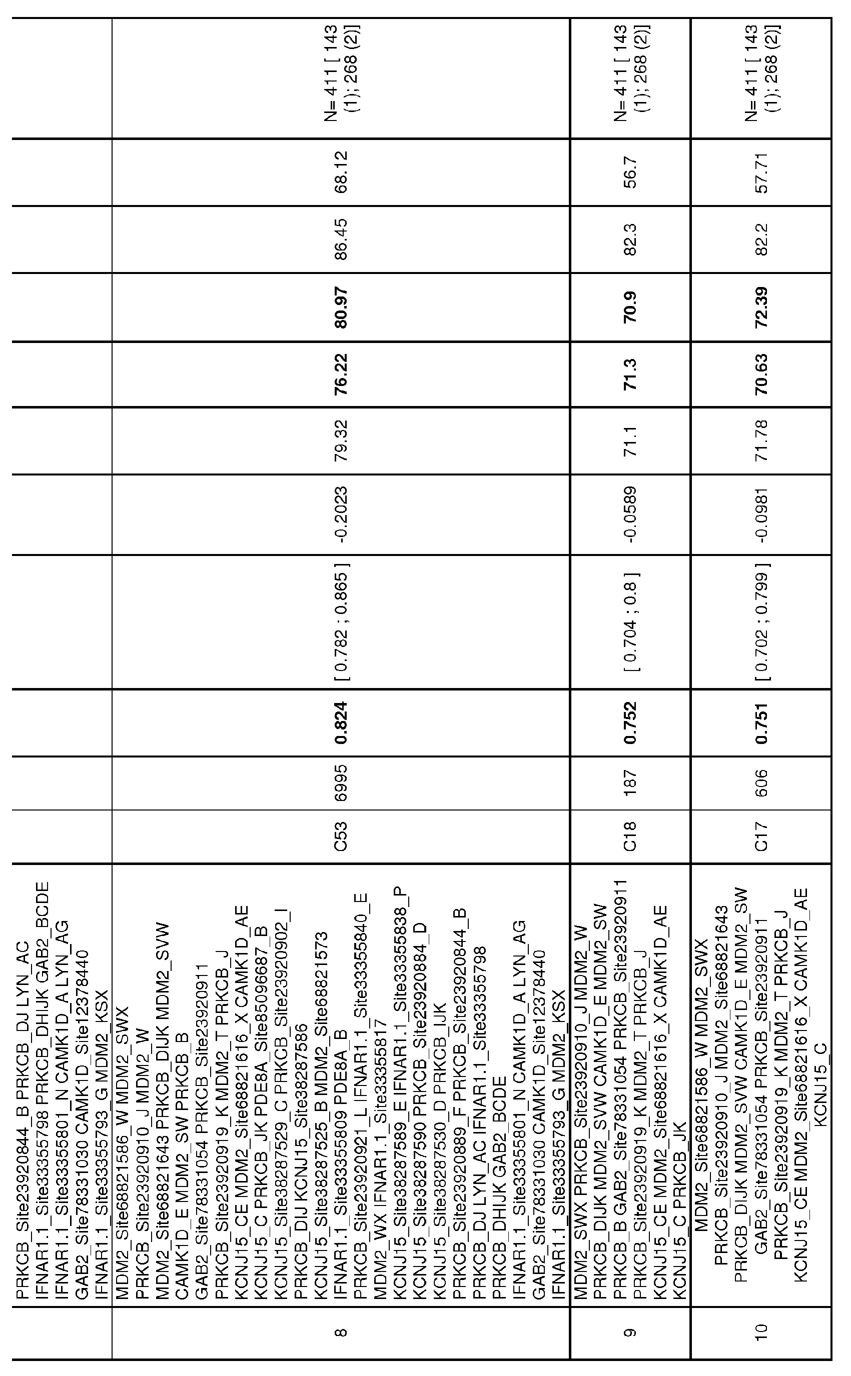

The panel of biomarkers analyzed above thus gave excellent diagnostic performances on to diagnose MDD the whole population of the validation cohort (411 subjects), suggesting that our panel of biomarkers could indeed have clinical applications in the diagnosis of MDD.
We next sought to investigate whether the diagnostic performances of our assay could be further increased by taking into account sex-specific differences. Indeed, the existence of sex-specific transcriptional signatures has been suggested in MDD. It has previously been shown that the brain transcriptional profile of MDD differs greatly by sex, suggesting that the molecular mechanisms underlying MDD could differ between women and men⁴². Modifications in RNA editing were thus analyzed separately in the male and the female population of the validation cohort. When sex-specificity was introduced in the analysis, a significant improvement of diagnostic performances was observed. Noteworthy, the female validation cohort (n=277) included more subjects than the male validation cohort (n=134), as this is reflecting the proportion of males and females suffering MDD in the general population worldwide. Indeed, in the female population of the validation cohort (n=277, 90 healthy controls and 187 MDD patients), when we combined 11 RNA editing variants, representing 4 different BMKS, were combined, the AUC was 0.784 (Sp 71.1%; Se 78.6%, Figure 7A). The equation used in this case is the following: Z= - (-0.000533736120198011)* MDM2_Site68821586_W - (0.211359639047036)* MDM2_W - (0.146264452332436)* MDM2_SWX - (0.160086235452429)* GAB2_Site78331054 - (0.090792634422732)* MDM2_SW + (0.117672779615208 )* MDM2_T + (0.209398709260439)* CAMK1 D_AE + (0.115253600910406)* PRKCB_J - (0.193553830436994)* CAMK1D_BC + (0.0404496406797322)* CAMK1D_E + (0.0771184775348071)* PRKCB_Site23920910_J

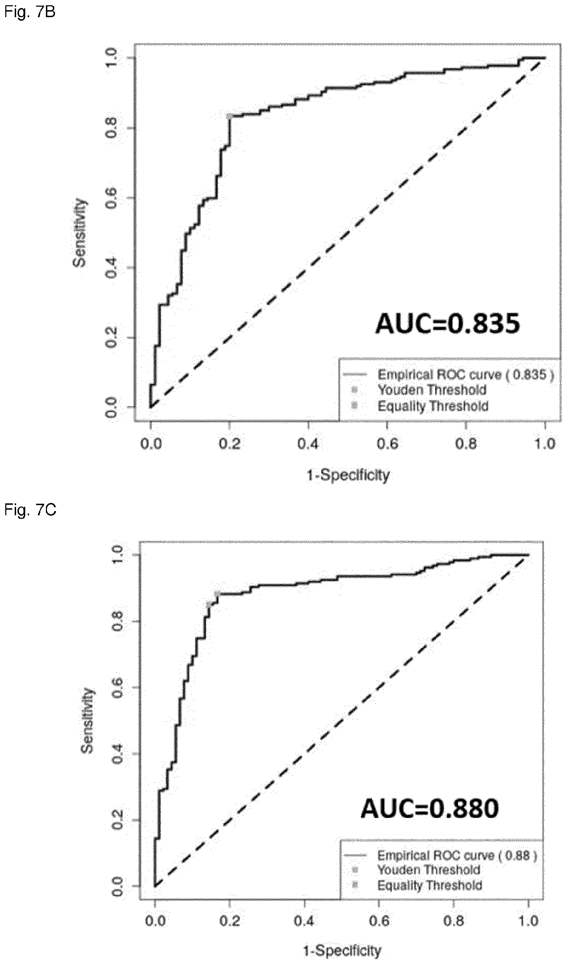

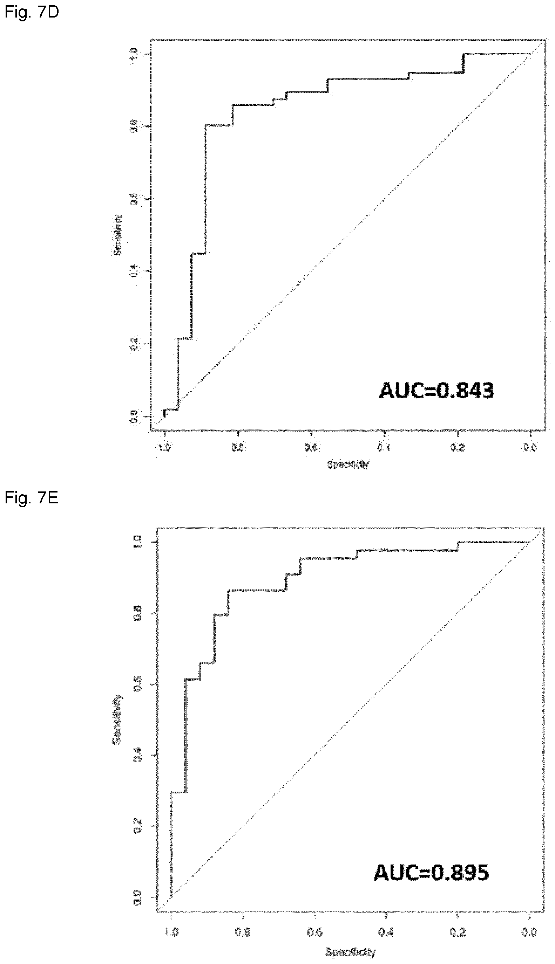

When the number of biomarkers was increased to 6 different targets and included 30 RNA editing variants, the AUC rose to 0.835 (Sp 80.0%; Se 83.4%, Figure 7B). Moreover, when we used the 8 biomarkers included in the multiplex and combined 49 differentially edited sites, the AUC reached to 0.880, with both specificity and sensitivity above 80% (Sp 83.3%; Se 88.2%, Figure 7C).
Using machine-learning approach with both mROC and RandomForest (see material and methods), we randomly separated the sex-specific populations into a train- and a test set. When randomly splitting the female population into a train set (75% of the whole population) and a test set (25% of the whole population) and applying the above-mentioned methods, we obtained an AUC of 0.843 in the test set with the mROC method (Sp 88.9%; Se 80.4%, Figure 7D) and an AUC of 0.895 with the MultDS RandomForest method (Sp 86%; Se 80%, Figure 7E). Altogether, the data obtained by two different machine-learning methods on the female validation cohort confirmed the excellent diagnostic performances of our panel of biomarkers.
The list of combinations tested in the female validation cohort (n=277), is presented in Table 16. All combinations were statistically significant between control and cases with a p value <10⁻⁴..

**Table 16: Diagnostic performances obtained with patients of the female population of the validation cohort analyzed by multiplex sequencing of 8 targets.**

| **Num** | **Combination mROC** | **Combi** | **ID** | **AUC ROC** | **CI 95%** | **Threshold** | **Acc (%)** | **Sp (%)** | **Se (%)** | **VPP (%)** | **VPN (%)** | **Population** |
|---|---|---|---|---|---|---|---|---|---|---|---|---|
| **1** | MDM2_Site68821586_W MDM2_W MDM2_SWX GAB2_Site78331054 MDM2_SW MDM2_T CAMK1D_AE PRKCB_J CAMK1D_BC CAMK1D_E MDM2_Site68821612_A MDM2_Site68821576_V MDM2_Site68821643 PRKCB _Site23920910_J MDM2_Site68821573 PRKCB_DJ MDM2_Site68821616_X CAMK1D_AF PRKCB_DHIJK MDM2_Site68821570 MDM2_Site68821602 MDM2_WX MDM2_SVW MDM2_SVWX CAMK1D_Site12378375_G PRKCB_I CAMK1D_A PDE8A_Site85096687_B CAMK1D_Site12378321_C PRKCB_Site23920952 MDM2_X LYN_A CAMK1D_ABG PRKCB_JK CAMK1D_FS GAB2_Site78331030 MDM2_K KCNJ15_C GAB2_IJ PRKCB_Site23920921_L PRKCB_IJK IFNAR1.1_Site33355809 MDM2_Site68821572_T GAB2_BDEHJ GAB2_H PRKCB_AD PDE8A_B KCNJ15_CD GAB2_BCDJ IFNAR1.1 Site33355840_E | C50 | 443 | **0.883** | [0.841 ; 0.925] | -0.2642 | 85.6 | **84.4** | **86.1** | 92.0 | 74.5 | N= 277 [90 (1); 187 (2)] |
| **2** | MDM2_Site68821586_W MDM2_W MDM2_SWX GAB2_Site78331054 MDM2_SW MDM2_T CAMK1D_AE PRKCB_J CAMK1 D_BC CAMK1D_E MDM2_Site68821612_A MDM2 _Site68821576_V MDM2_Site68821643 PRKCB_Site23920910_J MDM2_Site68821573 PRKCB_DJ MDM2_Site68821616_X CAMK1 D_AF PRKCB_DHIJK MDM2_Site68821570 MDM2_Site68821602 MDM2_WX MDM2_SVW MDM2_SVWX CAMK1D_Site12378375_G PRKCB_I CAMK1D_A PDE8A_Site85096687_B CAMK1D_Site12378321_C PRKCB_Site23920952 MDM2_X LYN_A CAMK1 D_ABG PRKCB_JK CAMK1D_FS GAB2_Site78331030 MDM2_K KCNJ15_C GAB2_IJ PRKCB_Site23920921_LPRKCB_IJK IFNAR1.1_Site33355809 MDM2_Site68821572_T GAB2_BEJ GAB2_BDEHJ GAB2_H PDE8A_B KCNJ15_CD GAB2_BCDJ IFNAR1.1_Site33355840_E | C50 | 440 | **0.880** | [0.837 ; 0.923] | -0.2692 | 85.2 | **85.6** | **85.0** | 92.4 | 73.3 | N= 277 [90 (1); 187 (2)] |
| **3** | MDM2_Site68821586_W MDM2_W MDM2_SW MDM2_T CAMK1 D_AE PRKCB_J CAMK1 D_BC CAMK1D_E MDM2_Site68821612_A MDM2_Site68821576_V PRKCB_Site23920910_J MDM2_Site68821573 PRKCB_DJ MDM2_Site68821616_X CAMK1 D_AF PRKCB_DHIJK MDM2_Site68821570 MDM2_Site68821602 PRKCB_DIJK MDM2_WX MDM2_SVW MDM2_SVWX CAMK1D_Site12378375_G PRKCB_I CAMK1D-A PDE8A_Site85096687_B CAMK1D_Site12378321_C PRKCB_Site23920952 MDM2_X LYN_A CAMK1D_ABG PRKCB_JK CAMK1 D_FS GAB2_Site78331030 MDM2_K KCNJ15_C GAB2_IJ PRKCB_Site23920921_L PRKCB_IJK IFNAR1.1_Site33355809 MDM2_Site68821572_T GAB2_BEJ GAB2_BDEHJ GAB2_H PRKCB_AD PDE8A_B KCNJ15_CD GAB2_BCDJ IFNAR1.1_Site33355840_E | C49 | 19592 | **0.880** | [0.834 ; 0.925] | -0.3719 | 87.0 | **80.0** | **90.4** | 90.4 | 80.0 | N= 277 [90 (1); 187 (2)] |
| **4** | MDM2_Site68821586_W MDM2_W MDM2_SWX MDM2_SW MDM2_T CAMK1 D_AE PRKCB_J CAMK1 D_BC CAMK1 D_E MDM2_Site68821612_A MDM2 _Site68821576_V MDM2_Site68821643 PRKCB_Site23920910_J MDM2_Site68821573 MDM2_Site68821616_X CAMK1 D_AF PRKCB_DHIJK MDM2_Site68821570 MDM2_Site68821602 PRKCB_DIJK MDM2_WX MDM2_SVW MDM2_SVWX CAMK1D_Site12378375_G PRKCB_I CAMK1 D_A PDE8A_Site85096687_B CAMK1D_Site12378321_C PRKCB_Site23920952 MDM2_X LYN_A CAMK1 D_ABG PRKCB_JK CAMK1 D_FS GAB2_Site78331030 MDM2_K KCNJ15_C PRKCB_Site23920921_L PRKCB_IJK IFNAR1.1_Site33355809 MDM2_Site68821572_T GAB2_BEJ GAB2_BDEHJ GAB2_H PRKCB_AD PDE8A_B KCNJ15_CD GAB2_BCDJ IFNAR1.1 Site33355840_E | C49 | 17939 | **0.880** | [0.835 ; 0.924] | -0.2916 | 86.6 | **83.3** | **88.2** | 91.7 | 77.3 | N= 277 [90 (1); 187 (2)] |
| **5** | MDM2_Site68821586_W MDM2_W MDM2_SWX MDM2_SW MDM2_T CAMK1D_AE PRKCB_J CAMK1D_BC MDM2_Site68821612_A MDM2_Site68821643 PRKCB_Site23920910_J MDM2_Site68821573 PRKCB_DJ MDM2_Site68821616_X CAMK1D_AF PRKCB_DHIJK MDM2_Site68821570 MDM2_Site68821602 PRKCB_DIJK MDM2_WX MDM2_SVW MDM2_SVWX CAMK1D_Site12378375_G PRKCB_I CAMK1D_A PDE8A_Site85096687_B CAMK1D_Site12378321_C PRKCB_Site23920952 MDM2_X LYN_A CAMK1 D_ABG PRKCB_JK CAMK1D_FS GAB2_Site78331030 MDM2_K KCNJ15_C GAB2_IJ PRKCB_Site23920921_L PRKCB_IJK IFNAR1.1_Site33355809 MDM2_Site68821572_T GAB2_BEJ GAB2_BDEHJ GAB2_H PRKCB_AD PDE8A_B KCNJ15_CD GAB2_BCDJ IFNAR1.1_Site33355840_E | C49 | 18198 | **0.879** | [0.835 ; 0.924] | -0.3089 | 86.6 | **82.2** | **88.8** | 91.2 | 77.9 | N= 277 [90 (1); 187 (2)] |
| **6** | MDM2_Site68821586_W MDM2_SWX GAB2_Site78331054 MDM2_SW MDM2_T CAMK1 D_AE PRKCB_J CAMK1 D_BC CAMK1 D_E MDM2_ Site68821612_A MDM2_ Site68821576_V MDM2_ Site68821643 PRKCB_ Site23920910 _J MDM2_ Site68821573 PRKCB_ DJ MDM2_Site68821616_X CAMK1 D_AF PRKCB_ DHIJK MDM2_ Site68821570 MDM2_ Site68821602 PRKCB_ DIJK MDM2_WX MDM2_SVW PRKCB_I CAMK1 D_A PDE8A_ Site85096687_ B CAMK1D_Site12378321_C PRKCB_Site23920952 MDM2_ X_LYN_ A | C31 | 4531 | **0.835** | [0.782 ; 0.887] | -0.2749 | 82.3 | **80.0** | **83.4** | 89.7 | 69.9 | N= 277 [90 (1); 187 (2)] |
| **7** | MDM2_Site68821586_W MDM2_SWX GAB2_Site78331054 MDM2_SW MDM2_T CAMK1D_AE PRKCB_J CAMK1 D_BC CAMK1 D_E MDM2_ Site68821612_A MDM2_ Site68821576 _V MDM2_ Site68821643 PRKCB_ Site23920910 _J MDM2_ Site68821573 PRKCB_ DJ MDM2_Site68821616_X CAMK1D_AF PRKCB_DHIJK MDM2_Site68821570 PRKCB_DIJK MDM2_WX MDM2_SVW CAMK1D_Site12378375_G PRKCB I CAMK1D_A PDE8A_Site85096687_B CAMK1D_Site12378321_C PRKCB_Site23920952 MDM2_X LYN_A | C31 | 4570 | **0.834** | [0.781 ; 0.886] | -0.279 | 82.3 | **80.0** | **83.4** | 89.7 | 69.9 | N= 277 [90 (1); 187 (2)] |
| **8** | MDM2_Site68821586_W MDM2_W MDM2_SWX GAB2_Site78331054 MDM2_SW MDM2_T CAMK1D_AE PRKCB_J CAMK1 D_BC CAMK1D_E MDM2_Site68821612_A MDM2_Site68821576_V PRKCB_Site23920910_J MDM2_Site68821573 PRKCB_DJ MDM2_Site68821616_X PRKCB_DHIJK MDM2_Site68821570 MDM2_Site68821602 PRKCB_DIJK MDM2_WX MDM2_SVW CAMK1 D_Site12378375_G PRKCB_I CAMK1D_A PDE8A_Site85096687_B CAMK1D_Site12378321_C PRKCB_Site23920952 MDM2_X LYN_A | C31 | 1254 | **0.833** | [0.78 ; 0.886] | -0.1426 | 80.9 | **81.1** | **80.8** | 89.9 | 67.0 | N= 277 [90 (1); 187 (2)] |
| **9** | MDM2_Site68821586_W MDM2_W GAB2_Site78331054 MDM2_T CAMK1 D_AE PRKCB_J CAMK1D_BC CAMK1D_E MDM2_Site68821612_A MDM2_Site68821576_V MDM2_Site68821643 | C11 | 202 | **0.781** | [0.723 ; 0.838] | -0.0475 | 74.7 | **78.9** | **72.7** | 87.7 | 58.2 | N= 277 [90 (1); 187 (2)] |
| **10** | MDM2_W MDM2_SWX GAB2_Site78331054 MDM2_T CAMK1D_AE PRKCB_J CAMK1D_BC CAMK1D_E MDM2_Site68821612_A MDM2_Site68821643 PRKCB Site23920910 J | C11 | 325 | **0.787** | [0.731 ; 0.842] | -0.0183 | 73.7 | **80.0** | **70.6** | 88.0 | 56.7 | N= 277 [90 (1); 187 (2)] |
| **11** | MDM2_Site68821586_W MDM2_W MDM2_SWX GAB2_Site78331054 MDM2_SW MDM2_T CAMK1D_AE PRKCB_J CAMK1D_BC CAMK1D_E PRKCB_Site23920910_J | C11 | 4 | **0.784** | [0.729 ; 0.84] | -0.1626 | 76.2 | **71.1** | **78.6** | 85.0 | 61.5 | N= 277 [90 (1); 187 (2)] |

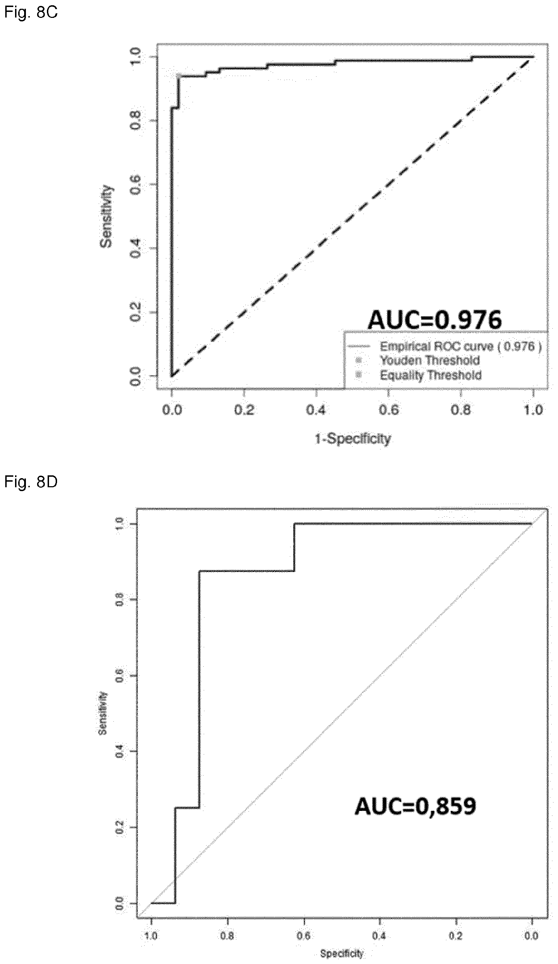

The performances of the male population were even better. Indeed, when we analyzed separately the male population of the validation cohort (n=134, 53 healthy controls and 81 MDD patients) and included 8 editing variants in 6 BMKs, we obtained an AUC of 0.831 (Sp 83.0%; Se 75.3%, Figure 8A). In this case, the equation used is the following: Z= - (0.238400918804649)* GAB2_CH - (0.230156347297484)* CAMK1 D_DS - ( 0.19154950723228)* KCNJ15_Site38287525_B - (0.21914392389275)* GAB2_CE - (0.155783908162178)* LYN_Site56012491 - (0.268738279594405)* MDM2_SVW - (0.107036976834934)* MDM2_Site68821643 + (0.400819265369961)* PRKCB_Site23920893_G.
With 7 biomarkers, including 39 RNA editing variants, the AUC was above 0.9 (AUC 0.936, Spe 92.5%, Se 87.7%, figure 8B). When the 8 biomarkers included in the panel were combined, with 68 editing variants in these BMKS, we obtained an outstanding AUC of 0.976 (Spe 98.1%, Se 93.8%, Figure 8C). Using machine-learning, we randomly split the male population into a training (70% of the whole population) and a test set (30% of the whole population) and obtained an AUC with mROC was 0.859 (Sp 87.5%, Se 87.5%, Figure 8D), while the RandomForest method led to an AUC of 0.875 (Sp 82%, Se 80%, Figure 8E).

The list of combinations tested in the male validation cohort (n=134), is presented in Table 8. All combinations were statistically significant between control and cases with a p value <10⁻⁴.

**Table 8: Diagnostic performances obtained with patients of the male population (n=134) of the validation cohort analyzed by multiplex sequencing of 8 targets.**

| **Num** | **Combination mROC** | **Combi** | **ID** | **AUC ROC** | **CI 95%** | **Threshold** | **Acc (%)** | **Sp (%)** | **Se (%)** | **VPP (%)** | **VPN (%)** | **Population** |
|---|---|---|---|---|---|---|---|---|---|---|---|---|
| 1 | GAB2_CH CAMK1 D_DS KCNJ15_Site38287525_B GAB2_CE LYN_Site56012491 LYN_AC KCNJ15_Site38287530_D MDM2_SVW MDM2_Site68821643 KCNJ15_ACE PRKCB_Site23920893_G KCNJ15_Site38287529_C CAMK1 D_BF KCNJ15_CE PDE8A_E KCNJ15_ACD MDM2_KST GAB2_JK PRKCB_JM PRKCB_ADJK CAMK1D_D GAB2_Site78330939_H KCNJ15_Site38287526_A CAMK1D_R GAB2_ABDE KCNJ15_C KCNJ15_Site38287531 CAMK1D_AF GAB2_H GAB2_DH GAB2_Site78331054 LYN_Site56012495 MDM2_KSX LYN_Site56012468_C MDM2_BG LYN_Site56012521 LYN_Site56012556_F KCNJ15_AC PRKCB_DIJK GAB2_Site78331011_C KCNJ15_ABC PRKCB_DJM PDE8A_Site85096729_F CAMK1 D_F GAB2_DEHJ KCNJ15_Site38287591 LYN_Site56012567 PRKCB_Site23920911 LYN_Site56012553_A MDM2_SWX KCNJ15_Site38287586 CAMK1 D_FS GAB2_DK KCNJ15_AB PRKCB_Site23920889_F PRKCB_AJ PRKCB_Site23920919_K LYN_Site56012469_D PRKCB_Site23920910_J IFNAR1.1_AC LYN_Site56012446 PRKCB_Site23920884_D IFNAR1.1_C GAB2_BCDE LYN_Site56012568 PRKCB_IJK IFNAR1.1_AB PDE8A_B | C68 | 1992 | 0.976 | [0.952 ; 1] | -0.7316 | 95.5 | **98.1** | **93.8** | 98.7 | 91.2 | N= 134 [53 (1); 81 (2)] |
| 2 | GAB2_CH CAMK1 D_DS KCNJ15_Site38287525_B LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C CAMK1D_BF KCNJ15_CE PDE8A_E KCNJ15_ACD MDM2_KST GAB2_JK PRKCB_JM PRKCB_ADJK CAMK1 D_D GAB2_Site78330939_H KCNJ15_Site38287526_A CAMK1D_R GAB2_ABDE KCNJ15_C KCNJ15_Site38287531 CAMK1 D_AF GAB2_H GAB2_DH LYN_Site56012495MDM2_KSX LYN_Site56012468_C MDM2_BG LYN_Site56012521 LYN_Site56012556_F KCNJ15_AC PRKCB_DIJK GAB2_Site78331011_C KCNJ15_ABC PRKCB_DJM PDE8A_Site85096729_F CAMK1 D_F GAB2_DEHJ KCNJ15_Site38287591 LYN_Site56012567 PRKCB_Site23920911 LYN_Site56012553_A MDM2_SWX KCNJ15_Site38287586 CAMK1 D_FS GAB2_DK KCNJ15_AB PRKCB_Site23920889_F PRKCB_AJ PRKCB_Site23920919_K LYN_Site56012469_D PRKCB_Site23920910_J IFNAR1.1_AC LYN_Site56012446 PRKCB_Site23920884_D IFNAR1.1_C GAB2_BCDE LYN_Site56012568 PRKCB_IJK IFNAR1.1_AB PDE8A_B | C67 | 46501 | 0.975 | [0.953 ; 0.997] | -0.679 | 95.5 | **100.0** | **92.6** | 100.0 | 89.8 | N= 134 [53 (1); 81 (2)] |
| 3 | GAB2_CH CAMK1 D_DS KCNJ15_Site38287525_B GAB2_CE LYN_Site56012491 LYN_AC KCNJ15_Site38287530_D MDM2_SVW MDM2_Site68821643 KCNJ15_ACE PRKCB_Site23920893_G CAMK1D_BF KCNJ15_CE PDE8A_E KCNJ15_ACD MDM2_KST GAB2_JK PRKCB_JM PRKCB_ADJK CAMK1D_D GAB2_Site78330939_H KCNJ15 _Site38287526_A CAMK1D_R GAB2_ABDE KCNJ15_C KCNJ15_Site38287531 CAMK1D_AF GAB2_H GAB2_DH GAB2_Site78331054 LYN_Site56012495 MDM2_KSX LYN_Site56012468_C MDM2_BG LYN_Site56012521 LYN_Site56012556_F KCNJ15_AC PRKCB_DIJK GAB2_Site78331011_C KCNJ15 ABC PRKCB_DJM PDE8A_Site85096729_F CAMK1 D_F GAB2_DEHJ KCNJ15_Site38287591 LYN_Site56012567 PRKCB_Site23920911 LYN_Site56012553_A MDM2_SWX KCNJ15_Site38287586 CAMK1 D_FS GAB2_DK KCNJ15_AB PRKCB_Site23920889_F PRKCB_AJ PRKCB_Site23920919_K LYN_Site56012469_D PRKCB_Site23920910_J IFNAR1.1_AC LYN_Site56012446 PRKCB_Site23920884_D IFNAR1.1_C GAB2_BCDE LYN_Site56012568 PRKCB_IJK IFNAR1.1_AB PDE8A_B | C67 | 41346 | 0.977 | [0.952 ; 1] | -0.7686 | 95.5 | **98.1** | **93.8** | 98.7 | 91.2 | N= 134 [53 (1); 81 (2)] |
| 4 | GAB2_CH CAMK1D_DS KCNJ15_Site38287525_B GAB2_CE LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C CAMK1D_BF KCNJ15_CE PDE8A_E KCNJ15_ACD MDM2_KST GAB2_JK PRKCB_JM PRKCB_ADJK CAMK1 D_D GAB2_Site78330939_H KCNJ15_Site38287526_A CAMK1 D_R GAB2_ABDE KCNJ15_C KCNJ15_Site38287531 CAMK1 D_AF GAB2_H GAB2_DH PRKCB_B GAB2_Site78331054 LYN_Site56012495 MDM2_KSX LYN_Site56012468_C MDM2_BG LYN_Site56012556_F KCNJ15_AC PRKCB_DIJK GAB2_Site78331011_C KCNJ15_ABC PRKCB_DJM PDE8A_Site85096729_F CAMK1 D_F GAB2_DEHJ KCNJ15_Site38287591 LYN_Site56012567 PRKCB_Site23920911 LYN_Site56012553_A MDM2_SWX KCNJ15_Site38287586 CAMK1D_FS GAB2_DK PRKCB_Site23920889_F PRKCB_AJ PRKCB_Site23920919_K LYN_Site56012469_D PRKCB_Site23920910_J IFNAR1.1_AC LYN_Site56012446 IFNAR1.1_C GAB2_BCDE LYN_Site56012568 PRKCB_IJK IFNAR1.1_AB PDE8A_B | C67 | 5058 | 0.980 | [0.958 ; 1] | -1.2498 | 95.5 | **96.2** | **95.1** | 97.5 | 92.7 | N= 134 [53 (1); 81 (2)] |
| 5 | GAB2_CH CAMK1D_DS KCNJ15_Site38287525_B GAB2_CE LYN_Site56012491 LYN_AC LYN_AF KCNJ15_ Site38287530_D MDM2_SVW MDM2_Site68821643 KCNJ15_ACE PRKCB_Site23920893_G KCNJ15_Site38287529_C CAMK1D_BF KCNJ15_CE PDE8A_E KCNJ15_ACD GAB2_JK PRKCB_ADJK CAMK1D_D GAB2_Site78330939_H KCNJ15_Site38287526_A CAMK1 D_R GAB2_ABDE KCNJ15_ C KCNJ15_Site38287531 CAMK1D_AF GAB2_H GAB2_DH PRKCB_B GAB2_Site78331054 LYN_ Site56012495 MDM2_KSX LYN_ Site56012468_C LYN_ Site56012521 KCNJ15_AC KCNJ15_ABC PRKCB_DJM PDE8A Site85096729_F | C39 | 2261 | 0.936 | [0.898 ; 0.975] | -0.0614 | 88.1 | **100.0** | **80.3** | 100.0 | 76.8 | N= 134 [53 (1); 81 (2)] |
| 6 | GAB2_CH CAMK1D_DS KCNJ15_Site38287525_B GAB2_CE LYN_Site56012491 LYN_AC LYN_AF KCNJ15_ Site38287530 _D MDM2_SVW MDM2_Site68821643 KCNJ15_ACE PRKCB_Site23920893_G KCNJ15_Site38287529_C KCNJ15_CE PDE8A_E KCNJ15_ACD MDM2_KST GAB2_JK PRKCB_ADJK CAMK1D_D GAB2_Site78330939_H KCNJ15_Site38287526_A CAMK1 D_R GAB2_ABDE KCNJ15_ C KCNJ15_Site38287531 CAMK1D_AF GAB2_H GAB2_DH PRKCB_B GAB2_Site78331054 MDM2_KSX LYN_ Site56012468_C MDM2_BG LYN_ Site56012521 KCNJ15 _AC KCNJ15 _ABC PRKCB _DJM PDE8A Site85096729_F | C39 | 3516 | 0.936 | [0.896 ; 0.975] | -0.3892 | 89.6 | **92.5** | **87.7** | 94.7 | 83.1 | N= 134 [53 (1); 81 (2)] |
| 7 | GAB2_CH CAMK1D_DS KCNJ15_Site38287525_B GAB2_CE LYN_Site56012491 LYN_AC LYN_AF KCNJ15_ Site38287530_D MDM2_SVW MDM2_Site68821643 KCNJ15_ACE PRKCB_Site23920893_G KCNJ15_Site38287529_C KCNJ15_CE PDE8A_E KCNJ15_ACD MDM2_KST GAB2_JK PRKCB_JM PRKCB_ADJK CAMK1D_D GAB2_Site78330939_H KCNJ15_Site38287526_A CAMK1D_R GAB2_ABDE KCNJ15_C KCNJ15_Site38287531 CAMK1D_AF GAB2_H GAB2_DH PRKCB_B GAB2_Site78331054 LYN_Site56012495 MDM2_KSX LYN_Site56012468_C LYN_Site56012521 KCNJ15_AC KCNJ15_ABC PRKCB_DJM | C39 | 3287 | 0.937 | [0.898 ; 0.975] | -0.4206 | 89.6 | **88.7** | **90.1** | 92.4 | 85.5 | N= 134 [53 (1); 81 (2)] |
| 8 | GAB2_CH CAMK1D_DS KCNJ15_Site38287525_B GAB2_CE LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C KCNJ15_CE PDE8A_E KCNJ15_ACD MDM2_KST GAB2_JK PRKCB_JM PRKCB_ADJK CAMK1D_D GAB2_Site78330939_H KCNJ15_Site38287526_A CAMK1D_R GAB2_ABDE KCNJ15_C CAMK1D_AF GAB2_H GAB2_DH PRKCB_B GAB2_Site78331054 LYN_Site56012495 MDM2_KSX MDM2_BG LYN_Site56012521 KCNJ15_AC KCNJ15_ABC PRKCB_DJM PDE8A_Site85096729_F | C39 | 3374 | 0.943 | [0.908 ; 0.979] | -0.2029 | 88.1 | **96.2** | **82.7** | 97.1 | 78.5 | N= 134 53 (1); 81 (2)] |
| 9 | GAB2_CH CAMK1D_DS KCNJ15_Site38287525_B GAB2_CE LYN_Site56012491 LYN_AC KCNJ15_Site38287530_D MDM2_SVW PRKCB Site23920893_G | C9 | 8 | 0.833 | [0.764 ; 0.901] | -0.1683 | 78.4 | **79.3** | **77.8** | 85.1 | 70.0 | N= 134 [53(1); 81 (2)] |
| 10 | GAB2_CH CAMK1D_DS GAB2_CE LYN_Site56012491 LYN_AC LYN_AF MDM2_Site68821643 PRKCBSite23920893_G | C8 | 62 | 0.806 | [0.731 ; 0.881] | -0.2557 | 79.9 | **71.7** | **85.2** | 82.1 | 76.0 | N= 134 [53 (1); 81 (2)] |
| 11 | GAB2_CH CAMK1D_DS KCNJ15_Site38287525_B GAB2_CE LYN_Site56012491 MDM2_SVW MDM2_ Site68821643_PRKCB_Site23920893_G | C8 | 20 | 0.831 | [0.763 ; 0.899] | -0.1107 | 78.4 | **83.0** | **75.3** | 87.1 | 68.8 | N= 134 [53 (1); 81 (2)] |

The data obtained with both methods thus gave excellent diagnostic performances for sex-specific diagnosis of MDD, with AUCs above 0.850, specificities and sensitivities above 80% for both females and males, indicative of a diagnostic assay that could be used into the clinic.

As we have identified RNA editing biomarkers for the diagnosis of MDD, we sought to investigate whether the non-edited isoforms of the validated targets could be useful to discriminate the control and patient populations and might be used in combination with RNA editing biomarkers. The AUC ROC for each non-edited target was then calculated, along with the significance for each non-edited isoform. Analysis of the non-edited isoforms of PRKCB, MDM2, IFNAR1 and PDE8A on the validation cohort (n=411 subjects) gave AUCs above 0.5; the non -edited isoforms of both PRKCB and MDM2 were significantly different between control and MDD (p<0.05, Table 9).

**Table 9: Diagnostic performances obtained with non-edited isoforms on the validation cohort analyzed with multiplex sequencing;**

| **Target** | **P-value** | **AUC ROC** | **% Nonedited** | **Population** |
|---|---|---|---|---|
| PRKCB non-edited | 0.0001 | 0.622 | 43.6 | N= 411 [143 (0) ; 268 (1)] |
| MDM2 non-edited | 0.0132 | 0.573 | 33.7 | N= 411 [143 (0) ; 268 (1)] |
| IFNAR1 non-edited | 0.0785 | 0.553 | 74.4 | N= 411 [143 (0) ; 268 (1)] |
| PDE8A non-edited | 0.0915 | 0.551 | 78.1 | N= 411 [143 (0) ; 268 (1)] |

In addition, when the non-edited isoforms were combined with one isoform of the same targets, we observed significant differences between healthy control and MDD patients (Table 19), suggesting that the non-edited isoforms could also be used in the diagnostic assay;

**Table 19: Diagnostic performances obtained with non-edited isoforms combined with edited targets on the validation cohort analyzed with multiplex sequencing;**

| **Target** | **Ratio** | **p-value** | **AUC ROC** | **Population** |
|---|---|---|---|---|
| **CAMK1D** | BGNS / non-edited | 0.0002 | 0.615 | N= 411 [143 (0) ; 268 (1)] |
| **CAMK1D** | ABCDEFRS / non-edited | 0.0002 | 0.613 | N= 411 [143 (0) ; 268 (1)] |
| **CAMK1D** | ABCEFGR / non-edited | 0.0007 | 0.599 | N= 411 [143 (0) ; 268 (1)] |
| **CAMK1D** | BCM / non-edited | 0.0008 | 0.599 | N= 411 [143 (0) ; 268 (1)] |
| **CAMK1D** | BEIRS / non-edited | 0.0009 | 0.599 | N= 411 [143 (0) ; 268 (1)] |
| **CAMK1D** | ABDG/ non-edited | 0.0026 | 0.592 | N= 411 [143 (0) ; 268(1.)] |
| **GAB2** | AEFHI / non-edited | 0.0005 | 0.603 | N= 411 [143 (0) ; 268 (1)] |
| **IFNAR1** | ABCEFGILNP / non-edited | 0.001 | 0.601 | N= 411 [143 (0) ; 268 (1)] |
| **MDM2** | KOSX / non-edited | 0.0007 | 0.602 | N= 411 [143 (0) ; 268 (1)] |
| **MDM2** | AJSWX / non-edited | 0.0003 | 0.609 | N= 411 [143 (0) ; 268 (1)] |
| **MDM2** | OP / non-edited | 0.0007 | 0.602 | N= 411 [143 (0) ; 268 (1)] |
| **PRKCB** | BDFIJK / non-edited | 0.0006 | 0.601 | N= 411 [143 (0) ; 268 (1)] |
| **PRKCB** | DJP / non-edited | 0.0008 | 0.600 | N= 411 [143 (0) ; 268 (1)] |
| **PRKCB** | BN / non-edited | 0.0009 | 0.600 | N= 411 [143 (0) ; 268 (1)] |
| **PRKCB** | CDFIJK / non-edited | 0.0013 | 0.593 | N= 411 [143 (0) ; 268 (1)] |
| **PRKCB** | BJKM / non-edited | 0.0024 | 0.591 | N= 411 [143 (0) ; 268 (1)] |
| **PRKCB** | AGHIJKLM / non-edited | 0.0031 | 0.594 | N= 411 [143 (0) ; 268 (1)] |
| **PRKCB** | DJ / non-edited | 0.0003 | 0.612 | N= 411 [143 (0) ; 268 (1)] |

### Conclusion

To our knowledge, this research is the first study to investigate RNA editing modifications to be used as blood biomarkers to diagnose mood disorders. We performed an editome analysis of MDD in patients recruited from the outpatients of the Department of Emergency Psychiatry and Post-Acute Care (CHRU of Montpellier). The presence of psychiatric diagnoses was confirmed by trained psychiatrists, who managed the Montgomery-Åsberg Depression Rating Scale (MADRS) in french and the 30-item Inventory of Depressive Symptomatology, Clinician Rated (IDS-C30) to score depression. We sought association with depression in the editome of patients following RNA-Seq study in a discovery cohort of 64 subjects. We identified 646 A-to-I editing variants, representing 366 genes, which were differentially edited between depressed patients and healthy controls. This gene set showed a strong enrichment for biological processes including multiple immune categories, thus confirming a strong immune component in the physiopathology of MDD⁴³. The list of variants was then narrowed down to a subset of 14 genes (17 variants) by using much stringent criteria, and validated these biomarkers individually in a prioritization cohort of 86 individuals. These genes also showed a strong enrichment in genes related to regulation of immune system process. In addition, some were strongly related to pathways involved in neuronal system and neurotransmitter receptor-related pathways. Finally, we identified different combinations of a panel of 8 markers and validated this combination in a large cohort of 411 patients. Moreover, when sex specificity was introduced, and a machine-learning approach was used, the performances of the diagnostic assay were substantially increased. Our findings identified combinations of biomarkers with high diagnostic performances, suitable to be clinically useful to objectively diagnose depressive disorders from blood samples.

We previously identified alterations of the serotonin receptor 5HT2CR and PDE8A mRNA editing in brain samples of suicide decedents by capillary electrophoresis single-strand conformation 5 polymorphism (CE-SSCP)^{5,44}. We recently demonstrated altered RNA editing in psychiatric patients with chronic hepatitis C virus (HCV) undergoing antiviral combination therapy with IFN-α and ribavirin, in whom treatment-emergent depression is a common complication²². In the latter study, measurements of editing levels were based on targeted next-generation sequencing (NGS), which provides both high throughput and sufficient per base depth to allow reliable quantification for all sites studied.

### References

1. American Psychiatric Association. Diagnostic and Statistical Manual of Mental Disorders. 5th ed. Washington, DC. 2013.
2. Dunlop K, Talishinsky A, Liston C. Intrinsic Brain Network Biomarkers of Antidepressant Response: a Review. Current psychiatry reports. Aug 13 2019;21(9):87.
3. Jentsch MC, Van Buel EM, Bosker FJ, et al. Biomarker approaches in major depressive disorder evaluated in the context of current hypotheses. Biomarkers in medicine. 2015;9(3):277-297.
4. Smith KM, Renshaw PF, Bilello J. The diagnosis of depression: current and emerging methods. Comprehensive psychiatry. Jan 2013;54(1):1-6.
5. Chimienti F, Cavarec L, Vincent L, et al. Brain region-specific alterations of RNA editing in PDE8A mRNA in suicide decedents. Translational psychiatry. Feb 15 2019;9(1):91.
6. Dobin A, Gingeras TR. Optimizing RNA-Seq Mapping with STAR. Methods in molecular biology. 2016;1415:245-262.
7. DePristo MA, Banks E, Poplin R, et al. A framework for variation discovery and genotyping using next-generation DNA sequencing data. Nature genetics. May 2011;43(5):491-498.
8. John D, Weirick T, Dimmeler S, Uchida S. RNAEditor: easy detection of RNA editing events and the introduction of editing islands. Briefings in bioinformatics. Nov 1 2017;18(6):993-1001.
9. Yang H, Wang K. Genomic variant annotation and prioritization with ANNOVAR and wANNOVAR. Nature protocols. Oct 2015;10(10):1556-1566.
10. Tempel S. Using and understanding RepeatMasker. Methods in molecular biology. 2012;859:29-51.
11. Kasprzyk A, Keefe D, Smedley D, et al. EnsMart: a generic system for fast and flexible access to biological data. Genome research. Jan 2004;14(1):160-169.
12. Langmead B, Salzberg SL. Fast gapped-read alignment with Bowtie 2. Nature methods. Mar 4 2012;9(4):357-359.
13. Li H, Handsaker B, Wysoker A, et al. The Sequence Alignment/Map format and SAMtools. Bioinformatics. Aug 15 2009;25(16):2078-2079.
14. Li H. A statistical framework for SNP calling, mutation discovery, association mapping and population genetical parameter estimation from sequencing data. Bioinformatics. Nov 1 2011;27(21):2987-2993.
15. Gentleman RC, Carey VJ, Bates DM, et al. Bioconductor: open software development for computational biology and bioinformatics. Genome biology. 2004;5(10):R80.
16. Linden A. Measuring diagnostic and predictive accuracy in disease management: an introduction to receiver operating characteristic (ROC) analysis. Journal of evaluation in clinical practice. Apr 2006;12(2):132-139.
17. Kramar A, Faraggi D, Fortune A, Reiser B. mROC: a computer program for combining tumour markers in predicting disease states. Computer methods and programs in biomedicine. Sep 2001;66(2-3):199-207.
18. Breiman L. Random Forests. Machine Learning. Kluwer Academic Publishers. 2001;45:5-32.
19. Consortium GO. Gene Ontology Consortium: going forward. Nucleic acids research. Jan 2015;43(Database issue):D1049-1056.
20. Raudvere U, Kolberg L, Kuzmin I, et al. g:Profiler: a web server for functional enrichment analysis and conversions of gene lists (2019 update). Nucleic acids research. Jul 2 2019;47(W1):W191-W198.
21. Pomaznoy M, Ha B, Peters B. GOnet: a tool for interactive Gene Ontology analysis. BMC bioinformatics. Dec 7 2018;19(1):470.
22. Salvetat N, Van der Laan S, Vire B, et al. RNA editing blood biomarkers for predicting mood alterations in HCV patients. Journal of neurovirology. Jul 22 2019.
23. Szklarczyk D, Gable AL, Lyon D, et al. STRING v11: protein-protein association networks with increased coverage, supporting functional discovery in genome-wide experimental datasets. Nucleic acids research. Jan 8 2019;47(D1):D607-D613.
24. Shelton RC. The molecular neurobiology of depression. The Psychiatric clinics of North America. Mar 2007;30(1):1-11.
25. Costas J, Gratacos M, Escaramis G, et al. Association study of 44 candidate genes with depressive and anxiety symptoms in post-partum women. Journal of psychiatric research. Aug 2010;44(11):717-724.
26. Colledge M, Snyder EM, Crozier RA, et al. Ubiquitination regulates PSD-95 degradation and AMPA receptor surface expression. Neuron. Oct 30 2003;40(3):595-607.
27. Liu B, Mink S, Wong KA, et al. PIAS1 selectively inhibits interferon-inducible genes and is important in innate immunity. Nature immunology. Sep 2004;5(9):891-898.
28. Murakami Y, Ishibashi T, Tomita E, et al. Depressive symptoms as a side effect of Interferon-alpha therapy induced by induction of indoleamine 2,3-dioxygenase 1. Scientific reports. Jul 20 2016;6:29920.
29. Brodie EJ, Infantino S, Low MSY, Tarlinton DM. Lyn, Lupus, and (B) Lymphocytes, a Lesson on the Critical Balance of Kinase Signaling in Immunity. Frontiers in immunology. 2018;9:401.
30. Hayashi T, Umemori H, Mishina M, Yamamoto T. The AMPA receptor interacts with and signals through the protein tyrosine kinase Lyn. Nature. Jan 7 1999;397(6714):72-76.
31. Moura-Alves P, Fae K, Houthuys E, et al. AhR sensing of bacterial pigments regulates antibacterial defence. Nature. Aug 28 2014;512(7515):387-392.
32. Song MS, Song SJ, Kim SY, Oh HJ, Lim DS. The tumour suppressor RASSF1A promotes MDM2 self-ubiquitination by disrupting the MDM2-DAXX-HAUSP complex. The EMBO journal. Jul 9 2008;27(13):1863-1874.
33. Wang H, Nestor CE, Benson M, Zhang H. GAB2 regulates type 2 T helper cell differentiation in humans. Cytokine. Aug 2017;96:234-237.
34. Verploegen S, Lammers JW, Koenderman L, Coffer PJ. Identification and characterization of CKLiK, a novel granulocyte Ca(++)/calmodulin-dependent kinase. Blood. Nov 1 2000;96(9):3215-3223.
35. Lin E, Kuo PH, Liu YL, Yu YW, Yang AC, Tsai SJ. A Deep Learning Approach for Predicting Antidepressant Response in Major Depression Using Clinical and Genetic Biomarkers. Frontiers in psychiatry. 2018;9:290.
36. Zhou X, Chen Y, Mok KY, et al. Identification of genetic risk factors in the Chinese population implicates a role of immune system in Alzheimer's disease pathogenesis. Proceedings of the National Academy of Sciences of the United States of America. Feb 20 2018;115(8):1697-1706.
37. Beurel E, Lowell JA. Th17 cells in depression. Brain, behavior, and immunity. Mar 2018;69:28-34.
38. Fischer EH, Charbonneau H, Tonks NK. Protein tyrosine phosphatases: a diverse family of intracellular and transmembrane enzymes. Science. Jul 26 1991;253(5018):401-406.
39. Irie-Sasaki J, Sasaki T, Matsumoto W, et al. CD45 is a JAK phosphatase and negatively regulates cytokine receptor signalling. Nature. Jan 18 2001;409(6818):349-354.
40. Basterzi AD, Yazici K, Buturak V, et al. Effects of venlafaxine and fluoxetine on lymphocyte subsets in patients with major depressive disorder: a flow cytometric analysis. Progress in neuro-psychopharmacology & biological psychiatry. Feb 1 2010;34(1):70-75.
41. Aston C, Jiang L, Sokolov BP. Transcriptional profiling reveals evidence for signaling and oligodendroglial abnormalities in the temporal cortex from patients with major depressive disorder. Molecular psychiatry. Mar 2005;10(3):309-322.
42. Seney ML, Huo Z, Cahill K, et al. Opposite Molecular Signatures of Depression in Men and Women. Biological psychiatry. Jul 1 2018;84(1):18-27.
43. Wohleb ES, Franklin T, Iwata M, Duman RS. Integrating neuroimmune systems in the neurobiology of depression. Nature reviews. Neuroscience. Aug 2016;17(8):497-511.
44. Weissmann D, van der Laan S, Underwood MD, et al. Region-specific alterations of A-to-I RNA editing of serotonin 2c receptor in the cortex of suicides with major depression. Translational psychiatry. Aug 30 2016;6(8):e878.

## Claims

1. A method for selecting a combination of at least two biomarkers which can be used to diagnose depression disorders, preferably major depressive disease (MDD), in a human patient from a blood sample of said patient, said method comprising the step of:
a) analyzing the RNA-Seq dataset using Editome analysis pipeline and identifying A-to-I differentially edited positions with a minimum coverage of 30x, which ensures a high degree of confidence at particular base positions;
b) performing a differential analysis to identify sites whose editing could be specifically different between patients having depressive disorders, preferably MDD and healthy controls,
c) applying the following pre-specified quality criteria of the group consisting of: coverage >30; AUC>0.6; 0.95>FoldChange>1.05, p<0.05 and exclusion of intergenic sites;
d) optionally, checking that no difference in global RNA editing was observed between patients and controls, preferably by calculating the global Alu editing index (AEI), which is a measure of the overall rate of RNA editing in Alu repeats; e) by GSEA (enrichment analysis on gene sets) on the biomarkers selecting in step c) or d), preferably by using gene ontology tools, identifying and selecting biomarkers reflecting changes in different biological process including immune and CNS (central nervous system) functions ; and
f) applying the following specified quality criteria of the group consisting of: coverage >30; AUC>0.8; 0.8>FoldChange>1.20 and p<0.05) to a combination of several biomarkers selected in step e) representing different biological mechanisms, and selected those which clearly discriminated healthy controls and patients having depression disorders, preferably major depressive disease (MDD) in two separate groups.

2. An in vitro method for diagnosing depression disorders, in a human patient from a biological sample of said patient, said method comprising:
a) determining for a combination of at least two A to I editing RNA biomarker identified by the method of claim 1:
- for each selected biomarker, the relative proportion of RNA editing at a given editing site for at least one or a combination of sites which can be edited on the RNA transcript of said at least two biomarkers, and/or
- the relative percentage of an isoform or of a combination of isoforms of the RNA transcript of each of said two biomarkers;
wherein said at least two A to I editing RNA biomarkers are selected from the group consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers,
b) determining a result value obtained for each of said at least two biomarkers and a final result value based on an algorithm or equation that includes the result value obtained for each selected biomarker;
c) determining the final result value or depression score obtained for the patient and a control result value obtained for a healthy control subject, wherein the control result value was determined in a manner comparable to that of the final result value; and
d) if said final result or depression score value is greater than a threshold/cut-off, classifying said patient as being positive or having depression disorders, or, if said final result value is not greater than said threshold, classifying said patient as being negative or not having depression disorders.

3. The in vitro method for diagnosing depression according to claim 2, wherein in step b), said at least two A to I editing RNA biomarkers are selected from the group consisting of PRKCB, MDM2, IFNAR1, KCNJ15, LYN, CAMK1D, GAB2 and PDE8A.

4. The in vitro method for diagnosing depression disorders, preferably MDD, according to one of claims 2 and 3, wherein in step b), the result value or depression score is calculated by an algorithm implementing a multivariate method including:
- mROC program, particularly to identify the linear combination, which maximizes the AUC (Area Under the Curve) ROC and wherein the equation for the respective combination is provided and can be used as a new virtual marker Z, as follows: Z = a.(Biomarker 1) + b.(Biomarker 2) + ...i.(Biomarker i) +....n .(Biomarker n) where i are calculated coefficients and (Biomarker i) are the level of the considered biomarker; and/or
- a Random Forest (RF) approach applied to assess the RNA editing site(s) and/or isoforms combinations, particularly to rank the importance of the RNA editing site(s) and/or isoform(s), and to combine the best RNA editing site(s) and/or isoform(s), and/or optionally
- a multivariate analysis applied to assess the RNA editing site(s) and/or isoforms combinations for the diagnostic, said multivariate analysis being selecting for example from the group consisting of:
- logistic regression model applied for univariate and multivariate analysis to estimate the relative risk of patient at different level of RNA editing site or isoforms values; and/or
- CART (Classification And Regression Trees) approach applied to assess RNA editing site(s) and/or isoforms combinations; and/or
- Support Vector Machine (SVM) approach;
- Artificial Neural Network (ANN) approach;
- Bayesian network approach;
- WKNN (weighted k-nearest neighbours) approach;
- Partial Least Square - Discriminant Analysis (PLS-DA);
- Linear and Quadratic Discriminant Analysis (LDA / QDA), and
- Any other mathematical method that combines biomarkers.

5. The method of one of claims 2 to 4, wherein said depression disorder is MDD.

6. The method of one of claims 2 to 5, wherein said biological sample is whole blood, serum, plasma, urine, cerebrospinal fluid or saliva, preferred is the whole blood sample.

7. The method of one of claims 2 to 6, wherein for each selected biomarker, said result value is statistically/specifically different from said control result value at p<0.05.

8. The method of one of claims 2 to 7, wherein the following criteria has to be satisfied for the biomarker or the combination of biomarkers selected tin step a):
- the coverage >30;
- AUC>0.6;
- 0.95 >FoldChange >1.05, and
- p<0.05.

9. The method of one of claims 2 to 8, wherein said selected A to I editing RNA biomarker(s) comprised in step a) is selected from the group consisting of:
a combination comprising at least 3, 4, 5, 6, 7 or 8 of the following biomarkers: PRKCB, MDM2, IFNAR1, KCNJ15, LYN, CAMK1D, GAB2 and PDE8A, preferably the combination of the cited 8 biomarkers.

10. The method of one of claims 2 to 9, wherein said combination of at least 2, 3, 4, 5, 6, 7 or 8 selected biomarkers comprises the calculation of the relative percentage of at least one of the RNA edition site or isoform listed in Table 2A (edition sites), 2B (isoforms) or 3 (edition sites) for each of the selected biomarker.

11. The method of one of claims 3 to 10, wherein said combination of at least 2, 3, 4, 5, 6, 7 or 8 selected biomarkers is selected from the biomarkers combinations given in Tables 12, 13 and 15, preferably the combinations comprising the following 8 biomarkers PRKCB, MDM2, IFNAR1, KCNJ15, LYN, CAMK1D, GAB2 and PDE8A.

12. The method of one of claims 2 to 11, wherein the algorithm or equation allowing the calculation of the result value or depression score Z are selected from the Z equations listed in the examples and the figures, preferably the equation implemented a combination of the following 8 biomarkers PRKCB, MDM2, IFNAR1, KCNJ15, LYN, CAMK1D, GAB2 and PDE8A.

13. An in vitro method for diagnosing depression disorders, preferably MDD, in a human patient according to one of claims 2 to 12, wherein if the patient to be tested is a female, in step a) the biomarkers of said combination of at least two one A to I editing RNA biomarker are selected from the group of biomarkers consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers, preferably the PRKCB, MDM2, IFNAR1, KCNJ15, LYN, CAMK1D, GAB2 and PDE8A biomarkers, preferably the combination of 8 biomarkers listed in Table 7.

14. An in vitro method for diagnosing depression disorders, preferably MDD, in a human patient according to one of claims 2 to 12, wherein if the patient to be tested is a male, in step a) the biomarkers of said combination of at least two one A to I editing RNA biomarker are selected from the group of biomarkers consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers, preferably the PRKCB, MDM2, IFNAR1, KCNJ15, LYN, CAMK1D, GAB2 and PDE8A biomarkers, preferably the combination of 8 biomarkers listed in Table 9.

15. An in vitro method for diagnosing depression disorders, preferably MDD, in a human patient wherein said method comprises the method for diagnosing depression for a human patient according to one of claims 2 to 14, wherein the combination of biomarkers and/or the Z equation associated with said combination used for the diagnosing of depression disorders is different whether the patient to be tested is a female or a male.

16. The method of one of claims 2 to 15, wherein in step a) the relative proportion of RNA editing at a given editing and/or the percentage of an isoform are measuring by NGS in said biological sample.

17. The method of one of claims 2 to 16, wherein in step a), the amplicon/nucleic sequence used for the detection of the RNA editing sites and/or the isoforms of the RNA transcript of said biomarker is obtained or obtainable with:
- the set of primers listed in Table 1 for each of the selected biomarkers (SEQ ID NO. 1 to 36), or
- a set or a combination of sets of primers allowing to obtain amplicon(s) including, or identical to, the amplicon(s) obtainable by the set of primers listed in Table 1 (SEQ ID No.1 to SEQ ID No.36).

18. A method for monitoring treatment for depression disorders, preferably MDD, in a human subject, said method comprising:
A) diagnosing depression disorders in said human by the method according to one of claims 2 to 17 before the beginning of the treatment which is desired to be monitored.
B) repeating steps (a) to c) of the method of one of claims 2 to 17, after a period of time during which said patient receives treatment for said depression disorders to obtain a post-treatment depression score;
C) comparing the post-treatment depression score from step (c) to:
- the depression score obtained before the period of time during which said patient receives treatment for said depression disorders, and
- to the depression score (control final value) for normal/healthy subjects, and classifying said treatment as being effective if the post-treatment depression score obtained in step B) is closer than the depression score obtained before the period of time during which said patient receives treatment for the depression disorders score obtained for normal/healthy subjects.

19. Kit for determining whether a patient present a depression disorder, preferably MDD, said kit comprising:
1) optionally instructions to apply the method according to one claims 1 to 17, in order to obtain the result value or depression score the analysis of which determining whether said patient present a depression disorders, preferably MDD; and
2)
- a combination of at least two set of primers from the group of pairs of primers selecting from the SEQ ID No.1 to SEQ ID No.36, the selection of which being dependent of the biomarkers combination used for depression disorders, preferably MDD, diagnostic;
or
- a combination of at least two sets of primers allowing to obtain amplicons including, or identical to, the amplicons obtainable by the set of primers listed in Table 1 (SEQ ID No.1 to SEQ ID No.36).
